(19) Europäisches Patentamt
European Patent Office
Office européen des brevets

(11) EP 3 268 484 B1

(12) EUROPEAN PATENT SPECIFICATION

(45) Date of publication and mention of the grant of the patent:
**17.06.2020 Bulletin 2020/25**

(21) Application number: **16710650.9**

(22) Date of filing: **11.03.2016**

(51) Int Cl.:
***C12P 19/02*** (2006.01) ***C12P 19/14*** (2006.01)
***C12P 7/06*** (2006.01)

(86) International application number:
**PCT/US2016/022080**

(87) International publication number:
**WO 2016/145350 (15.09.2016 Gazette 2016/37)**

(54) **MULTI-STAGE ENZYMATIC HYDROLYSIS OF LIGNOCELLULOSIC BIOMASS**

MEHRSTUFIGE ENZYMATISCHE HYDROLYSE VON LIGNOCELLULOSEHALTIGER BIOMASSE

HYDROLYSE ENZYMATIQUE DE BIOMASSE LIGNOCELLULOSIQUE À PLUSIEURS ÉTAPES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.03.2015 US 201562132126 P**

(43) Date of publication of application:
**17.01.2018 Bulletin 2018/03**

(73) Proprietors:
- **Novozymes A/S**
  **2880 Bagsvaerd (DK)**
- **BETA RENEWABLES S.p.A.**
  **15057 Tortona (IT)**

(72) Inventors:
- **FRICKMANN, Jesper**
  **Raleigh, North Carolina 27614 (US)**
- **BELFRAGE, Johan**
  **31272 Skummeslövsstrand (SE)**
- **ABBATE, Eric**
  **Drew, California 95618 (US)**
- **OSBORN, David**
  **Davis, California 95618 (US)**
- **MOXLEY, Geoffrey**
  **Franklinton, North Carolina 27525 (US)**
- **RIVA, Daniele**
  **18100 Prela Imperia (IT)**
- **OTONELLO, Piero**
  **20143 Milano (IT)**
- **FERRERO, Simone**
  **15057 Tortona (IT)**
- **PURROTTI, Micol**
  **10055 Condove (IT)**
- **PARAVISI, Stefano**
  **15057 Tortona (IT)**
- **PREFUMO, Chiara**
  **16163 Genova (IT)**

(74) Representative: **NZ EPO Representatives**
**Krogshoejvej 36**
**2880 Bagsvaerd (DK)**

(56) References cited:
**WO-A1-2009/046524 WO-A1-2011/157427**
**WO-A1-2014/108454**

- **GAO, D. ET AL.: "Mixture optimization of six core glycosyl hydrolases for maximizing saccharification of ammonia fiber expansion (AFEX) pretreated corn stover", BIORESOURCE TECHNOLOGY, vol. 101, no. 8, 30 November 2009 (2009-11-30), pages 2770-2781, XP026833855,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present invention relates generally to processes for enhancing enzymatic hydrolysis of biomass by conducting hydrolysis in at least two stages, where in a stage a first enzyme preparation comprising a combination of a xylanase, a beta-xylosidase and an endoglucanase is added, followed by a latter stage in which a second enzyme composition comprising cellulases is added. The invention also relates to processes for obtaining hydrolysis products and fermentation products using processes of the invention.

### DESCRIPTION OF RELATED ART

Renewable energy sources provide an alternative to current fossil fuel dependence. Production of ethanol as an energy source includes the basic steps of hydrolysis and fermentation. These steps are integrated within larger processes to obtain ethanol from various source materials.

Lignocellulosic biomass is comprised of cellulose, hemicellulose and lignin. To make the biomass accessible for hydrolysis, pretreatment is often performed, which may increase availability of the material for hydrolysis and thereby increase yields from hydrolysis processes. Selection of a pretreatment method may depend on many factors, such as biomass type, source and composition.

While pretreatment methods are effective to render the biomass available for hydrolysis, such methods may also generate inhibitors to hydrolysis and/or fermentation. Ideally, a selected pretreatment method will balance these considerations, maximizing availability of the biomass for hydrolysis, while minimizing formation of inhibitors.

In the hydrolysis step the source material is hydrolyzed to break down cellulose and/or hemicellulose to fermentable sugars. Commonly, enzymatic hydrolysis is utilized, but the presence of inhibitors, as well as other limitations may limit the yield achieved.

Hydrolysis processes may include batch reactors, continuously operating reactors, semi-batch reactors or semi-continuous reactors, or a combination thereof. Where the hydrolysis process includes use of, *e.g.*, a mixed flow reactor, a fed batch reactor, or a continuously stirred tank reactor (CSTR), or series of such reactors, cost savings may be realized, as well as process advantages, such as ease of construction, increased volume production and decreased downtime.

However such systems may also provide limitations such as more detailed operations and possible problems arising from inefficient mixing within the reactor, as well as a startup time required to reach a steady state of operation.

Despite the potential limitations arising from selection of a reactor for hydrolysis, it is desired to boost production of fermentable sugars from the hydrolysis, while maintaining low overall expenditures of both time and resources.

While it is known that simply adding more enzyme during hydrolysis can often boost overall sugar production, and, correspondingly, fermentation yields, such an approach is not generally desirable in large scale production of ethanol, due to the increased costs of adding additional enzymes, as well as the possible inhibitory effects from accumulation of hydrolysis products, e.g., cellobiose, glucose and xylose.

There is therefore a need in the art for additional processes of hydrolyzing lignocellulosic biomass that address the inhibitors that may be present from pretreatment and improve the production of fermentable sugars and/or fermentation yields. Where continuously operating reactors are used in hydrolysis processes, there is a particular need for such improvement, without excessive increase in the total amount of enzymes or in enzyme consumption. The present invention provides such processes.

### SUMMARY OF THE INVENTION

Described herein are processes for hydrolyzing lignocellulosic material to improve yields of the resultant sugars for fermentation. The present invention is based on the surprising discovery that in a hydrolysis process comprising use of a continuous reactor, providing enzymes in a divided manner, as at least two different enzyme compositions, increases the yield of glucose in the resultant hydrolyzate as compared to adding all enzymes for enzymatic hydrolysis in a single stage hydrolysis. As such, the invention provides a multi stage hydrolysis process in which the enzyme compositions are added in separate stages.

The hydrolysis includes use of a continuously operating reactor a continuously stirred tank reactor (CSTR)) and the sugar yield is increased to levels similar to those yields achieved in a pure batch process. Also described are processes for producing a fermentation product from the hydrolyzate of such a hydrolysis process.

Thus in one aspect, the invention relates to a Z r process of improving a glucose yield of saccharification of a lignocellulosic material, the process comprising the steps of:

a) a first stage comprising saccharifying a lignocellulosic material in a continuous reactor with a first enzyme composition comprising a xylanase, a beta-xylosidase and an endoglucanase; and

b) a second stage comprising continuing saccharification of the lignocellulosic material, comprising combining the material of step a) with a second enzyme composition comprising one or more cellulases to form a hydrolyzate,

wherein the hydrolyzate has a glucose yield that is improved as compared to the yield from a process comprising a single saccharification step,

wherein step a) is performed in a continuously stirred tank reactor (CSTR) and step b) is carried out in a separate batch reactor in series with the reactor from step a). In another aspect the invention relates to a process of producing a fermentation product comprising fermentation of the hydrolyzate.

In another aspect the invention relates to a process of multi-stage hydrolysis of a lignocellulosic material, the process comprising the steps of: a first stage comprising saccharifying a lignocellulosic material with a first enzyme composition comprising a xylanase in an amount of about 4.3 U to about 716.1 U per gram of the lignocellulosic material, a beta-xylosidase in an amount of about 0.005 U to about 0.86 U per gram of the lignocellulosic material and an endoglucanase in an amount of about 2.84 U to about 117.2 U, per gram of the lignocellulosic material and a second stage comprising continuing saccharification of the lignocellulosic material, comprising combining the material of the first stage with a second enzyme composition comprising one or more cellulase, wherein the first step is performed in a continuously stirred tank reactor (CSTR) and the second step is carried out in a separate batch reactor in series with the reactor from the first step.

In a further aspect, the invention relates to a process of producing a fermentation product from a lignocellulosic material, the process comprising the steps of: hydrolyzing the lignocellulosic material, comprising: a first stage comprising saccharifying a lignocellulosic material with a first enzyme composition comprising a xylanase in an amount of about 4.3 U to about 716.1 U per gram of the lignocellulosic material, a beta-xylosidase in an amount of about 0.005 U to about 0.86 U per gram of the lignocellulosic material and an endoglucanase in an amount of about 2.84 U to about 117.2 U per gram of the lignocellulosic material; and a second stage comprising continuing saccharification of the lignocellulosic material, comprising combining the material of the first stage with a second enzyme composition comprising one or more cellulases to form a hydrolyzate and fermenting the hydrolyzate to produce a fermentation product, wherein the first stage is performed in a continuously stirred tank reactor (CSTR) and the second stage is carried out in a separate batch reactor in series with the reactor from the first stage.

## DEFINITIONS

**Acetylxylan esterase:** The term "acetylxylan esterase" means a carboxylesterase (EC 3.1.1.72) that catalyzes the hydrolysis of acetyl groups from polymeric xylan, acetylated xylose, acetylated glucose, alpha-napthyl acetate, and p-nitrophenyl acetate. Acetylxylan esterase activity can be determined using 0.5 mM p-nitrophenylacetate as substrate in 50 mM sodium acetate pH 5.0 containing 0.01% TWEEN™ 20 (polyoxyethylene sorbitan monolaurate). One unit of acetylxylan esterase is defined as the amount of enzyme capable of releasing 1 μmole of p-nitrophenolate anion per minute at pH 5, 25°C.

**Allelic variant:** The term "allelic variant" means any of two or more (e.g., several) alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

**Alpha-L-arabinofuranosidase:** The term "alpha-L-arabinofuranosidase" means an alpha-L-arabinofuranoside arabinofuranohydrolase (EC 3.2.1.55) that catalyzes the hydrolysis of terminal non-reducing alpha-L-arabinofuranoside residues in alpha-L-arabinosides. The enzyme acts on alpha-L-arabinofuranosides, alpha-L-arabinans containing (1,3)- and/or (1,5)-linkages, arabinoxylans, and arabinogalactans. Alpha-L-arabinofuranosidase is also known as arabinosidase, alpha-arabinosidase, alpha-L-arabinosidase, alpha-arabinofuranosidase, polysaccharide alpha-L-arabinofuranosidase, alpha-L-arabinofuranoside hydrolase, L-arabinosidase, or alpha-L-arabinanase. Alpha-L-arabinofuranosidase activity can be determined using 5 mg of medium viscosity wheat arabinoxylan (Megazyme International Ireland, Ltd., Bray, Co. Wicklow, Ireland) per ml of 100 mM sodium acetate pH 5 in a total volume of 200 μl for 30 minutes at 40°C followed by arabinose analysis by AMINEX® HPX-87H column chromatography (Bio-Rad Laboratories, Inc., Hercules, CA, USA).

**Alpha-glucuronidase:** The term "alpha-glucuronidase" means an alpha-D-glucosiduronate glucuronohydrolase (EC 3.2.1.139) that catalyzes the hydrolysis of an alpha-D-glucuronoside to D-glucuronate and an alcohol. Alpha-glucuronidase activity can be determined according to de Vries, 1998, J. Bacteriol. 180: 243-249. One unit of alpha-glucuronidase equals the amount of enzyme capable of releasing 1 μmole of glucuronic or 4-O-methylglucuronic acid per minute at pH 5, 40°C.

**Auxiliary Activity 9 polypeptide:** The term "Auxiliary Activity 9 polypeptide" or "AA9 polypeptide" means a polypeptide classified as a lytic polysaccharide monooxygenase (Quinlan et al., 2011, Proc. Natl. Acad. Sci. USA 208: 15079-15084; Phillips et al., 2011, ACS Chem. Biol. 6: 1399-1406; Lin et al., 2012, Structure 20: 1051-1061). AA9 polypeptides were formerly classified into the glycoside hydrolase Family 61 (GH61) according to Henrissat, 1991, Biochem. J. 280: 309-316, and Henrissat and Bairoch, 1996, Biochem. J. 316: 695-696.

AA9 polypeptides enhance the hydrolysis of a cellulosic material by an enzyme having cellulolytic activity. Cellulolytic enhancing activity can be determined by measuring the increase in reducing sugars or the increase of the total of cellobiose and glucose from the hydrolysis of a cellulosic material by cellulolytic enzyme under the following conditions: 1-50 mg of total protein/g of cellulose in pretreated corn stover (PCS), wherein total protein is comprised of 50-99.5% w/w cellulolytic enzyme protein and 0.5-50% w/w protein of an AA9 polypeptide for 1-7 days at a suitable temperature, such as 40°C-80°C, e.g., 40°C, 45°C, 50°C, 55°C, 60°C, 65°C, 70°C, 75°C, or 80°C, and a suitable pH, such as 4-9, e.g., 4.0, 4.5, 5.0, 5.5, 6.0, 6.5, 7.0, 7.5, 8.0, 8.5, or 9.0, compared to a control hydrolysis with equal total protein loading without cellulolytic enhancing activity (1-50 mg of cellulolytic protein/g of cellulose in PCS).

AA9 polypeptide enhancing activity can be determined using a mixture of CELLUCLAST® 1.5L (Novozymes A/S, Bagsværd, Denmark) and beta-glucosidase as the source of the cellulolytic activity, wherein the beta-glucosidase is present at a weight of at least 2-5% protein of the cellulase protein loading. In one aspect, the beta-glucosidase is an *Aspergillus oryzae* beta-glucosidase *(e.g.,* recombinantly produced in *Aspergillus oryzae* according to WO 02/095014). In another aspect, the beta-glucosidase is an *Aspergillus fumigatus* beta-glucosidase *(e.g.,* recombinantly produced in *Aspergillus oryzae* as described in WO 02/095014).

AA9 polypeptide enhancing activity can also be determined by incubating an AA9 polypeptide with 0.5% phosphoric acid swollen cellulose (PASC), 100 mM sodium acetate pH 5, 1 mM $MnSO_4$, 0.1% gallic acid, 0.025 mg/ml of *Aspergillus fumigatus* beta-glucosidase, and 0.01% TRITON® X-100 (4-(1,1,3,3-tetramethylbutyl)phenyl-polyethylene glycol) for 24-96 hours at 40°C followed by determination of the glucose released from the PASC.

AA9 polypeptide enhancing activity can also be determined according to WO 2013/028928 for high temperature compositions.

AA9 polypeptides enhance the hydrolysis of a cellulosic material catalyzed by enzyme having cellulolytic activity by reducing the amount of cellulolytic enzyme required to reach the same degree of hydrolysis preferably at least 1.01-fold, e.g., at least 1.05-fold, at least 1.10-fold, at least 1.25-fold, at least 1.5-fold, at least 2-fold, at least 3-fold, at least 4-fold, at least 5-fold, at least 10-fold, or at least 20-fold.

The AA9 polypeptide can also be used in the presence of a soluble activating divalent metal cation according to WO 2008/151043 or WO 2012/122518, e.g., manganese or copper.

The AA9 polypeptide can be used in the presence of a dioxy compound, a bicylic compound, a heterocyclic compound, a nitrogen-containing compound, a quinone compound, a sulfur-containing compound, or a liquor obtained from a pretreated cellulosic or hemicellulosic material such as pretreated corn stover (WO 2012/021394, WO 2012/021395, WO 2012/021396, WO 2012/021399, WO 2012/021400, WO 2012/021401, WO 2012/021408, and WO 2012/021410).

**Beta-glucosidase:** The term "beta-glucosidase" means a beta-D-glucoside glucohydrolase (E.C. 3.2.1.21) that catalyzes the hydrolysis of terminal non-reducing beta-D-glucose residues with the release of beta-D-glucose. Beta-glucosidase activity can be determined using p-nitrophenyl-beta-D-glucopyranoside as substrate according to the procedure of Venturi et al., 2002, J. Basic Microbiol. 42: 55-66. One unit of beta-glucosidase is defined as 1.0 μmole of *p*-nitrophenolate anion produced per minute at 25°C, pH 4.8 from 1 mM p-nitrophenyl-beta-D-glucopyranoside as substrate in 50 mM sodium citrate containing 0.01% TWEEN® 20.

**Beta-xylosidase:** The term "beta-xylosidase" means a beta-D-xyloside xylohydrolase (E.C. 3.2.1.37) that catalyzes the exo-hydrolysis of short beta (1→4)-xylooligosaccharides to remove successive D-xylose residues from non-reducing termini. Beta-xylosidase activity can be determined as set forth in Example 10 herein.

**Catalase:** The term "catalase" means a hydrogen-peroxide:hydrogen-peroxide oxidoreductase (EC 1.11.1.6) that catalyzes the conversion of 2 $H_2O_2$ to $O_2$ + 2 $H_2O$. For purposes of the present invention, catalase activity is determined according to U.S. Patent No. 5,646,025. One unit of catalase activity equals the amount of enzyme that catalyzes the oxidation of 1 μmole of hydrogen peroxide under the assay conditions.

**Catalytic domain:** The term "catalytic domain" means the region of an enzyme containing the catalytic machinery of the enzyme.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Cellobiohydrolase:** The term "cellobiohydrolase" means a 1,4-beta-D-glucan cellobiohydrolase (E.C. 3.2.1.91 and E.C. 3.2.1.176) that catalyzes the hydrolysis of 1,4-beta-D-glucosidic linkages in cellulose, cellooligosaccharides, or any beta-1,4-linked glucose containing polymer, releasing cellobiose from the reducing end (cellobiohydrolase I) or non-reducing end (cellobiohydrolase II) of the chain (Teeri, 1997, Trends in Biotechnology 15: 160-167; Teeri et al., 1998, Biochem. Soc. Trans. 26: 173-178). Cellobiohydrolase activity can be determined according to the procedures described by Lever et al., 1972, Anal. Biochem. 47: 273-279; van Tilbeurgh et al., 1982, FEBS Letters 149: 152-156; van Tilbeurgh and Claeyssens, 1985, FEBS Letters 187: 283-288; and Tomme et al., 1988, Eur. J. Biochem. 170: 575-581.

**Cellulolytic enzyme, cellulolytic composition, or cellulase:** The term "cellulolytic enzyme," "cellulolytic enzyme preparation", "cellulolytic composition", or "cellulase" means one or more (e.g., several) enzymes that hydrolyze a cellulosic material. Such enzymes include endoglucanase(s), cellobiohydrolase(s), beta-glucosidase(s), or combinations thereof. The two basic approaches for measuring cellulolytic enzyme activity include: (1) measuring the total cellulolytic enzyme activity, and (2) measuring the individual cellulolytic enzyme activities (endoglucanases, cellobiohydrolases, and beta-glucosidases) as reviewed in Zhang et al., 2006, Biotechnology Advances 24: 452-481. Total cellulolytic enzyme activity can be measured using insoluble substrates, including Whatman №1 filter paper, microcrystalline cellulose, bacterial cellulose, algal cellulose, cotton, pretreated lignocellulose, etc. The most common total cellulolytic activity assay is the filter paper assay using Whatman №1 filter paper as the substrate. The assay was established by the International Union of Pure and Applied Chemistry (IUPAC) (Ghose, 1987, Pure Appl. Chem. 59: 257-68). Cellulase activity can be determined as set forth in Example 8 herein.

Cellulolytic enzyme activity can be determined by measuring the increase in production/release of sugars during hydrolysis of a cellulosic material by cellulolytic enzyme(s) under the following conditions: 1-50 mg of cellulolytic enzyme protein/g of cellulose in pretreated corn stover (PCS) (or other pretreated cellulosic material) for 3-7 days at a suitable temperature such as 40°C-80°C, *e.g.,* 50°C, 55°C, 60°C, 65°C, or 70°C, and a suitable pH such as 4-9, *e.g.,* 5.0, 5.5, 6.0, 6.5, or 7.0, compared to a control hydrolysis without addition of cellulolytic enzyme protein. Typical conditions are 1 ml reactions, washed or unwashed PCS, 5% insoluble solids (dry weight), 50 mM sodium acetate pH 5, 1 mM $MnSO_4$, 50°C, 55°C, or 60°C, 72 hours, sugar analysis by an AMINEX® HPX-87H column (Bio-Rad Laboratories, Inc., Hercules, CA, USA).

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Control sequences:** The term "control sequences" means nucleic acid sequences necessary for expression of a polynucleotide encoding a mature polypeptide of the present invention. Each control sequence may be native *(i.e.,* from the same gene) or foreign *(i.e.,* from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Endoglucanase:** The term "endoglucanase" means a 4-(1,3;1,4)-beta-D-glucan 4-glucanohydrolase (E.C. 3.2.1.4) that catalyzes endohydrolysis of 1,4-beta-D-glycosidic linkages in cellulose, cellulose derivatives (such as carboxymethyl cellulose and hydroxyethyl cellulose), lichenin, beta-1,4 bonds in mixed beta-1,3-1,4 glucans such as cereal beta-D-glucans or xyloglucans, and other plant material containing cellulosic components. Endoglucanase activity can be determined by measuring reduction in substrate viscosity or increase in reducing ends determined by a reducing sugar assay (Zhang *et al*., 2006, *supra).* Endoglucanase activity can also be determined using carboxymethyl cellulose (CMC) as substrate according to the procedure of Ghose, 1987, *supra,* at pH 5, 40°C.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to control sequences that provide for its expression.

**Feruloyl esterase:** The term "feruloyl esterase" means a 4-hydroxy-3-methoxycinnamoyl-sugar hydrolase (EC 3.1.1.73) that catalyzes the hydrolysis of 4-hydroxy-3-methoxycinnamoyl (feruloyl) groups from esterified sugar, which is usually arabinose in natural biomass substrates, to produce ferulate (4-hydroxy-3-methoxycinnamate). Feruloyl esterase (FAE) is also known as ferulic acid esterase, hydroxycinnamoyl esterase, FAE-III, cinnamoyl ester hydrolase, FAEA, cinnAE, FAE-I, or FAE-II. Feruloyl esterase activity can be determined using 0.5 mM p-nitrophenylferulate as substrate in 50 mM sodium acetate pH 5.0. One unit of feruloyl esterase equals the amount of enzyme capable of releasing 1 μmole of p-nitrophenolate anion per minute at pH 5, 25°C.

**Fragment:** The term "fragment" means a polypeptide having one or more (e.g., several) amino acids absent from the amino and/or carboxyl terminus of a mature polypeptide main; wherein the fragment has enzyme activity. In one aspect, a fragment contains at least 85%, e.g., at least 90% or at least 95% of the amino acid residues of the mature polypeptide of an enzyme.

**High stringency conditions:** The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 0.2X SSC, 0.2% SDS at 65°C.

**Hemicellulolytic enzyme, hemicellulolytic composition or hemicellulase:** The term "hemicellulolytic enzyme", "hemicellulolytic enzyme preparation," "hemicellulolytic composition" or "hemicellulase" means one or more (e.g., several) enzymes that hydrolyze a hemicellulosic material. See, for example, Shallom and Shoham, 2003, Current Opinion In Microbiology 6(3): 219-228). Hemicellulases are key components in the degradation of plant biomass. Examples of hemicellulases include, but are not limited to, an acetylmannan esterase, an acetylxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase. The substrates for these enzymes, hemicelluloses, are a heterogeneous group of branched and linear polysaccharides that are bound via hydrogen bonds to the cellulose microfibrils in the plant cell wall, crosslinking them into a robust network. Hemicelluloses are also covalently attached to lignin, forming together with cellulose a highly complex structure. The variable structure and organization of hemicelluloses require the concerted action of many enzymes for its complete degradation. The catalytic modules of hemicellulases are either glycoside hydrolases (GHs) that hydrolyze glycosidic bonds, or carbohydrate esterases (CEs), which hydrolyze ester linkages of acetate or ferulic acid side groups. These catalytic modules, based on homology of their primary sequence, can be assigned into GH and CE families. Some families, with an overall similar fold, can be further grouped into clans, marked alphabetically (e.g., GH-A). A most informative and updated classification of these and other carbohydrate active enzymes is available in the Carbohydrate-Active Enzymes (CAZy) database. Hemicellulolytic enzyme activities can be measured according to Ghose and Bisaria, 1987, Pure & Appl. Chem. 59: 1739-1752, at a suitable temperature such as 40°C-80°C, e.g., 50°C, 55°C, 60°C, 65°C, or 70°C, and a suitable pH such as 4-9, *e.g.*, 5.0, 5.5, 6.0, 6.5, or 7.0.

**Homologous 3' or 5' region:** The term "homologous 3' region" means a fragment of DNA that is identical in sequence or has a sequence identity of at least 70%, e.g., at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% to a region in the genome and when combined with a homologous 5' region can target integration of a piece of DNA to a specific site in the genome by homologous recombination. The term "homologous 5' region" means a fragment of DNA that is identical in sequence to a region in the genome and when combined with a homologous 3' region can target integration of a piece of DNA to a specific site in the genome by homologous recombination. The homologous 5' and 3' regions must be linked in the genome which means they are on the same chromosome and within at least 200 kb of one another.

**Homologous flanking region:** The term "homologous flanking region" means a fragment of DNA that is identical or has a sequence identity of at least 70%, e.g., at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% to a region in the genome and is located immediately upstream or downstream of a specific site in the genome into which extracellular DNA is targeted for integration.

**Homologous repeat:** The term "homologous repeat" means a fragment of DNA that is repeated at least twice in the recombinant DNA introduced into a host cell and which can facilitate the loss of the DNA, *i.e.,* selectable marker that is inserted between two homologous repeats, by homologous recombination. A homologous repeat is also known as a direct repeat.

**Host cell:** The term "host cell" means any cell type that is susceptible to transformation, transfection, transduction, or the like with a nucleic acid construct or expression vector comprising a polynucleotide encoding a polypeptide. The term "host cell" encompasses any progeny of a parent cell that is not identical to the parent cell due to mutations that occur during replication.

**Isolated:** The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (*e.g.*, recombinant production in a host cell; multiple copies of a gene encoding the substance; and use of a stronger promoter than the promoter naturally associated with the gene encoding the substance).

**Low stringency conditions:** The term "low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 0.2X SSC, 0.2% SDS at 50°C.

**Mature polypeptide:** The term "mature polypeptide" means a polypeptide in its final form following translation and any post-translational modifications, such as N-terminal processing, C-terminal truncation, glycosylation, phosphorylation, etc. For instance, the mature polypeptide may be identified, using, *e.g.,* the SignalP program (Nielsen et al., 1997, Protein Engineering 10: 1-6) that predicts a portion of the amino acid sequence as a signal peptide. As such, the mature polypeptide would be identified as the sequence lacking such redicted signal portion.

In one aspect, the mature polypeptide of a beta-glucosidase is amino acids 20 to 863 of SEQ ID NO: 2 based on the SignalP 3.0 program (Bendtsen et al., 2004, J. Mol. Biol. 340: 783-795) that predicts amino acids 1 to 19 of SEQ ID NO: 2 are a signal peptide. In another aspect, the mature polypeptide of a beta-glucosidase variant is amino acids 20 to 863 of SEQ ID NO: 4 based on the SignalP 3.0 program that predicts amino acids 1 to 19 of SEQ ID NO: 4 are a signal peptide. In another aspect, the mature polypeptide of a cellobiohydrolase I is amino acids 27 to 532 of SEQ ID NO: 6 based on the SignalP 3.0 program that predicts amino acids 1 to 26 of SEQ ID NO: 6 are a signal peptide. In another aspect, the mature polypeptide of a cellobiohydrolase II is amino acids 20 to 454 of SEQ ID NO: 8 based on the SignalP 3.0 program that predicts amino acids 1 to 19 of SEQ ID NO: 8 are a signal peptide. In another aspect, the mature polypeptide of In another aspect, the mature polypeptide of an AA9 polypeptide is amino acids 26 to 253 of SEQ ID NO: 10 based on the SignalP 3.0 program that predicts amino acids 1 to 25 of SEQ ID NO: 10 are a signal peptide. In another aspect, the mature polypeptide of a GH10 xylanase is amino acids 20 to 397 of SEQ ID NO: 12 based on the SignalP 3.0 program that predicts amino acids 1 to 19 of SEQ ID NO: 12 are a signal peptide. In another aspect, the mature polypeptide of a GH10 xylanase is amino acids 20 to 398 of SEQ ID NO: 14 based on the SignalP 3.0 program that predicts amino acids 1 to 19 of SEQ ID NO: 14 are a signal peptide. In another aspect, the mature polypeptide of a beta-xylosidase is amino acids 21 to 792 of SEQ ID NO: 16 based on the SignalP 3.0 program that predicts amino acids 1 to 20 of SEQ ID NO: 16 are a signal peptide. In another aspect, the mature polypeptide of a beta-xylosidase is amino acids 22 to 796 of SEQ ID NO: 18 based on the SignalP 3.0 program that predicts amino acids 1 to 21 of SEQ ID NO: 18 are a signal peptide. In another aspect, the mature polypeptide of an endoglucanase II is amino acids 19 to 335 of SEQ ID NO: 20 based on the SignalP 3.0 program that predicts amino acids 1 to 18 of SEQ ID NO: 20 are a signal peptide.

It is known in the art that a host cell may produce a mixture of two of more different mature polypeptides (*i.e.,* with a different C-terminal and/or N-terminal amino acid) expressed by the same polynucleotide.

**Mature polypeptide coding sequence:** The term "mature polypeptide coding sequence" means a polynucleotide that encodes a mature polypeptide having enzyme activity.

**Medium stringency conditions:** The term "medium stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 0.2X SSC, 0.2% SDS at 55°C.

**Medium-high stringency conditions:** The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 0.2X SSC, 0.2% SDS at 60°C.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

**Operably linked:** The term "operably linked" means a configuration in which a control sequence is placed at an appropriate position relative to the coding sequence of a polynucleotide such that the control sequence directs expression of the coding sequence.

**Parent Enzyme:** The term "parent" means an enzyme to which an alteration is made to produce a variant. The parent may be a naturally occurring (wild-type) polypeptide or a variant thereof.

**Pretreated cellulosic or hemicellulosic material:** The term "pretreated cellulosic or hemicellulosic material" means a cellulosic or hemicellulosic material derived from biomass by treatment with heat and dilute sulfuric acid, alkaline pretreatment, neutral pretreatment, or any pretreatment known in the art.

**Pretreated corn stover:** The term "Pretreated Corn Stover" or "PCS" means a cellulosic material derived from corn stover by treatment with heat and dilute sulfuric acid, alkaline pretreatment, neutral pretreatment, or any pretreatment known in the art.

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present invention, the sequence identity between two deoxyribonucleotide sequences is determined using the Needleman-Wunsch algorithm (Needleman and Wunsch, 1970, *supra)* as implemented in the Needle program of the EMBOSS package (EMBOSS: The European Molecular Biology Open Software Suite, Rice et al., 2000, *supra),* preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment)

**Subsequence:** The term "subsequence" means a polynucleotide having one or more (e.g., several) nucleotides absent from the 5' and/or 3' end of a mature polypeptide coding sequence, wherein the subsequence encodes a fragment having enzyme activity. In one aspect, a subsequence contains at least 85%, e.g., at least 90% or at least 95% of the nucleotides of the mature polypeptide coding sequence of an enzyme.

**Transformant:** The term "transformant" means a cell which has taken up extracellular DNA (foreign, artificial or modified) and expresses the gene(s) contained therein.

**Transformation:** The term "transformation" means the introduction of extracellular DNA into a cell, *i.e.,* the genetic alteration of a cell resulting from the direct uptake, incorporation and expression of exogenous genetic material (exogenous DNA) from its surroundings and taken up through the cell membrane(s).

**Variant:** The term "variant" means a polypeptide having enzyme or enzyme enhancing activity comprising an alteration, *i.e.,* a substitution, insertion, and/or deletion, at one or more (e.g., several) positions. A substitution means replacement of the amino acid occupying a position with a different amino acid; a deletion means removal of the amino acid occupying a position; and an insertion means adding an amino acid adjacent to and immediately following the amino acid occupying a position.

**Very high stringency conditions:** The term "very high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 0.2X SSC, 0.2% SDS at 70°C.

**Very low stringency conditions:** The term "very low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 0.2X SSC, 0.2% SDS at 45°C.

**Whole broth preparation:** The term "whole broth preparation" means a composition produced by a naturally-occurring source, *i.e.,* a naturally-occurring microorganism that is unmodified with respect to the cellulolytic and/or hemicellulolytic enzymes produced by the naturally-occurring microorganism, or a non-naturally-occurring source, *i.e.,* a non-naturally-occurring microorganism, *e.g.*, mutant, that is unmodified with respect to the cellulolytic and/or hemicellulolytic enzymes produced by the non-naturally-occurring microorganism.

**Wild-Type Enzyme:** The term "wild-type" enzyme means an enzyme expressed by a naturally occurring microorganism, such as a bacterium, yeast, or filamentous fungus found in nature.

**Xylan-containing material:** The term "xylan-containing material" means any material comprising a plant cell wall polysaccharide containing a backbone of beta-(1-4)-linked xylose residues. Xylans of terrestrial plants are heteropolymers possessing a beta-(1-4)-D-xylopyranose backbone, which is branched by short carbohydrate chains. They comprise D-glucuronic acid or its 4-*O*-methyl ether, L-arabinose, and/or various oligosaccharides, composed of D-xylose, L-arabinose, D- or L-galactose, and D-glucose. Xylan-type polysaccharides can be divided into homoxylans and heteroxylans, which include glucuronoxylans, (arabino)glucuronoxylans, (glucurono)arabinoxylans, arabinoxylans, and complex heteroxylans. See, for example, Ebringerova et al., 2005, Adv. Polym. Sci. 186: 1-67.

In processes of the present invention, any material containing xylan may be used. In a preferred aspect, the xylan-containing material is lignocellulose.

**Xylan degrading activity or xylanolytic activity:** The term "xylan degrading activity" or "xylanolytic activity" means a biological activity that hydrolyzes xylan-containing material. The two basic approaches for measuring xylanolytic activity include: (1) measuring the total xylanolytic activity, and (2) measuring the individual xylanolytic activities (e.g., endoxylanases, beta-xylosidases, arabinofuranosidases, alpha-glucuronidases, acetylxylan esterases, feruloyl esterases, and alpha-glucuronyl esterases). Recent progress in assays of xylanolytic enzymes was summarized in several publications including Biely and Puchard, 2006, Journal of the Science of Food and Agriculture 86(11): 1636-1647; Spanikova and Biely, 2006, FEBS Letters 580(19): 4597-4601; Herrmann et al., 1997, Biochemical Journal 321: 375-381.

Total xylan degrading activity can be measured by determining the reducing sugars formed from various types of xylan, including, for example, oat spelt, beechwood, and larchwood xylans, or by photometric determination of dyed xylan fragments released from various covalently dyed xylans. A common total xylanolytic activity assay is based on production of reducing sugars from polymeric 4-O-methyl glucuronoxylan as described in Bailey et al., 1992, Interlaboratory testing of methods for assay of xylanase activity, Journal of Biotechnology 23(3): 257-270.

Xylan degrading activity can be determined by measuring the increase in hydrolysis of birchwood xylan (Sigma Chemical Co., Inc., St. Louis, MO, USA) by xylan-degrading enzyme(s) under the following typical conditions: 1 ml reactions, 5 mg/ml substrate (total solids), 5 mg of xylanolytic protein/g of substrate, 50 mM sodium acetate pH 5, 50°C, 24 hours, sugar analysis using p-hydroxybenzoic acid hydrazide (PHBAH) assay as described by Lever, 1972, Anal. Biochem. 47: 273-279.

**Xylanase:** The term "xylanase" means a 1,4-beta-D-xylan-xylohydrolase (E.C. 3.2.1.8) that catalyzes the endohydrolysis of 1,4-beta-D-xylosidic linkages in xylans. Xylanase activity can be determined as set forth in Example 9 herein.

Reference to "about" a value or parameter herein includes aspects that are directed to that value or parameter *per se.* For example, description referring to "about X" includes the aspect "X".

As used herein and in the appended claims, the singular forms "a," "or," and "the" include plural referents unless the context clearly dictates otherwise. It is understood that the aspects of the invention described herein include "consisting" and/or "consisting essentially of" aspects.

Unless defined otherwise or clearly indicated by context, all technical and scientific terms used herein have the same meaning as commonly understood by one of ordinary skill in the art to which this invention belongs.

## BRIEF DESCRIPTION OF THE DRAWINGS

Fig. 1 is a graph showing the glucose yield of two hydrolysis samples as described in Example 1.

Fig. 2 is a graph of the measured viscosity of biomass slurry versus time for the three different enzyme dosages as described in Example 2.

Fig. 3 is a graph showing the MIN pressure measurements obtained with a ViPr viscometer for 5 mg EP/g glucan of each enzyme composition, as set forth in Example 3.

Fig. 4 is a graph showing the MIN pressure measurements obtained with a ViPr viscometer for varied doses (3, 4, 5, 6, 7 and 8 mg EP/g glucan) of enzyme composition CPrepA, as set forth in Example 3.

Fig. 5 is a graph showing the MIN pressure measurements obtained with a ViPr viscometer for varied doses (1.5, 2, 3, 4, 5, 6mg EP/g glucan) of the 50:50 blend of XPrepB and EG1, as set forth in Example 3.

Fig. 6 provides graphs of the glucose yield (A)and xylose yield (B) from each of the four reactors described Example 5: R1: CPrepA, 2% DO, pH5.2; R2: EG1+XPrepB for 18.5h, CPrepA 2% DO, pH5.2; R3: EG1+XPrepB for 18.5h at pH 4.8, CPrepA 2% DO, pH5.2; R4: EG1+XPrepB for 18.5h at pH 5.2, CPrepA 2% DO, pH5.2.

Fig. 7 provides graphs of the pH activity (A) and temperature activity (B) of the endoglucanase composition described in Example 6.

Fig. 8 provides a graph of the pH activity of the beta-xylosidase composition as described in Example 7.

## DETAILED DESCRIPTION

Described herein are processes for improving the yield of one or more sugars from a hydrolysis process, the process comprising administration of enzymes for hydrolysis as at least two different enzyme compositions. Further described are processes of hydrolysis and processes of fermentation incorporating such improved sugar yield processes. Also described are enzyme compositions suitable for use in the processes and/or methods described herein.

The present inventors have surprisingly found that by conducting hydrolysis in at least two stages, a first stage comprising contacting a pretreated lignocellulose-containing material with a first enzyme composition comprising a combination of xylanase, beta-xylosidase and an endoglucanase, followed by a latter stage in which a second enzyme composition comprising cellulases is added, the hydrolysis yield can be increased, wherein the first stage is performed in a continuously stirred tank reactor (CSTR) and the second stage is carried out in a separate batch reactor in series with the reactor from the first stage.

Increase of the yield is achieved as compared to the yield obtained from an equivalent process not utilizing a multi-stage process as described herein. The hydrolysis yield is increased relative to a process in which all enzymes for enzymatic hydrolysis are added in a single saccharification stage. In another example the hydrolysis yield is increased relative to a process in which all enzymes for enzymatic hydrolysis are blended prior to administration. In still another example the hydrolysis yield is increased relative to a process in which all enzymes for enzymatic hydrolysis are added in a constant feed. In still another example, the hydrolysis yield from a multi-stage hydrolysis process comprising use of a continuous reactor is increased to a level comparable to the yield obtained from a pure batch hydrolysis.

The first and second enzyme compositions are different from one another. In an example a first enzyme composition comprises a combination of a xylanase, a beta-xylosidase and one or more cellulases. In the invention the first enzyme composition comprises a combination of a xylanase, a beta-xylosidase and an endoglucanase. In a still further example a first enzyme composition comprises at least an endoglucanase. Taken together, the first and second enzyme compositions provide up to about 100% of the total enzymes added during enzymatic hydrolysis. In an example the first enzyme composition provides about 1 to about 99%, e.g., about 1% to about 45%, about 2% to about 40%, or about 5% to about 35% of the total enzyme protein added in hydrolysis.

## Cellulosic Material

Processes of the present invention are carried out using lignocellulosic material. The term "cellulosic material" means any material containing cellulose. The predominant polysaccharide in the primary cell wall of biomass is cellulose, the second most abundant is hemicellulose, and the third is pectin. The secondary cell wall, produced after the cell has stopped growing, also contains polysaccharides and is strengthened by polymeric lignin covalently cross-linked to hemicellulose. Cellulose is a homopolymer of anhydrocellobiose and thus a linear beta-(1-4)-D-glucan, while hemicelluloses include a variety of compounds, such as xylans, xyloglucans, arabinoxylans, and mannans in complex branched structures with a spectrum of substituents. Although generally polymorphous, cellulose is found in plant tissue primarily as an insoluble crystalline matrix of parallel glucan chains. Hemicelluloses usually hydrogen bond to cellulose, as well as to other hemicelluloses, which help stabilize the cell wall matrix.

Cellulose is generally found, for example, in the stems, leaves, hulls, husks, and cobs of plants or leaves, branches, and wood of trees. The cellulosic material may be, but is not limited to, agricultural residue, herbaceous material (including energy crops), municipal solid waste, pulp and paper mill residue, waste paper, and wood (including forestry residue) (see, for example, Wiselogel et al., 1995, in Handbook on Bioethanol (Charles E. Wyman, editor), pp.105-118, Taylor & Francis, Washington D.C.; Wyman, 1994, Bioresource Technology 50: 3-16; Lynd, 1990, Applied Biochemistry and Biotechnology 24/25: 695-719; Mosier et al., 1999, Recent Progress in Bioconversion of Lignocellulosics, in Advances in Biochemical Engineering/Biotechnology, T. Scheper, managing editor, Volume 65, pp. 23-40, Springer-Verlag, New York). It is understood herein that the cellulose may be in the form of lignocellulose, a plant cell wall material containing lignin, cellulose, and hemicellulose in a mixed matrix. For example, the cellulosic material is any biomass material. In the invention the cellulosic material is lignocellulose-containing biomass material. In the invention the cellulosic material is lignocellulose, which comprises cellulose, hemicelluloses, and lignin.

For example, the cellulosic material is agricultural residue, herbaceous material (including energy crops), municipal solid waste, pulp and paper mill residue, waste paper, or wood (including forestry residue).

In another example the cellulosic material is arundo, bagasse, bamboo, corn cob, corn fiber, corn stover, miscanthus, rice straw, switchgrass, or wheat straw.

In one example, the cellulosic material is fiber, such as corn fiber or wheat fiber. Fiber, such as corn or wheat fiber, may be obtained by fractionation. Fractionation technologies are well-known in the art. In one example the cellulosic material is fiber obtained from dry fractionation processes. In one example the cellulosic material is fiber obtained from wet fractionation processes.

In another example, the cellulosic material is aspen, eucalyptus, fir, pine, poplar, spruce, or willow.

In another example, the cellulosic material is algal cellulose, bacterial cellulose, cotton linter, filter paper, microcrystalline cellulose (*e.g.*, AVICEL®), or phosphoric-acid treated cellulose.

In another example, the cellulosic material is an aquatic biomass. As used herein the term "aquatic biomass" means biomass produced in an aquatic environment by a photosynthesis process. The aquatic biomass may be algae, emergent plants, floating-leaf plants, or submerged plants.

The cellulosic material may be used as is or may be subjected to pretreatment, using conventional methods known in the art, as described more fully herein. In a preferred embodiment, the cellulosic material is pretreated.

## Hemicellulosic material

The term "hemicellulosic material" means any material comprising hemicelluloses. Hemicelluloses include xylan, glucuronoxylan, arabinoxylan, glucomannan, and xyloglucan. These polysaccharides contain many different sugar monomers. Sugar monomers in hemicellulose can include xylose, mannose, galactose, rhamnose, and arabinose. Hemicelluloses contain most of the D-pentose sugars. Xylose is in most cases the sugar monomer present in the largest amount, although in softwoods mannose can be the most abundant sugar. Xylan contains a backbone of beta-(1-4)-linked xylose residues. Xylans of terrestrial plants are heteropolymers possessing a beta-(1-4)-D-xylopyranose backbone, which is branched by short carbohydrate chains. They comprise D-glucuronic acid or its 4-O-methyl ether, L-arabinose, and/or various oligosaccharides, composed of D-xylose, L-arabinose, D- or L-galactose, and D-glucose. Xylan-type polysaccharides can be divided into homoxylans and heteroxylans, which include glucuronoxylans, (arabino)glucuronoxylans, (glucurono)arabinoxylans, arabinoxylans, and complex heteroxylans. See, for example, Ebringerova et al., 2005, Adv. Polym. Sci. 186: 1-67. Hemicellulosic material is also known herein as "xylan-containing material".

Sources for hemicellulosic material are essentially the same as those for cellulosic material described herein. It is understood herein that the hemicellulose may be in the form of lignocellulose, a plant cell wall material containing lignin, cellulose, and hemicellulose in a mixed matrix. For example, the hemicellulosic material is any biomass material. In another example, the hemicellulosic material is lignocellulose, which comprises cellulose, hemicelluloses, and lignin.

## Pretreatment of Cellulosic Material

In practicing the processes of the present invention, the cellulosic material used may be pretreated by any pretreatment process known in the art, used to disrupt plant cell wall components of cellulosic or hemicellulosic material (Chandra et al., 2007, Adv. Biochem. Engin./Biotechnol. 108: 67-93; Galbe and Zacchi, 2007, Adv. Biochem. Engin./Biotechnol. 108: 41-65; Hendriks and Zeeman, 2009, Bioresource Technology 100: 10-18; Mosier et al., 2005, Bioresource Technology 96: 673-686; Taherzadeh and Karimi, 2008, Int. J. Mol. Sci. 9: 1621-1651; Yang and Wyman, 2008, Biofuels Bioproducts and Biorefining-Biofpr. 2: 26-40).

The cellulosic or hemicellulosic material may also be subjected to particle size reduction, sieving, pre-soaking, wetting, washing, and/or conditioning prior to or with additional pretreatment methods, using methods known in the art or as otherwise described herein.

Conventional pretreatments include, but are not limited to, steam pretreatment (with or without explosion), dilute acid pretreatment, hot water pretreatment, alkaline pretreatment, lime pretreatment, wet oxidation, wet explosion, ammonia fiber explosion, organosolv pretreatment, and biological pretreatment. Additional pretreatments include ammonia percolation, ultrasound, electroporation, microwave, supercritical $CO_2$, supercritical $H_2O$, ozone, ionic liquid, and gamma irradiation pretreatments.

In an embodiment the cellulosic or hemicellulosic material is pretreated before hydrolysis and/or fermentation. Pretreatment is preferably performed prior to the hydrolysis. Alternatively, the pretreatment can be carried out simultaneously with enzyme hydrolysis to release fermentable sugars, such as glucose, xylose, and/or cellobiose. In most cases the pretreatment step itself results in some conversion of biomass to fermentable sugars (even in absence of enzymes).

Steam Pretreatment. In steam pretreatment, the cellulosic or hemicellulosic material is heated to disrupt the plant cell wall components, including lignin, hemicellulose, and cellulose to make the cellulose and other fractions, e.g., hemicellulose, accessible to enzymes. The cellulosic material is passed to or through a reaction vessel where steam is injected to increase the temperature to the required temperature and pressure and is retained therein for the desired reaction time. Steam pretreatment is preferably performed at 140-250°C, e.g., 160-200°C or 170-190°C, where the optimal temperature range depends on optional addition of a chemical catalyst. Residence time for the steam pretreatment is preferably 1-60 minutes, e.g., 1-30 minutes, 1-20 minutes, 3-12 minutes, or 4-10 minutes, where the optimal residence time depends on the temperature and optional addition of a chemical catalyst. Steam pretreatment allows for relatively high solids loadings, so that the cellulosic material is generally only moist during the pretreatment. The steam pretreatment is often combined with an explosive discharge of the material after the pretreatment, which is known as steam explosion, that is, rapid flashing to atmospheric pressure and turbulent flow of the material to increase the accessible surface area by fragmentation (Duff and Murray, 1996, Bioresource Technology 855: 1-33; Galbe and Zacchi, 2002, Appl. Microbiol. Biotechnol. 59: 618-628; U.S. Patent Application No. 2002/0164730). During steam pretreatment, hemicellulose acetyl groups are cleaved and the resulting acid autocatalyzes partial hydrolysis of the hemicellulose to monosaccharides and oligosaccharides. Lignin is removed to only a limited extent.

Chemical Pretreatment: The term "chemical pretreatment" refers to any chemical pretreatment that promotes the separation and/or release of cellulose, hemicellulose, and/or lignin. Such a pretreatment may convert crystalline cellulose to amorphous cellulose. Examples of suitable chemical pretreatment processes include, for example, dilute acid pretreatment, lime pretreatment, wet oxidation, ammonia fiber/freeze expansion (AFEX), ammonia percolation (APR), ionic liquid, and organosolv pretreatments.

A chemical catalyst such as $H_2SO_4$ or $SO_2$ (typically 0.3 to 5% w/w) is sometimes added prior to steam pretreatment, which decreases the time and temperature, increases the recovery, and improves enzymatic hydrolysis (Ballesteros et al., 2006, Appl. Biochem. Biotechnol. 129-132: 496-508; Varga et al., 2004, Appl. Biochem. Biotechnol. 113-116: 509-523; Sassner et al., 2006, Enzyme Microb. Technol. 39: 756-762). In dilute acid pretreatment, the cellulosic material is mixed with dilute acid, typically $H_2SO_4$, and water to form a slurry, heated by steam to the desired temperature, and after a residence time flashed to atmospheric pressure. The dilute acid pretreatment may be performed with a number of reactor designs, e.g., plug-flow reactors, counter-current reactors, or continuous counter-current shrinking bed reactors (Duff and Murray, 1996, *supra;* Schell et al., 2004, Bioresource Technology 91: 179-188; Lee et al., 1999, Adv. Biochem. Eng. Biotechnol. 65: 93-115).

Several methods of pretreatment under alkaline conditions may also be used. These alkaline pretreatments include, but are not limited to, sodium hydroxide, lime, wet oxidation, ammonia percolation (APR), and ammonia fiber/freeze expansion (AFEX) pretreatment.

Lime pretreatment is performed with calcium oxide or calcium hydroxide at temperatures of 85-150°C and residence times from 1 hour to several days (Wyman et al., 2005, Bioresource Technology 96: 1959-1966; Mosier *et al.*, 2005, *supra).* WO 2006/110891, WO 2006/110899, WO 2006/110900, and WO 2006/110901 disclose pretreatment methods using ammonia.

Wet oxidation is a thermal pretreatment performed typically at 180-200°C for 5-15 minutes with addition of an oxidative agent such as hydrogen peroxide or over-pressure of oxygen (Schmidt and Thomsen, 1998, Bioresource Technology 64: 139-151; Palonen et al., 2004, Appl. Biochem. Biotechnol. 117: 1-17; Varga et al., 2004, Biotechnol. Bioeng. 88: 567-574; Martin et al., 2006, J. Chem. Technol. Biotechnol. 81: 1669-1677). The pretreatment is performed preferably at 1-40% dry matter, e.g., 2-30% dry matter or 5-20% dry matter, and often the initial pH is increased by the addition of alkali such as sodium carbonate.

A modification of the wet oxidation pretreatment method, known as wet explosion (combination of wet oxidation and steam explosion) can handle dry matter up to 30%. In wet explosion, the oxidizing agent is introduced during pretreatment after a certain residence time. The pretreatment is then ended by flashing to atmospheric pressure (WO 2006/032282).

Ammonia fiber expansion (AFEX) involves treating the cellulosic material with liquid or gaseous ammonia at moderate temperatures such as 90-150°C and high pressure such as 17-20 bar for 5-10 minutes, where the dry matter content can be as high as 60% (Gollapalli et al., 2002, Appl. Biochem. Biotechnol. 98: 23-35; Chundawat et al., 2007, Biotechnol. Bioeng. 96: 219-231; Alizadeh et al., 2005, Appl. Biochem. Biotechnol. 121: 1133-1141; Teymouri et al., 2005, Bioresource Technology 96: 2014-2018). During AFEX pretreatment cellulose and hemicelluloses remain relatively intact. Lignin-carbohydrate complexes are cleaved.

Organosolv pretreatment delignifies the cellulosic material by extraction using aqueous ethanol (40-60% ethanol) at 160-200°C for 30-60 minutes (Pan et al., 2005, Biotechnol. Bioeng. 90: 473-481; Pan et al., 2006, Biotechnol. Bioeng. 94: 851-861; Kurabi et al., 2005, Appl. Biochem. Biotechnol. 121: 219-230). Sulphuric acid is usually added as a catalyst. In organosolv pretreatment, the majority of hemicellulose and lignin is removed.

Other examples of suitable pretreatment methods are described by Schell et al., 2003, Appl. Biochem. Biotechnol. 105-108: 69-85, and Mosier *et al*., 2005, *supra,* and U.S. Published Application 2002/0164730.

In one embodiment, the chemical pretreatment is preferably carried out as a dilute acid treatment, and more preferably as a continuous dilute acid treatment. The acid is typically sulfuric acid, but other acids may also be used, such as acetic acid, citric acid, nitric acid, phosphoric acid, tartaric acid, succinic acid, hydrogen chloride, or mixtures thereof. Mild acid treatment is conducted in the pH range of preferably 1-5, *e.g.,* 1-4 or 1-2.5. In one embodiment, the acid concentration is in the range from preferably 0.01 to 10 wt. % acid, e.g., 0.05 to 5 wt. % acid or 0.1 to 2 wt. % acid. The acid is contacted with the cellulosic material and held at a temperature in the range of preferably 140-200°C, e.g., 165-190°C, for periods ranging from 1 to 60 minutes.

In another embodiment, pretreatment takes place in an aqueous slurry. In preferred embodiments, the cellulosic material is present during pretreatment in amounts preferably between 10-80 wt. %, e.g., 20-70 wt. % or 30-60 wt. %, such as around 40 wt. %. The pretreated cellulosic material may be unwashed or washed using any method known in the art, e.g., washed with water.

Mechanical Pretreatment or Physical Pretreatment: The term "mechanical pretreatment" or "physical pretreatment" refers to any pretreatment that promotes size reduction of particles. For example, such pretreatment may involve various types of grinding or milling (e.g., dry milling, wet milling, or vibratory ball milling).

The cellulosic material may be pretreated both physically (mechanically) and chemically. Mechanical or physical pretreatment may be coupled with steaming/steam explosion, hydrothermolysis, dilute or mild acid treatment, high temperature, high pressure treatment, irradiation (*e.g*., microwave irradiation), or combinations thereof. In one embodiment, high pressure means pressure in the range of preferably about 100 to about 400 psi, *e.g*., about 150 to about 250 psi. In another embodiment, high temperature means temperature in the range of about 100 to about 300°C, *e.g*., about 140 to about 200°C. In a preferred embodiment, mechanical or physical pretreatment is performed in a batch-process using a steam gun hydrolyzer system that uses high pressure and high temperature as defined above, *e.g*., a Sunds Hydrolyzer available from Sunds Defibrator AB, Sweden. The physical and chemical pretreatments may be carried out sequentially or simultaneously, as desired.

Accordingly, in a preferred embodiment, the cellulosic material is subjected to physical (mechanical) or chemical pretreatment, or any combination thereof, to promote the separation and/or release of cellulose, hemicellulose, and/or lignin.

Biological Pretreatment: The term "biological pretreatment" refers to any biological pretreatment that promotes the separation and/or release of cellulose, hemicellulose, and/or lignin from the cellulosic material. Biological pretreatment techniques may involve applying lignin-solubilizing microorganisms and/or enzymes (see, for example, Hsu, T.-A., 1996, Pretreatment of biomass, in Handbook on Bioethanol: Production and Utilization, Wyman, C. E., ed., Taylor & Francis, Washington, DC, 179-212; Ghosh and Singh, 1993, Adv. Appl. Microbiol. 39: 295-333; McMillan, J. D., 1994, Pretreating lignocellulosic biomass: a review, in Enzymatic Conversion of Biomass for Fuels Production, Himmel, M. E., Baker, J. O., and Overend, R. P., eds., ACS Symposium Series 566, American Chemical Society, Washington, DC, chapter 15; Gong, C. S., Cao, N. J., Du, J., and Tsao, G. T., 1999, Ethanol production from renewable resources, in Advances in Biochemical Engineering/Biotechnology, Scheper, T., ed., Springer-Verlag Berlin Heidelberg, Germany, 65: 207-241; Olsson and Hahn-Hagerdal, 1996, Enz. Microb. Tech. 18: 312-331; and Vallander and Eriksson, 1990, Adv. Biochem. Eng./Biotechnol. 42: 63-95).

**Hydrolysis (Saccharification)**

In the hydrolysis step (*i.e.,* saccharification step) the lignocellulosic material, *e.g*., pretreated lignocellulose, is hydrolyzed to break down cellulose and/or hemicellulose to fermentable sugars, such as glucose, cellobiose, xylose, xylulose, arabinose, mannose, galactose, and/or soluble oligosaccharides. The hydrolysis is performed enzymatically by providing enzyme compositions in the saccharification stages.

In conversion of biomass substrates to ethanol and other fuels, particularly in large scale operations, many factors may limit the resultant yield. Specifically addressing such limitations may allow an increase in yield from hydrolysis and, subsequently, fermentation.

While it is desirable in saccharification to provide efficient conversion of biomass to fermentable sugars, simply increasing the solids loading does not produce a corresponding increase in converted product. In fact, as the solids loading is increased, a decrease in enzymatic digestion is generally observed. Such decrease may be attributable to factors such as increased viscosity, difficulty of maintaining enzyme distribution, and increased generation of inhibitors.

In large scale biomass processing, handling of high solids is necessary. However, attempting to process high solids in a batchwise manner will result in a high viscosity, which may result in a slurry that is difficult to pump or stir or otherwise requiring additional means for handling. One method of addressing viscosity has been to operate in a continuous or semi-continuous manner in which the substrate and/or enzymes are fed to a reactor, continuously or periodically. Another manner of addressing viscosity has been shown by administering an endoglucanase for liquefaction prior to saccharification of a lignocellulosic material (WO 2014/108454).

However, even when the viscosity is reduced or otherwise addressed, inhibitors may provide a further hurdle to achieving high yields from hydrolysis. In use of hemicellulose-containing substrates, pretreatment and hydrolysis will result in generation of xylan and various xylooligomers. It is known that the presence of such can inhibit enzymatic activity and inhibit hydrolysis. It has been shown that supplementation with xylanase and beta-xylosidase in large amounts prior to addition of cellulases is beneficial in reducing these known inhibitors in a batch process. (Qing and Wyman, Biotechnology for Biofuels 2011, 4:18.)

The inventors have surprisingly discovered that by using a multi-stage hydrolysis with administration of enzymes as at least two separate enzyme compositions, hydrolysis yields in a hydrolysis process comprising a continuous reactor can be increased, without requiring large amounts of total enzyme protein. Such yields can be increased to levels similar to those achieved from a pure batch process.

Processing of cellulosic material can be implemented using any conventional biomass processing apparatus configured to operate in accordance with embodiments of the invention.

A conventional apparatus can include a fed-batch stirred reactor, a batch stirred reactor, a continuous flow stirred reactor with ultrafiltration, and/or a continuous plug-flow column reactor (de Castilhos Corazza et al., 2003, Acta Scientiarum. Technology 25: 33-38; Gusakov and Sinitsyn, 1985, Enz. Microb. Technol. 7: 346-352), an attrition reactor (Ryu and Lee, 1983, Biotechnol. Bioeng. 25: 53-65). Additional reactor types include fluidized bed, upflow blanket, immobilized, and extruder type reactors for hydrolysis and/or fermentation.

The hydrolysis can be carried out as a continuous process, or series of batch and/or series of continuous processes, where the cellulosic or hemicellulosic material is fed gradually to, for example, an enzyme-containing hydrolysis solution. In an example comprising a multi-stage hydrolysis, at least two stages are carried out in a single reactor. The hydrolysis may also be carried out as a series of batch and continuous processes.

Operation of multiple reactors in series allows for closer control of elements within each reactor, e.g., temperature, pH, mixing, concentration, and the like. Therefore in an example comprising a multi-stage hydrolysis, at least two stages are carried out in separate reactors. In an example, each stage of a multi-stage hydrolysis is carried out in a separate reactor. In a further example, at least one stage in a multi-stage hydrolysis is carried out in a continuous reactor (e.g., CSTR). In a still further example, a continuous reactor in a multi-stage hydrolysis process is followed in series with at least one additional reactor. In the invention a CSTR in a multi-stage hydrolysis process is followed in series by at least one batch reactor.

Continuous operation, such as in use of a CSTR, provides advantages of continuous production and a steady state of operation once the reactor is running. Use of a continuous reactor permits management of high viscosity unhydrolyzed substrate, which also permits operation with higher total solids than might be available in a batch reactor. Semi-batch and semi-continuous operation may permit control of environmental conditions and provide additional flexibility, compared to pure batch processes for selection of optimal conditions. For large scale hydrolysis processes, continuous operation is often preferred to eliminate downtime and to maximize production, though semi-batch and semi-continuous operation may also be used. However, as provided in Example 1, a gap in performance may be seen via a reduced sugar yield when a CSTR is used in a hydrolysis process, versus using a pure batch reactor.

Example 1 provides a two stage hydrolysis of steam exploded wheat straw where a first stage is conducted in CSTR and a second stage is conducted in batch reactor, as compared to the hydrolysis of steam exploded wheat straw in a pure batch process. It is shown in Fig. 1 that a gap in yield is observed, where the process with a first stage CSTR presents a lower glucose yield than the yield from a pure batch process.

In order to improve the hydrolysis yield, particularly in processes comprising a continuous reactor, the present inventors have discovered that improved yields can be achieved through administration of enzymes as at least two separate enzyme compositions in a multi-stage hydrolysis. Such improvement is achieved while keeping enzyme loading low. As described herein, hydrolysis of cellulosic material is performed enzymatically by two or more enzyme compositions in two or more stages of hydrolysis. The invention provides processes including multi-stage hydrolysis including a first stage of hydrolysis comprising adding enzymes to reduce inhibitors and/or reduce viscosity and a second stage of hydrolysis comprising adding hydrolyzing enzymes. Such process is sufficient to improve or increase sugar yields in a hydrolysis process comprising use of a continuous reactor, as compared to yields from a process that does not use a multi-stage enzyme administration. In another example the yields from a hydrolysis process comprising use of a continuous reactor can be improved to a level similar to the yields obtained from a pure batch process. In a further example the improvement or increase in sugar yield from a process of the invention is sufficient to decrease or eliminate an observed gap in yield between a single-stage hydrolysis process including a continuous reactor, as compared to a pure batch process, where the process including a continuous reactor has a lower sugar yield than the pure batch process.

For example, processes include a first stage of hydrolysis where the enzyme activity is sufficient to reduce the inhibitors, e.g., xylo-oligomers, and/or to reduce the viscosity as compared to the inhibitors present in and the viscosity of a pretreated lignocellulosic material not subjected to such a first stage of hydrolysis.

The present invention therefore relates to processes of improving a glucose yield of saccharification of a lignocellulosic material, the process comprising the steps of: a) a first stage comprising saccharifying a lignocellulosic material in a continuous reactor with a first enzyme composition comprising a xylanase, a beta-xylosidase and an endoglucanase; and b) a second stage comprising continuing saccharification of the lignocellulosic material, comprising combining the material of step a) with a second enzyme composition comprising one or more cellulases to form a hydrolyzate, wherein the hydrolyzate has a glucose yield that is improved as compared to the yield from a process comprising a single saccharification step, wherein step a) is performed in a continuously stirred tank reactor (CSTR) and step b) is carried out in a separate batch reactor in series with the reactor from step a). In one embodiment, the processes further comprise recovering the hydrolyzate. Soluble products of degradation of the cellulosic material can be separated from insoluble cellulosic material using a method known in the art such as, for example, centrifugation, filtration, or gravity settling. The first enzyme composition is added in a first stage of hydrolysis and the second enzyme composition is added in a later (e.g., second) stage of hydrolysis. In a further embodiment, the stages of hydrolysis are conducted at a pH independently selected from about 3.5 to about 5.5. In a still further embodiment, the first stage of hydrolysis is conducted at a lower pH than the second stage of hydrolysis. In another embodiment, the second enzyme composition is added at least about 2 hours, at least about 3 hours, at least about 5 hours, at least about 10 hours, or at least about 20 hours following contacting of the lignocellulosic material and the first enzyme composition. For example, the glucose yield or xylose yield is increased to a level similar to a yield obtained from a pure batch saccharification process.

The present invention further relates to processes of multi-stage hydrolysis of a lignocellulosic material, the process comprising the steps of a first stage comprising saccharifying a lignocellulosic material with a first enzyme composition comprising a xylanase in an amount of about 4.3 U to about 716.1 U per gram of the lignocellulosic material, a beta-xylosidase in an amount of about 0.005 U to about 0.86 U per gram of the lignocellulosic material, and an endoglucanase in an amount of about 2.84 U to about 117.2 U per gram of the lignocellulosic material to form a first saccharified material, and a second stage comprising continuing saccharification of the lignocellulosic material, comprising combining the first saccharified material with a second enzyme composition comprising one or more cellulases, wherein the first stage is performed in a continuously stirred tank reactor (CSTR) and the sceond stage is carried out in a separate batch reactor in series with the reactor from the first stage. The first enzyme composition is added in a first stage of hydrolysis and the second enzyme composition is added in a later (e.g., second) stage of hydrolysis. In an embodiment, the stages of hydrolysis are conducted at a pH independently selected from about 3.5 to about 5.5. In a still further embodiment, the first stage of hydrolysis is conducted at a lower pH than the second stage of hydrolysis. In another embodiment, the second enzyme composition is added at least about 2 hours, at least about 3 hours, at least about 5 hours, at least about 10 hours, or at least about 20 hours following contacting of the lignocellulosic material and the first enzyme composition.

In a still further embodiment, the first stage of hydrolysis is conducted at a lower pH than the second stage of hydrolysis. In another embodiment, the second enzyme composition is added at least about 2 hours, at least about 3 hours, at least about 5 hours, at least about 10 hours, or at least about 20 hours following contacting of the lignocellulosic material and the first enzyme composition.

Enzymatic hydrolysis (*i.e.,* saccharification) is preferably carried out in a suitable aqueous environment under conditions that may be readily determined by one skilled in the art. In one embodiment, hydrolysis is performed under conditions suitable for the activity of the enzyme composition, preferably optimal for the enzyme composition.

The hydrolysis is generally performed in stirred-tank reactors or fermentors under controlled pH, temperature, and mixing conditions. Suitable process time, temperature and pH conditions may readily be determined by one skilled in the art.

As used herein "multi-stage hydrolysis" or "multi-stage saccharification" refers to a hydrolysis performed in two or more stages. Stages of hydrolysis may include, but are not limited to, use of one or more reactors, variations in temperature during the hydrolysis process, variations in pH during the hydrolysis process, variations in mixing or stirring, variations in timing (e.g., length of time of each stage) during the hydrolysis process, and variations of enzyme addition during the hydrolysis process. In various embodiments of the invention, stages of hydrolysis may comprise one or more of: different reactors, different temperatures, different pH, different mixing/stirring, and administration of different enzyme compositions.

For example, the hydrolysis may last up to 200 hours, but is typically performed for preferably about 12 to about 120 hours, e.g., about 16 to about 72 hours or about 24 to about 48 hours. In an embodiment of the invention a first stage of hydrolysis is carried out for about 3 to about 36 hours, *e.g.*, about 15 to about 30 hours. In another embodiment of the invention a second stage of hydrolysis is carried out for about for about 3 to about 36 hours, e.g., about 15 to about 30 hours.

The hydrolysis temperature is in the range of preferably about 25°C to about 70°C, e.g., about 30°C to about 65°C, about 40°C to about 60°C, or about 50°C to about 55°C. In one embodiment the first stage of hydrolysis and the second stage of hydrolysis are performed at about the same temperature. In another embodiment a first stage of hydrolysis has a temperature that is varied from the temperature of a second stage of hydrolysis.

The pH of hydrolysis is in the range of preferably about 3 to about 8, e.g., about 3.5 to about 7, about 4 to about 6, or about 4.5 to about 5.5. In an embodiment of the invention the pH of a first stage and a second stage are independently selected from a pH of about 3.5 to about 5.5, i.e., about 3.5, about 3.6, about 3.7, about 3.8, bout 3.9, about 4.0, about 4.1, about 4.2, about 4.3, about 4.4, about 4.5, about 4.6, about 4.7, about 4.8, about 4.9, about 5.0, about 5.1, about 5.2, about 5.3, about 5.4, or about 5.5. In an embodiment a first stage of hydrolysis has a pH that is varied from the pH of a second stage of hydrolysis. In another embodiment a first stage of hydrolysis has a pH that is lower than the pH of a second stage of hydrolysis.

The dry solids content is in the range of preferably about 5 to about 50 wt. %, e.g., about 10 to about 40 wt. % or about 15 to about 30 wt. %.

In a further embodiment at least two stages are conducted at different pH

**Enzymes for Hydrolysis**

The present invention relates to use of enzymes in a multi-stage hydrolysis comprising administration of the enzymes as two or more enzyme compositions. The invention comprises administration of different enzyme compositions in a multi-stage hydrolysis process. Preferably, a first enzyme composition is sufficient to reduce inhibitors or reduce viscosity in a substrate-containing slurry. In a further embodiment the first enzyme composition is sufficient to both reduce inhibitors and reduce viscosity in a substrate-containing slurry. For example, a first enzyme composition of the invention comprises a xylanase, a beta-xylosidase and a cellulase. In the invention the cellulase is an endoglucanase. In a further example a first enzyme composition comprises at least endoglucanase. A multi-stage hydrolysis process of the invention further comprises one or more additional enzyme compositions. In the invention, the process further comprises administration of a second enzyme composition comprising one or more cellulases.

One or more (e.g., several) components of the enzyme compositions may be native proteins, recombinant proteins, or a combination of native proteins and recombinant proteins. For example, one or more (*e.g.*, several) components may be native proteins of a cell, which is used as a host cell to express recombinantly one or more (*e.g.*, several) other components of the enzyme compositions. It is understood herein that the recombinant proteins may be heterologous (*e.g.,* foreign) and/or native to the host cell. One or more (*e.g.,* several) components of the enzyme compositions may be produced as monocomponents, which are then combined to form the enzyme compositions. The enzyme compositions may be a combination of multicomponent and monocomponent protein preparations. The compositions may be further combined with one or more additional enzyme compositions.

The enzymes used in processes of the present invention may be in any form suitable for use, such as, for example, a fermentation broth formulation or a cell composition, a cell lysate with or without cellular debris, a semi-purified or purified enzyme composition, or a host cell as a source of the enzymes. The enzyme compositions may be a dry powder or granulate, a non-dusting granulate, a liquid, a stabilized liquid, or a stabilized protected enzyme. Liquid enzyme compositions may, for instance, be stabilized by adding stabilizers such as a sugar, a sugar alcohol or another polyol, and/or lactic acid or another organic acid according to established processes.

The optimum amounts of the enzymes and polypeptides depend on several factors including, but not limited to, the mixture of cellulolytic enzymes and/or hemicellulolytic enzymes, the cellulosic material, the concentration of cellulosic material, the pretreatment of the cellulosic material, temperature, time, pH, and inclusion of a fermenting organism (*e.g.*, for Simultaneous Saccharification and Fermentation).

For example, a first enzyme composition of the invention comprises a xylanase, a beta-xylosidase and a cellulase. In the invention the cellulase is an endoglucanase. The invention provides a multi-stage hydrolysis process, wherein a first enzyme composition is added in a first stage of hydrolysis and a second enzyme composition is added in a second or subsequent stage of hydrolysis, following administration of a first enzyme composition in a prior stage of hydrolysis. In the invention a first enzyme composition is administered in a first stage of hydrolysis in a continuous reactor.

The first enzyme composition is present as about 1% to about 99%, *e.g.*, about 1% to about 45%, about 2% to about 40%, or about 5% to about 35% of the total enzyme protein added during hydrolysis.

The second enzyme composition is present as about 1% to about 99%, e.g., about 55% to about 99%, about 60% to about 98%, or about 65% to about 95% of the total enzyme protein added during hydrolysis. In a preferred embodiment, the combined first enzyme composition and second enzyme composition comprise 100% of the total enzyme protein added during hydrolysis. In another embodiment, the ratio of enzyme protein of a first enzyme composition to enzyme protein of a second enzyme composition by weight is about 1:2.

In one embodiment, an effective amount of total enzyme protein added during hydrolysis to the cellulosic material is about 0.5 to about 15 mg, e.g., about 0.5 to about 10 mg, about 0.5 to about 9 mg, about 0.5 to about 6 mg, or about 0.5 to about 5 mg per g of the cellulosic material. In a preferred embodiment the total enzyme protein added during hydrolysis comprising all enzyme compositions added in all stages of hydrolysis is about 4 to about 10 mg, or about 4 to about 7 mg per g of the cellulosic material.

The enzymes may be present or added during hydrolysis (i.e., saccharification) in amounts effective from about 0.001 to about 5.0 wt % of solids (TS), more preferably from about 0.025 to about 4.0 wt % of solids, and most preferably from about 0.005 to about 2.0 wt % of solids (TS).

The enzymes in enzyme compositions may be derived or obtained from any suitable origin, including, archaeal, bacterial, fungal, yeast, plant, or animal origin. The term "obtained" also means herein that the enzyme may have been produced recombinantly in a host organism employing methods described herein, wherein the recombinantly produced enzyme is either native or foreign to the host organism or has a modified amino acid sequence, e.g., having one or more (*e.g.,* several) amino acids that are deleted, inserted and/or substituted, *i.e.*, a recombinantly produced enzyme that is a mutant and/or a fragment of a native amino acid sequence or an enzyme produced by nucleic acid shuffling processes known in the art. Encompassed within the meaning of a native enzyme are natural variants and within the meaning of a foreign enzyme are variants obtained by, e.g., site-directed mutagenesis or shuffling.

Each polypeptide may be a bacterial polypeptide. For example, each polypeptide may be a Gram-positive bacterial polypeptide having enzyme activity, or a Gram-negative bacterial polypeptide having enzyme activity.

Each polypeptide may also be a fungal polypeptide, e.g., a yeast polypeptide or a filamentous fungal polypeptide.

Chemically modified or protein engineered mutants of polypeptides may also be used.

One or more (*e.g.*, several) components of the enzyme compositions may be a recombinant component, *i.e.,* produced by cloning of a DNA sequence encoding the single component and subsequent cell transformed with the DNA sequence and expressed in a host (see, for example, WO 91/17243 and WO 91/17244). The host may be a heterologous host (enzyme is foreign to host), but the host may under certain conditions also be a homologous host (enzyme is native to host). Monocomponent cellulolytic proteins may also be prepared by purifying such a protein from a fermentation broth.

For example, a first enzyme composition comprises a xylanase, a beta-xylosidase and a cellulase. In the invention the cellulase is an endoglucanase.

For example, the first enzyme composition has endo-acting and exo-acting activity in hydrolysis of xylans. In a further example the first enzyme composition has activity in viscosity reduction.

Examples of xylanases useful in the processes of the present invention include, but are not limited to, xylanases from *Aspergillus aculeatus* (GENSEQP™ Accession No. AAR63790; WO 94/21785), *Aspergillus fumigatus* (WO 2006/078256), *Penicillium pinophilum* (WO 2011/041405), *Penicillium* sp. (WO 2010/126772), *Talaromyces lanuginosus* GH11 (WO 2012/130965), *Talaromyces leycettanus* GH10 (GENSEQP™ Accession No. BAK46118), *Talaromyces*

*thermophilus* GH11 (WO 2012/130950), *Thielavia terrestris* NRRL 8126 (WO 2009/079210), and *Trichophaea saccata* GH10 (WO 2011/057083).

In one embodiment the xylanase is selected from the group consisting of: (i) a xylanase comprising or consisting of the mature polypeptide of SEQ ID NO: 12; (ii) a xylanase comprising or consisting of an amino acid sequence having at least 70%, e.g., at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or least 99% sequence identity to the mature polypeptide of SEQ ID NO: 12; (iii) a xylanase encoded by a polynucleotide comprising or consisting of a nucleotide sequence having at least 70%, e.g., at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or least 99% sequence identity to the mature polypeptide coding sequence of SEQ ID NO: 11; and (iv) a xylanase encoded by a polynucleotide that hybridizes under at least high stringency conditions, e.g., very high stringency conditions, with the mature polypeptide coding sequence of SEQ ID NO: 11 or the full-length complement thereof.

In another embodiment the xylanase is selected from the group consisting of: (i) a xylanase comprising or consisting of the mature polypeptide of SEQ ID NO: 14; (ii) a xylanase comprising or consisting of an amino acid sequence having at least 70%, e.g., at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or least 99% sequence identity to the mature polypeptide of SEQ ID NO: 14; (iii) a xylanase encoded by a polynucleotide comprising or consisting of a nucleotide sequence having at least 70%, e.g., at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or least 99% sequence identity to the mature polypeptide coding sequence of SEQ ID NO: 13; and (iv) a xylanase encoded by a polynucleotide that hybridizes under at least high stringency conditions, e.g., very high stringency conditions, with the mature polypeptide coding sequence of SEQ ID NO: 13 or the full-length complement thereof;

In another embodiment the xylanase is selected from the group consisting of: (i) a xylanase comprising or consisting of the mature polypeptide of SEQ ID NO: 22; (ii) a xylanase comprising or consisting of an amino acid sequence having at least 70%, e.g., at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or least 99% sequence identity to the mature polypeptide of SEQ ID NO: 22; (iii) a xylanase encoded by a polynucleotide comprising or consisting of a nucleotide sequence having at least 70%, e.g., at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or least 99% sequence identity to the mature polypeptide coding sequence of SEQ ID NO: 21; and (iv) a xylanase encoded by a polynucleotide that hybridizes under at least high stringency conditions, e.g., very high stringency conditions, with the mature polypeptide coding sequence of SEQ ID NO: 21 or the full-length complement thereof.

Examples of beta-xylosidases useful in the processes of the present invention include, but are not limited to, beta-xylosidases from *Aspergillus fumigatus* (GENSEQP™ Accession No. AZI05042; WO 2013/028928), *Neurospora crassa* (SwissProt:Q7SOW4), *Talaromyces emersonii* (SwissProt:Q8X212), *Trichoderma reesei* (UniProtKB/TrEMBL:Q92458), and *Trichoderma reesei* such as the mature polypeptide of GENSEQP™ Accession No. AZI04896.

In one embodiment the beta-xylosidase is selected from the group consisting of: (i) a beta-xylosidase comprising or consisting of the mature polypeptide of SEQ ID NO: 16; (ii) a beta-xylosidase comprising or consisting of an amino acid sequence having at least 70%, e.g., at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or least 99% sequence identity to the mature polypeptide of SEQ ID NO: 16; (iii) a beta-xylosidase encoded by a polynucleotide comprising or consisting of a nucleotide sequence having at least 70%, e.g., at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or least 99% sequence identity to the mature polypeptide coding sequence of SEQ ID NO: 15; and (iv) a beta-xylosidase encoded by a polynucleotide that hybridizes under at least high stringency conditions, e.g., very high stringency conditions, with the mature polypeptide coding sequence of SEQ ID NO: 15 or the full-length complement thereof.

In another embodiment the beta-xylosidase is selected from the group consisting of: (i) a beta-xylosidase comprising or consisting of the mature polypeptide of SEQ ID NO: 24; (ii) a beta-xylosidase comprising or consisting of an amino acid sequence having at least 70%, e.g., at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or least 99% sequence identity to the mature polypeptide of SEQ ID NO: 24; (iii) a beta-xylosidase encoded by a polynucleotide comprising or consisting of a nucleotide sequence having at least 70%, e.g., at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or least 99% sequence identity to the mature polypeptide coding sequence of SEQ ID NO: 23; and (iv) a beta-xylosidase encoded by a polynucleotide that hybridizes under at least high stringency conditions, e.g., very high stringency conditions, with the mature polypeptide coding sequence of SEQ ID NO: 23 or the full-length complement thereof.

Examples of bacterial endoglucanases that may be used in the present invention, include, but are not limited to, *Acidothermus cellulolyticus* endoglucanase (WO 91/05039; WO 93/15186; U.S. Patent No. 5,275,944; WO 96/02551; U.S. Patent No. 5,536,655; WO 00/70031; WO 05/093050), *Erwinia carotovara* endoglucanase (Saarilahti et al., 1990, Gene 90: 9-14), *Thermobifida fusca* endoglucanase III (WO 05/093050), and *Thermobifida fusca* endoglucanase V (WO 05/093050).

Examples of fungal endoglucanases that may be used in the present invention, include, but are not limited to, *Aspergillus aculeatus* endoglucanase (Ooi et al., 1990, Nucleic Acids Research 18: 5884), *Aspergillus kawachii* endoglucanase (Sakamoto et al., 1995, Current Genetics 27: 435-439), *Fusarium oxysporum* endoglucanase (GenBank:L29381), *Humicola grisea* var. *thermoidea* endoglucanase (GenBank:AB003107), *Humicola insolens* endoglucanase V, *Melanocarpus albomyces* endoglucanase (GenBank:MAL515703), *Myceliophthora thermophila* CBS 117.65 endoglucanase, *Neurospora crassa* endoglucanase (GenBank:XM_324477), *Thermoascus aurantiacus* endoglucanase I (GenBank:AF487830), *Thermoascus aurantiacus* Cel5 endoglucanase II (WO2011/057140), *Trichoderma reesei* endoglucanase I (Penttila et al., 1986, Gene 45: 253-263), *Trichoderma reesei* Cel7B endoglucanase I (GenBank:M15665), *Trichoderma reesei* endoglucanase II (Saloheimo et al., 1988, Gene 63:11-22), *Trichoderma reesei* Cel5A endoglucanase II (GenBank:M19373), *Trichoderma reesei* endoglucanase III (Okada et al., 1988, Appl. Environ. Microbiol. 64: 555-563, GenBank:AB003694), *Trichoderma reesei* endoglucanase V (Saloheimo et al., 1994, Molecular Microbiology 13: 219-228, GenBank:Z33381), and *Trichoderma reesei* strain No. VTT-D-80133 endoglucanase (GenBank:M15665).

In another embodiment, an enzyme composition of the invention further or even further comprises a *Trichoderma* endoglucanase I or a homolog thereof. In another aspect, an enzyme composition further comprises a *Trichoderma reesei* endoglucanase I or a homolog thereof. In another aspect, an enzyme composition further comprises a *Trichoderma reesei* Cel7B endoglucanase I (GENBANK™ accession no. M15665) or a homolog thereof. In another aspect, the *Trichoderma reesei* endoglucanase I or a homolog thereof is native to the host cell.

In another aspect, an enzyme composition of the invention further or even further comprises a *Trichoderma* endoglucanase II or a homolog thereof. In another aspect, an enzyme composition further comprises a *Trichoderma reesei* endoglucanase II or a homolog thereof. In another aspect, an enzyme composition further comprises a *Trichoderma reesei* Cel5A endoglucanase II (GENBANK™ accession no. M19373) or a homolog thereof. In another aspect, the *Trichoderma reesei* endoglucanase II or a homolog thereof is native to the host cell.

In one embodiment the endoglucanase is selected from the group consisting of: (i) an endoglucanase comprising or consisting of the mature polypeptide of SEQ ID NO: 20; (ii) an endoglucanase comprising or consisting of an amino acid sequence having at least 70%, e.g., at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or least 99% sequence identity to the mature polypeptide of SEQ ID NO: 20; (iii) an endoglucanase encoded by a polynucleotide comprising or consisting of a nucleotide sequence having at least 70%, e.g., at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or least 99% sequence identity to the mature polypeptide coding sequence of SEQ ID NO: 19; and (iv) an endoglucanase encoded by a polynucleotide that hybridizes under at least high stringency conditions, e.g., very high stringency conditions, with the mature polypeptide coding sequence of SEQ ID NO: 19 or the full-length complement thereof.

A first enzyme composition comprises a cellulase sufficient to reduce viscosity of the substrate-containing slurry. In a preferred embodiment endoglucanase is present in a first enzyme composition as about 0.5 to about 100% of the total enzyme protein added during hydrolysis, e.g., about 1% to about 90%, about 5% to about 50%, about 7% to about 25%, or about 7% to about 11%. In one embodiment endoglucanase is about 30% to about 50% of the enzyme protein in the first enzyme composition.

The amount of cellulase in the first enzyme composition may be determined as described in Example 8 and measured in U/mg total enzyme. Therefore, in an embodiment, the effective amount of endoglucanase in a first enzyme composition of the present invention is about 2.84 U to about 117.2 U, e.g., about 3.9 U to about 117.2 U, about 31.2 to about 91.3, about 3.9 U to about 78.1 U, about 3.9 U to about 70.3 U, about 3.9 U to about 46.9 U, or about 3.9 U to about 39.2 U per g of the lignocellulosic material.

In an embodiment, the amount of xylanase in a first enzyme composition of the present invention is 0% to 30% of the total enzyme protein added during hydrolysis, e.g., 0.5% to 30%, 1.0% to 27.5%, 1.5% to 25%, 2% to 22.5%, 2.5% to 20%, 3% to 19%, 3.5% to 18%, and 4% to 17% of the total enzyme protein added during hydrolysis.

The amount of xylanase may be determined as described in Example 9 and measured in U/mg total enzyme. Therefore in an embodiment, the effective amount of xylanase in a first enzyme composition of the present invention is about 4.3 U to about 716.1 U, e.g., about 23.8 U to about 716.1 U, about 23.8 U to about 477.4 U, about 190.9 U to about 477.4 U, about 23.8 U to about 429.7 U, about 23.8 U to about 286.4 U, or about 23.8 U to about 238.7 U per g of the lignocellulosic material.

In another embodiment, the amount of beta-xylosidase in a first enzyme composition of the present invention is 0% to 50% of the total enzyme protein added during hydrolysis, e.g., 0.5% to 30%, 1.0% to 27.5%, 1.5% to 25%, 2% to 22.5%, 2.5% to 22%, 3% to 19%, 3.5% to 18%, and 4% to 17% of the total enzyme protein added during hydrolysis.

The amount of beta-xylosidase may be determined as described in Example 10 and measured in U/mg total enzyme. Therefore in an embodiment, the effective amount of beta-xylosidase in a first enzyme composition of the present invention is about 0.005 U to about 0.86 U, e.g., about 0.03 U to about 0.86 U, about 0.03 U to about 0.57 U, about 0.23 U to about 0.57 U, about 0.03 U to about 0.51 U, about 0.03 U to about 0.34 U, or about 0.03 U to about 0.29 U per g of the lignocellulosic material.

In still another embodiment, the amount of xylanase and beta-xylosidase in a first enzyme composition, taken together, is about 0.5 to about 100% of the total enzyme protein added during hydrolysis, e.g., about 1% to about 90%, about 5% to about 50%, about 15% to about 25%, about 20% to about 23%. In one embodiment the xylanase and beta-xylosidase in a first enzyme composition, taken together, is about 40% to about 70% of the enzyme protein in the first enzyme composition.

In a particular embodiment a first enzyme composition is derived from *Trichoderma reesei,* further comprising a xylanase of SEQ ID NO: 12, 14 or 22, a beta xylosidase of SEQ ID NO: 16 or 24, and an endoglucanase of SEQ ID NO: 20.

In an embodiment the first enzyme composition comprises a commercial hemicellulolytic enzyme composition. Examples of commercial hemicellulolytic enzyme compositions suitable for use in the present invention include, for example, SHEARZYME™ (Novozymes A/S), CELLIC® HTec (Novozymes A/S), CELLIC® HTec2 (Novozymes A/S), CELLIC® HTec3 (Novozymes A/S), VISCOZYME® (Novozymes A/S), ULTRAFLO® (Novozymes A/S), PULPZYME® HC (Novozymes A/S), MULTIFECT® Xylanase (Danisco US Inc.), ACCELLERASE® XY (Danisco US Inc.), ACCELLERASE® XC (Danisco US Inc.), ACCELLERASE® TRIO (Danisco US Inc.), ECOPULP® TX-200A (Roal Oy LLC), HSP 6000 Xylanase (DSM), DEPOL™ 333P (Biocatalysts Limit, Wales, UK), DEPOL™ 740L. (Biocatalysts Limit, Wales, UK), and DEPOL™ 762P (Biocatalysts Limit, Wales, UK), ALTERNA FUEL 100P (Dyadic), and ALTERNA FUEL 200P (Dyadic).

In a further embodiment a second enzyme composition comprises a cellulolytic enzyme composition comprising one or more (*e.g.*, several) cellulolytic enzymes. In another embodiment, the enzymes in the second enzyme composition comprise or further comprise one or more (*e.g.*, several) hemicellulolytic enzymes. In another embodiment, the enzymes in the second enzyme composition comprise one or more (*e.g.*, several) cellulolytic enzymes and one or more (*e.g.*, several) hemicellulolytic enzymes. In another embodiment, the enzymes in the second enzyme composition comprise one or more (*e.g.*, several) enzymes selected from the group of cellulolytic enzymes and hemicellulolytic enzymes. In another embodiment, the enzymes in the second enzyme composition comprise a cellobiohydrolase. In a further embodiment the cellobiohydrolase is a cellobiohydrolase I, a cellobiohydrolase II, or a combination of a cellobiohydrolase I and a cellobiohydrolase II. In another embodiment, the enzymes in the second enzyme composition comprise a beta-glucosidase. In another embodiment, the enzymes in the second enzyme composition comprise an AA9 polypeptide. In another embodiment, the enzymes in the the second enzyme composition comprise an endoglucanase. In still another embodiment the enzymes in the second enzyme composition comprise a xylanase. In a still further embodiment the enzymes in the second enzyme composition comprise a beta-xylosidase.

In a further embodiment the second enzyme composition comprises enzymes selected from the group consisting of a cellobiohydrolase, a beta glucosidase and an AA9 polypeptide having cellulolytic enhancing activity.

Examples of cellobiohydrolases useful in the present invention include, but are not limited to, *Aspergillus aculeatus* cellobiohydrolase II (WO 2011/059740), *Aspergillus fumigatus* cellobiohydrolase I (GENSEQP™ Accession No. AZI04842), *Aspergillus fumigatus* cellobiohydrolase II (GENSEQP™ Accession No. AZI04854), *Chaetomium thermophilum* cellobiohydrolase I, *Chaetomium thermophilum* cellobiohydrolase II, *Humicola insolens* cellobiohydrolase I, *Myceliophthora thermophila* cellobiohydrolase II (WO 2009/042871), *Penicillium occitanis* cellobiohydrolase I (GenBank:AY690482), *Talaromyces emersonii* cellobiohydrolase I (GenBank:AF439936), *Talaromyces leycettanus* cellobiohydrolase I (GENSEQP™ Accession No. AZY49536), *Talaromyces leycettanus* cellobiohydrolase II (GENSEQP™ Accession No. AZY49446), *Thielavia hyrcanie* cellobiohydrolase II (WO 2010/141325), *Thielavia terrestris* cellobiohydrolase II (CEL6A, WO 2006/074435), *Trichoderma reesei* cellobiohydrolase I, *Trichoderma reesei* cellobiohydrolase II, and *Trichophaea saccata* cellobiohydrolase II (WO 2010/057086).

Examples of beta-glucosidases useful in the present invention include, but are not limited to, beta-glucosidases from *Aspergillus aculeatus* (Kawaguchi et al., 1996, Gene 173: 287-288), *Aspergillus fumigatus* (GENSEQP™ Accession No. AEA33202), an *Aspergillus fumigatus* variant such as GENSEQP™ Accession No. AZU67153, *Aspergillus niger* (Dan et al., 2000, J. Biol. Chem. 275: 4973-4980), *Aspergillus oryzae* (WO 02/095014) or the fusion protein having beta-glucosidase activity disclosed in WO 2008/057637, *Penicillium brasilianum* IBT 20888 (WO 2007/019442 and WO 2010/088387), *Thielavia terrestris* (WO 2011/035029), *Trichophaea saccata* (WO 2007/019442) and *Trichoderma reesei.*

Other useful endoglucanases, cellobiohydrolases, and beta-glucosidases are disclosed in numerous Glycosyl Hydrolase families using the classification according to Henrissat, 1991, Biochem. J. 280: 309-316, and Henrissat and Bairoch, 1996, Biochem. J. 316: 695-696.

In the processes of the present invention, any AA9 polypeptide may be used as a component of an enzyme composition.

Examples of AA9 polypeptides useful in the processes of the present invention include, but are not limited to, AA9 polypeptides from *Aspergillus aculeatus* (WO 2012/125925), *Aspergillus fumigatus* (WO 2010/138754), *Aurantiporus alborubescens* (WO 2012/122477), *Chaetomium thermophilum* (WO 2012/101206), *Humicola insolens* (WO 2012/146171), *Malbranchea cinnamomea* (WO 2012/101206), *Myceliophthora thermophila* (WO 2009/085935, WO 2009/085859, WO 2009/085864, WO 2009/085868, WO 2009/033071, WO 2012/027374, and WO 2012/068236), *Penicillium pinophilum* (WO 2011/005867), *Penicillium thomii* (WO 2012/122477), *Penicillium* sp. (*emersonii*) (WO 2011/041397 and WO 2012/000892), *Talaromyces emersonii* (WO 2012/000892), *Talaromyces leycettanus* (WO 2012/101206), *Talaromyces stipitatus* (WO 2012/135659), *Talaromyces thermophilus* (WO 2012/129697 and WO 2012/130950), *Thermoascus aurantiacus* (WO 2005/074656 and WO 2010/065830), *Thermoascus crustaceous* (WO 2011/041504), *Thermoascus* sp. (WO 2011/039319), *Thermomyces lanuginosus* (WO 2012/113340, WO 2012/129699, WO 2012/130964, and WO 2012/129699), *Thielavia terrestris* (WO 2005/074647, WO 2008/148131, and WO 2011/035027), *Trametes versicolor* (WO 2012/092676 and WO 2012/093149), *Trichoderma reesei* (WO 2007/089290 and WO 2012/149344), and *Trichophaea saccata* (WO 2012/122477).

In one embodiment, the AA9 polypeptide is used in the presence of a soluble activating divalent metal cation according to WO 2008/151043, e.g., copper.

In another embodiment, the AA9 polypeptide is used in the presence of a dioxy compound, a bicylic compound, a heterocyclic compound, a nitrogen-containing compound, a quinone compound, a sulfur-containing compound, or a liquor obtained from a pretreated cellulosic material such as pretreated corn stover (WO 2012/021394, WO 2012/021395, WO 2012/021396, WO 2012/021399, WO 2012/021400, WO 2012/021401, WO 2012/021408, and WO 2012/021410).

In one embodiment, such a compound is added at a molar ratio of the compound to glucosyl units of cellulose of about $10^{-6}$ to about 10, e.g., about $10^{-6}$ to about 7.5, about $10^{-6}$ to about 5, about $10^{-6}$ to about 2.5, about $10^{-6}$ to about 1, about $10^{-5}$ to about 1, about $10^{-5}$ to about $10^{-1}$, about $10^{-4}$ to about $10^{-1}$, about $10^{-3}$ to about $10^{-1}$, or about $10^{-3}$ to about $10^{-2}$. In another embodiment, an effective amount of such a compound is about 0.1 $\mu$M to about 1 M, e.g., about 0.5 $\mu$M to about 0.75 M, about 0.75 $\mu$M to about 0.5 M, about 1 $\mu$M to about 0.25 M, about 1 $\mu$M to about 0.1 M, about 5 $\mu$M to about 50 mM, about 10 $\mu$M to about 25 mM, about 50 $\mu$M to about 25 mM, about 10 $\mu$M to about 10 mM, about 5 $\mu$M to about 5 mM, or about 0.1 mM to about 1 mM.

The term "liquor" means the solution phase, either aqueous, organic, or a combination thereof, arising from treatment of a lignocellulose and/or hemicellulose material in a slurry, or monosaccharides thereof, *e.g.*, xylose, arabinose, mannose, etc., under conditions as described in WO 2012/021401, and the soluble contents thereof. A liquor for cellulolytic enhancement of an AA9 polypeptide may be produced by treating a lignocellulose or hemicellulose material (or feedstock) by applying heat and/or pressure, optionally in the presence of a catalyst, *e.g.*, acid, optionally in the presence of an organic solvent, and optionally in combination with physical disruption of the material, and then separating the solution from the residual solids. Such conditions determine the degree of cellulolytic enhancement obtainable through the combination of liquor and an AA9 polypeptide during hydrolysis of a cellulosic substrate by a cellulolytic enzyme composition. The liquor may be separated from the treated material using a method standard in the art, such as filtration, sedimentation, or centrifugation.

In one embodiment, an effective amount of the liquor to cellulose is about $10^{-6}$ to about 10 g per g of cellulose, e.g., about $10^{-6}$ to about 7.5 g, about $10^{-6}$ to about 5 g, about $10^{-6}$ to about 2.5 g, about $10^{-6}$ to about 1 g, about $10^{-5}$ to about 1 g, about $10^{-5}$ to about $10^{-1}$ g, about $10^{-4}$ to about $10^{-1}$ g, about $10^{-3}$ to about $10^{-1}$ g, or about $10^{-3}$ to about $10^{-2}$ g per g of cellulose.

In an embodiment, a second enzyme composition comprises a cellobiohydrolase I, a cellobiohydrolase II, a beta-glucosidase or variant thereof, and an AA9 polypeptide. In a particular embodiment a second enzyme composition is derived from *Trichoderma reesei,* further comprising AA9 (GH61) polypeptide having cellulolytic enhancing activity set forth as SEQ ID NO: 2 in WO 2011/041397, a beta-glucosidase (SEQ ID NO: 2 in WO 2005/047499) variant (F100D, S283G, N456E, F512Y) set forth in WO 2012/044915; a CBH I set forth as SEQ ID NO: 6 in WO 2011/057140 and a CBH II set forth as SEQ ID NO: 18 in WO 2011/057140.

In a further embodiment a second enzyme composition comprises one or more hemicellulases. In an embodiment, the hemicellulase is preferably one or more (e.g., several) enzymes selected from the group consisting of an acetylmannan esterase, an acetylxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase.

Examples of xylanases and xylosidases are as set forth herein with respect to the first enzyme composition.

Examples of acetylxylan esterases useful in the processes of the present invention include, but are not limited to, acetylxylan esterases from *Aspergillus aculeatus* (WO 2010/108918), *Chaetomium globosum* (UniProt:Q2GWX4), *Chaetomium gracile* (GeneSeqP:AAB82124), *Humicola insolens* DSM 1800 (WO 2009/073709), *Hypocrea jecorina* (WO 2005/001036), *Myceliophtera thermophila* (WO 2010/014880), *Neurospora crassa* (UniProt:q7s259), *Phaeosphaeria nodorum* (UniProt:Q0UHJ1), and *Thielavia terrestris* NRRL 8126 (WO 2009/042846).

Examples of feruloyl esterases (ferulic acid esterases) useful in the processes of the present invention include, but are not limited to, feruloyl esterases from *Humicola insolens* DSM 1800 (WO 2009/076122), *Neosartorya fischeri* (UniProt:A1D9T4), *Neurospora crassa* (UniProt:Q9HGR3), *Penicillium aurantiogriseum* (WO 2009/127729), and *Thielavia terrestris* (WO 2010/053838 and WO 2010/065448).

Examples of arabinofuranosidases useful in the processes of the present invention include, but are not limited to, arabinofuranosidases from *Aspergillus niger* (GeneSeqP:AAR94170), *Humicola insolens* DSM 1800 (WO 2006/114094 and WO 2009/073383), and *M. giganteus* (WO 2006/114094).

Examples of alpha-glucuronidases useful in the processes of the present invention include, but are not limited to, alpha-glucuronidases from *Aspergillus clavatus* (UniProt:alcc12), *Aspergillus fumigatus* (SwissProt:Q4WW45), *Aspergillus niger* (UniProt:Q96WX9), *Aspergillus terreus* (SwissProt:Q0CJP9), *Humicola insolens* (WO 2010/014706), *Penicillium aurantiogriseum* (WO 2009/068565), *Talaromyces emersonii* (UniProt:Q8X211), and *Trichoderma reesei* (UniProt:Q99024).

In a still further embodiment a second enzyme composition comprises one or more oxidoreductases. Examples of oxidoreductases useful in the processes of the present invention include, but are not limited to, *Aspergillus fumigatus* catalase, *Aspergillus lentilus* catalase, *Aspergillus niger* catalase, *Aspergillus oryzae* catalase, *Humicola insolens* catalase, *Neurospora crassa* catalase, *Penicillium emersonii* catalase, *Scytalidium thermophilum* catalase, *Talaromyces stipitatus* catalase, *Thermoascus aurantiacus* catalase, *Coprinus cinereus* laccase, *Myceliophthora thermophila* laccase, *Polyporus pinsitus* laccase, *Pycnoporus cinnabarinus* laccase, *Rhizoctonia solani* laccase, *Streptomyces coelicolor* laccase, *Coprinus cinereus* peroxidase, Soy peroxidase, Royal palm peroxidase.

In an embodiment the second enzyme composition is or comprises a commercial cellulolytic enzyme preparation. Examples of commercial cellulolytic enzyme preparations suitable for use in the present invention include, for example, CELLIC® CTec (Novozymes A/S), CELLIC® CTec2 (Novozymes A/S), CELLIC® Ctec3 (Novozymes A/S), CELLUCLAST® (Novozymes A/S), CELLUZYME™ (Novozymes A/S), CEREFLO® (Novo Nordisk A/S), and ULTRAFLO® (Novozymes A/S), ACCELLERASE® (Danisco US Inc.), LAMINEX® (Danisco US Inc.), SPEZYME® CP (Danisco US Inc.), ROHAMENT® 7069 W (AB Enzymes), FIBREZYME® LDI (Dyadic International, Inc.), FIBREZYME® LBR (Dyadic International, Inc.), or VISCOSTAR™ 150L (Dyadic International, Inc.).

In one embodiment, the amount of cellobiohydrolase I in a second enzyme composition of the present invention is 5% to 60% of the total enzyme protein added during hydrolysis, e.g., 7.5% to 55%, 10% to 50%, 12.5% to 45%, 15% to 40%, 17.5% to 35%, and 20% to 30% of the total enzyme protein added during hydrolysis.

In another embodiment, the amount of cellobiohydrolase II in a second enzyme composition of the present invention is 2.0-40% of the total enzyme protein added during hydrolysis, e.g., 3.0% to 35%, 4.0% to 30%, 5% to 25%, 6% to 20%, 7% to 15%, and 7.5% to 12% of the total enzyme protein added during hydrolysis.

In another embodiment, the amount of beta-glucosidase in a second enzyme composition of the present invention is 0% to 30% of the total enzyme protein added during hydrolysis, e.g., 1% to 27.5%, 1.5% to 25%, 2% to 22.5%, 3% to 20%, 4% to 19%, % 4.5 to 18%, 5% to 17%, and 6% to 16% of the total enzyme protein added during hydrolysis.

In another embodiment, the amount of AA9 polypeptide in a second enzyme composition of the present invention is 0% to 50% of the total enzyme protein added during hydrolysis, e.g., 2.5% to 45%, 5% to 40%, 7.5% to 35%, 10% to 30%, 12.5% to 25%, and 15% to 25% of the total enzyme protein added during hydrolysis.

In one embodiment the components of the first enzyme composition are mixed or blended prior to addition to the reactor. In another embodiment, the components of the second enzyme composition are mixed or blended prior to addition to the reactor. In the invention the first enzyme composition and the second enzyme composition are added in different stages of hydrolysis in a multi-stage hydrolysis process. For example, the first enzyme composition, or component parts thereof, is added to a reactor before, concurrent with, or after addition of the lignocellulosic material to the reactor. For example, the second enzyme composition, or component parts thereof, is added to a reactor after addition of the lignocellulosic material and the first enzyme composition to the reactor.

One or more (e.g., several) of the enzymes added during hydrolysis may be wild-type proteins expressed by the host strain, recombinant proteins, or a combination of wild-type proteins expressed by the host strain and recombinant proteins. For example, one or more (e.g., several) enzymes may be native proteins of a cell, which is used as a host cell to express recombinantly the enzymes added during hydrolysis.

The enzyme compositions may be prepared in accordance with methods known in the art and may be in the form of a liquid or a dry composition. The compositions may be stabilized in accordance with methods known in the art.

The enzyme compositions may result from a single fermentation or may be a blend of two or more fermentations, *e.g.*, three, four, five, six, seven, etc. fermentations.

The enzyme compositions may be in any form suitable for use, such as, for example, a crude fermentation broth with or without cells removed, a cell lysate with or without cellular debris, a semi-purified or purified enzyme composition, or a *Trichoderma* host cell as a source of the enzymes. The enzyme compositions may be a dry powder or granulate, a non-dusting granulate, a liquid, a stabilized liquid, or a stabilized protected enzyme. Liquid enzyme compositions may, for instance, be stabilized by adding stabilizers such as a sugar, a sugar alcohol or another polyol, and/or lactic acid or another organic acid according to established processes.

The enzyme compositions may also be a fermentation broth formulation or a cell composition. The fermentation broth product further comprises additional ingredients used in the fermentation process, such as, for example, cells (including, the host cells containing the gene encoding the polypeptide of the present invention which are used to produce the polypeptide), cell debris, biomass, fermentation media and/or fermentation products. In some embodiments, the composition is a cell-killed whole broth containing organic acid(s), killed cells and/or cell debris, and culture medium.

The term "fermentation broth" refers to a composition produced by cellular fermentation that undergoes no or minimal recovery and/or purification. For example, fermentation broths are produced when microbial cultures are grown to saturation, incubated under carbon-limiting conditions to allow protein synthesis (*e.g.*, expression of enzymes by host cells) and secretion into cell culture medium. The fermentation broth can contain unfractionated or fractionated contents of the fermentation materials derived at the end of the fermentation. Typically, the fermentation broth is unfractionated and comprises the spent culture medium and cell debris present after the microbial cells (*e.g.,* filamentous fungal cells) are removed, *e.g.,* by centrifugation. For example, the fermentation broth contains spent cell culture medium, extracellular enzymes, and viable and/or nonviable microbial cells.

For example, the fermentation broth formulation and cell compositions comprise a first organic acid component comprising at least one 1-5 carbon organic acid and/or a salt thereof and a second organic acid component comprising at least one 6 or more carbon organic acid and/or a salt thereof. In a specific example the first organic acid component is acetic acid, formic acid, propionic acid, a salt thereof, or a mixture of two or more of the foregoing and the second organic acid component is benzoic acid, cyclohexanecarboxylic acid, 4-methylvaleric acid, phenylacetic acid, a salt thereof, or a mixture of two or more of the foregoing.

In one example the composition contains an organic acid(s), and optionally further contains live cells, killed cells and/or cell debris. In one example, the composition comprises live cells. In another example, killed cells, and/or cell debris are removed from a cell-killed whole broth to provide a composition that is free of these components.

The fermentation broth formulations or cell compositions may further comprise a preservative and/or anti-microbial (*e.g.*, bacteriostatic) agent, including, but not limited to, sorbitol, sodium chloride, potassium sorbate, and others known in the art.

The cell-killed whole broth or composition may contain the unfractionated contents of the fermentation materials derived at the end of the fermentation. Typically, the cell-killed whole broth or composition contains the spent culture medium and cell debris present after the microbial cells (*e.g.*, filamentous fungal cells) are grown to saturation, incubated under carbon-limiting conditions to allow protein synthesis (*e.g.*, expression of cellulase and/or glucosidase enzyme(s)). In some examples, the cell-killed whole broth or composition contains the spent cell culture medium, extracellular enzymes, and killed filamentous fungal cells. In some examples, the microbial cells present in the cell-killed whole broth or composition can be permeabilized and/or lysed using methods known in the art.

A whole broth or cell composition as described herein is typically a liquid slurry, but may contain insoluble components, such as killed cells, cell debris, culture media components, and/or insoluble enzyme(s). In some examples, insoluble components may be removed to provide a clarified liquid composition.

The whole broth formulations and cell compositions may be produced by the methods described in WO 90/15861 or WO 2010/096673.

The fermentation may be any method of cultivation of a cell resulting in the expression or isolation of an enzyme or protein. Fermentation may, therefore, be understood as comprising shake flask cultivation, or small-scale or large-scale fermentation (including continuous, batch, fed-batch, or solid state fermentations) in laboratory or industrial fermentors performed in a suitable medium and under conditions allowing the enzyme to be expressed or isolated. The resulting enzymes produced by the methods described above may be recovered from the fermentation medium and purified by conventional procedures.

**Fermentation**

The fermentable sugars obtained from the hydrolyzed cellulosic material may be fermented by one or more (*e.g.*, several) fermenting microorganisms capable of fermenting the sugars directly or indirectly into a desired fermentation product.

"Fermentation" or "fermentation process" refers to any fermentation process or any process comprising a fermentation step. Fermentation processes also include fermentation processes used in the consumable alcohol industry (*e.g.,* beer and wine), dairy industry (*e.g.,* fermented dairy products), leather industry, and tobacco industry. The fermentation conditions depend on the desired fermentation product and fermenting organism and may easily be determined by one skilled in the art.

In the fermentation step, sugars, released from the cellulosic material as a result of the pretreatment and enzymatic hydrolysis steps, are fermented to a product, *e.g.*, ethanol, by a fermenting organism, such as yeast. Hydrolysis (saccharification) and fermentation may be separate or simultaneous. Hydrolysis as described herein includes multi-stage hydrolysis. Where hydrolysis and fermentation are simultaneous, fermentation is carried out with one or more stages of hydrolysis.

Hydrolysis (saccharification) and fermentation, separate or simultaneous, include, but are not limited to, separate hydrolysis and fermentation (SHF); simultaneous saccharification and fermentation (SSF); simultaneous saccharification and co-fermentation (SSCF); hybrid hydrolysis and fermentation (HHF); separate hydrolysis and co-fermentation (SHCF); hybrid hydrolysis and co-fermentation (HHCF); and direct microbial conversion (DMC), also sometimes called consolidated bioprocessing (CBP). SHF uses separate process steps to first enzymatically hydrolyze the cellulosic material to fermentable sugars, *e.g*., glucose, cellobiose, and pentose monomers, and then ferment the fermentable sugars to ethanol. In SSF, the enzymatic hydrolysis of the cellulosic material and the fermentation of sugars to ethanol are combined in one step (Philippidis, G. P., 1996, Cellulose bioconversion technology, in Handbook on Bioethanol: Production and Utilization, Wyman, C. E., ed., Taylor & Francis, Washington, DC, 179-212). SSCF involves the co-fermentation of multiple sugars (Sheehan and Himmel, 1999, Biotechnol. Prog. 15: 817-827). HHF involves a separate hydrolysis step, and in addition a simultaneous saccharification and hydrolysis step, which can be carried out in the same reactor. The steps in an HHF process can be carried out at different temperatures, i.e., high temperature enzymatic saccharification followed by SSF at a lower temperature that the fermentation strain can tolerate. DMC combines all three processes (enzyme production, hydrolysis, and fermentation) in one or more (e.g., several) steps where the same organism is used to produce the enzymes for conversion of the cellulosic material to fermentable sugars and to convert the fermentable sugars into a final product (Lynd et al., 2002, Microbiol. Mol. Biol. Reviews 66: 506-577). It is understood herein that any method known in the art comprising pretreatment, enzymatic hydrolysis (saccharification), fermentation, or a combination thereof, can be used in the practicing processes of the present invention.

Still further, the invention relates to processes of producing a fermentation product from a lignocellulosic material, the process comprising the steps of contacting the lignocellulosic material with 1) a first enzyme composition comprising a xylanase in an amount of about 4.3 U to about 716.1 U per gram of the lignocellulosic material, a beta-xylosidase in an amount of about 0.005 U to about 0.86 U per gram of the lignocellulosic material and an endoglucanase in an amount of about 2.84 U to about 117.2 U per gram of the lignocellulosic material and 2) a second enzyme composition comprising one or more cellulases to form a hydrolyzate, and fermenting the hydrolyzate to produce a fermentation Z r product, wherein step 1) is performed in a continuously stirred tank reactor (CSTR) and step 2) is carried out in a separate batch reactor in series with the reactor from step 1). The first enzyme composition is added in a first stage of hydrolysis and the second enzyme composition is added in a later (*e.g*., second) stage of hydrolysis. In a further embodiment, the stages of hydrolysis are conducted at a pH independently selected from about 3.5 to about 5.5. In a still further embodiment, the first stage of hydrolysis is conducted at a lower pH than the second stage of hydrolysis. In another embodiment, the second enzyme composition is added at least about 2 hours, at least about 3 hours, at least about 5 hours, at least about 10 hours, or at least about 20 hours following contacting of the lignocellulosic material and the first enzyme composition. In the invention the saccharification comprising combining the lignocellulosic material with a first enzyme composition is performed in a continuous reactor.

Further disclosed are processes of fermenting a lignocellulosic material, comprising: fermenting the lignocellulosic material with one or more (*e.g*., several) fermenting microorganisms, wherein the lignocellulosic material is hydrolyzed with 1) a first enzyme composition comprising a xylanase in an amount of about 4.3 U to about 716.1 U per gram of the lignocellulosic material, a beta-xylosidase in an amount of about 0.005 U to about 0.86 U per gram of the lignocellulosic material and an endoglucanase in an amount of about 2.84 U to about 117.2 U per gram of the lignocellulosic material and 2) a second enzyme composition comprising one or more cellulases to form a hydrolyzate. In one example, the fermenting of the cellulosic material produces a fermentation product. In another example, the processes further comprise recovering the fermentation product from the fermentation.

Any suitable hydrolyzed cellulosic material may be used in the fermentation step. The material is generally selected based on economics, *i.e*., costs per equivalent sugar potential, and recalcitrance to enzymatic conversion.

The term "fermentation medium" is understood herein to refer to a medium before the fermenting microorganism(s) is (are) added, such as, a medium resulting from a saccharification process, as well as a medium used in a simultaneous saccharification and fermentation process (SSF).

Suitable fermenting organisms used according of processes of the invention are described below in the "Fermenting Organism"-section below

Fermenting Organism

"Fermenting organism" or "fermenting microorganism" refers to any microorganism, including bacterial and fungal organisms, suitable for use in a desired fermentation process to produce a fermentation product. The fermenting organism may be hexose (*i.e.*, $C_6$) and/or pentose ($C_5$) fermenting organisms, or a combination thereof. Both hexose and pentose fermenting organisms are well known in the art. Suitable fermenting organisms are able to ferment, *i.e.,* convert, sugars, such as glucose, xylose, xylulose, arabinose, maltose, mannose, galactose, and/or oligosaccharides, directly or indirectly into the desired fermentation product. Examples of bacterial and fungal fermenting organisms producing ethanol are described by Lin et al., 2006, Appl. Microbiol. Biotechnol. 69: 627-642.

Examples of fermenting microorganisms that can ferment $C_6$ sugars include bacterial and fungal organisms, such as yeast. Yeast include strains of *Candida, Kluyveromyces,* and *Saccharomyces, e.g., Candida sonorensis, Kluyveromyces marxianus,* and *Saccharomyces cerevisiae.* Preferred yeast includes strains of the *Saccharomyces* spp., preferably *Saccharomyces cerevisiae.*

Examples of fermenting organisms that can ferment $C_5$ sugars include bacterial and fungal organisms, such as yeast. Preferred $C_5$ fermenting yeast include strains of *Pichia,* preferably *Pichia stipitis,* such as *Pichia stipitis* CBS 5773; strains of *Candida,* preferably *Candida boidinii, Candida brassicae, Candida sheatae, Candida diddensii, Candida pseudotropicalis,* or *Candida utilis.* Organisms not capable of fermenting pentose sugars, such as xylose and arabinose, may be genetically modified to do so by methods known in the art.

Examples of bacteria that can efficiently ferment hexose and pentose to ethanol include, for example, *Bacillus coagulans, Clostridium acetobutylicum, Clostridium thermocellum, Clostridium phytofermentans, Geobacillus* sp., *Thermoanaerobacter saccharolyticum,* and *Zymomonas mobilis* (Philippidis, 1996, *supra).*

Other fermenting organisms include strains of *Bacillus,* such as *Bacillus coagulans; Candida,* such as *C. sonorensis, C. methanosorbosa, C. diddensiae, C. parapsilosis, C. naedodendra, C. blankii, C. entomophilia, C. brassicae, C. pseudotropicalis, C. boidinii, C. utilis,* and *C. scehatae; Clostridium,* such as *C. acetobutylicum, C. thermocellum,* and *C. phytofermentans; E. coli,* especially *E. coli* strains that have been genetically modified to improve the yield of ethanol; *Geobacillus* sp.; *Hansenula,* such as *Hansenula anomala; Klebsiella,* such as *K. oxytoca; Kluyveromyces,* such as *K. marxianus, K. lactis, K. thermotolerans,* and *K. fragilis; Schizosaccharomyces,* such as S. *pombe; Thermoanaerobacter,* such as *Thermoanaerobacter saccharolyticum;* and *Zymomonas,* such as *Zymomonas mobilis.*

Commercially available yeast suitable for ethanol production include, e.g., BIO-FERM® AFT and XR, ETHANOL RED® yeast, FALI®, FERMIOL®, GERT STRAND™ (Gert Strand AB, Sweden), SUPERSTART™ and THERMOSACC® fresh yeast.

In an example, the fermenting organism has been genetically modified to provide the ability to ferment pentose sugars, such as xylose utilizing, arabinose utilizing, and xylose and arabinose co-utilizing microorganisms.

The cloning of heterologous genes into various fermenting microorganisms has led to the construction of organisms capable of converting hexoses and pentoses to ethanol (cofermentation) (Chen and Ho, 1993, Appl. Biochem. Biotechnol. 39-40: 135-147; Ho et al., 1998, Appl. Environ. Microbiol. 64: 1852-1859; Kotter and Ciriacy, 1993, Appl. Microbiol. Biotechnol. 38: 776-783; Walfridsson et al., 1995, Appl. Environ. Microbiol. 61: 4184-4190; Kuyper et al., 2004, FEMS Yeast Research 4: 655-664; Beall et al., 1991, Biotech. Bioeng. 38: 296-303; Ingram et al., 1998, Biotechnol. Bioeng. 58: 204-214; Zhang et al., 1995, Science 267: 240-243; Deanda et al., 1996, Appl. Environ. Microbiol. 62: 4465-4470; WO 03/062430).

It is well known in the art that the organisms described above may also be used to produce other substances, as described herein.

The fermenting organism is typically added to the degraded cellulosic material or hydrolyzate and the fermentation is performed for about 8 to about 96 hours, such as about 24 to about 60 hours. The temperature is typically between about 26°C to about 60°C, in particular about 32°C or 50°C, and at about pH 3 to about pH 8, such as around pH 4-5, 6, or 7.

In one example, the yeast and/or another microorganism are applied to the degraded cellulosic material and the fermentation is performed for about 12 to about 96 hours, such as typically 24-60 hours. In another example, the temperature is between about 20°C to about 60°C, *e.g.*, about 25°C to about 50°C, about 32°C to about 50°C, or about 32°C to about 50°C, and the pH is generally from about pH 3 to about pH 7, *e.g.*, about pH 4 to about pH 7. However, some fermenting organisms, *e.g.*, bacteria, have higher fermentation temperature optima. Yeast or another microorganism is preferably applied in amounts of approximately $10^5$ to $10^{12}$, preferably from approximately $10^7$ to $10^{10}$, especially approximately 2 x $10^8$ viable cell count per ml of fermentation broth. Further guidance in respect of using yeast for fermentation may be found in, *e.g.*, "The Alcohol Textbook" (Editors K. Jacques, T.P. Lyons and D.R. Kelsall, Nottingham University Press, United Kingdom 1999).

For ethanol production, following the fermentation the fermented slurry may be distilled to extract the ethanol. The ethanol obtained according to processes of the invention may be used as, *e.g.*, fuel ethanol, drinking ethanol, *i.e.*, potable neutral spirits, or industrial ethanol.

Fermentation Stimulators

A fermentation stimulator may be used in combination with any of the processes described herein to further improve the fermentation process, and in particular, the performance of the fermenting microorganism, such as, rate enhancement and ethanol yield. A "fermentation stimulator" refers to stimulators for growth of the fermenting microorganisms, in particular, yeast. Preferred fermentation stimulators for growth include vitamins and minerals. Examples of vitamins include multivitamins, biotin, pantothenate, nicotinic acid, meso-inositol, thiamine, pyridoxine, para-aminobenzoic acid, folic acid, riboflavin, and Vitamins A, B, C, D, and E. See, for example, Alfenore *et al.*, Improving ethanol production and viability of *Saccharomyces cerevisiae* by a vitamin feeding strategy during fed-batch process, Springer-Verlag (2002). Examples of minerals include minerals and mineral salts that can supply nutrients comprising P, K, Mg, S, Ca, Fe, Zn, Mn, and Cu.

Fermentation products

Processes of the present invention can be used to saccharify the lignocellulosic material to fermentable sugars and to convert the fermentable sugars to many useful fermentation products, e.g., fuel (ethanol, n-butanol, isobutanol, biodiesel, jet fuel) and/or platform chemicals (*e.g.*, acids, alcohols, ketones, gases, oils, and the like). The production of a desired fermentation product from the cellulosic material typically involves pretreatment, enzymatic hydrolysis (saccharification), and fermentation.

A fermentation product may be any substance derived from the fermentation. The fermentation product may be, without limitation, an alcohol (*e.g.*, arabinitol, n-butanol, isobutanol, ethanol, glycerol, methanol, ethylene glycol, 1,3-propanediol [propylene glycol], butanediol, glycerin, sorbitol, and xylitol); an alkane (*e.g.*, pentane, hexane, heptane, octane, nonane, decane, undecane, and dodecane), a cycloalkane (*e.g.*, cyclopentane, cyclohexane, cycloheptane, and cyclooctane), an alkene (*e.g.*, pentene, hexene, heptene, and octene); an amino acid (*e.g.*, aspartic acid, glutamic acid, glycine, lysine, serine, and threonine); a gas (*e.g.*, methane, hydrogen ($H_2$), carbon dioxide ($CO_2$), and carbon monoxide (CO)); isoprene; a ketone (*e.g.*, acetone); an organic acid (*e.g.*, acetic acid, acetonic acid, adipic acid, ascorbic acid, citric acid, 2,5-diketo-D-gluconic acid, formic acid, fumaric acid, glucaric acid, gluconic acid, glucuronic acid, glutaric acid, 3-hydroxypropionic acid, itaconic acid, lactic acid, malic acid, malonic acid, oxalic acid, oxaloacetic acid, propionic acid, succinic acid, and xylonic acid); and polyketide. The fermentation product may also be protein as a high value product.

In one embodiment, the fermentation product is an alcohol. The term "alcohol" encompasses a substance that contains one or more hydroxyl moieties. The alcohol may be, but is not limited to, n-butanol, isobutanol, ethanol, methanol, arabinitol, butanediol, ethylene glycol, glycerin, glycerol, 1,3-propanediol, sorbitol, xylitol. See, for example, Gong et al., 1999, Ethanol production from renewable resources, in Advances in Biochemical Engineering/Biotechnology, Scheper, T., ed., Springer-Verlag Berlin Heidelberg, Germany, 65: 207-241; Silveira and Jonas, 2002, Appl. Microbiol. Biotechnol. 59: 400-408; Nigam and Singh, 1995, Process Biochemistry 30(2): 117-124; Ezeji et al., 2003, World Journal of Microbiology and Biotechnology 19(6): 595-603.

In another embodiment, the fermentation product is an alkane. The alkane may be an unbranched or a branched alkane. The alkane may be, but is not limited to, pentane, hexane, heptane, octane, nonane, decane, undecane, or dodecane.

In another embodiment, the fermentation product is a cycloalkane. The cycloalkane may be, but is not limited to, cyclopentane, cyclohexane, cycloheptane, or cyclooctane.

In another embodiment, the fermentation product is an alkene. The alkene may be an unbranched or a branched alkene. The alkene may be, but is not limited to, pentene, hexene, heptene, or octene.

In another embodiment, the fermentation product is an amino acid. The organic acid may be, but is not limited to, aspartic acid, glutamic acid, glycine, lysine, serine, or threonine. See, for example, Richard and Margaritis, 2004, Biotechnology and Bioengineering 87(4): 501-515.

In another embodiment, the fermentation product is a gas. The gas may be, but is not limited to, methane, $H_2$, $CO_2$, or CO. See, for example, Kataoka et al., 1997, Water Science and Technology 36(6-7): 41-47; and Gunaseelan, 1997, Biomass and Bioenergy 13(1-2): 83-114.

In another embodiment, the fermentation product is isoprene.

In another embodiment, the fermentation product is a ketone. The term "ketone" encompasses a substance that contains one or more ketone moieties. The ketone may be, but is not limited to, acetone.

In another embodiment, the fermentation product is an organic acid. The organic acid may be, but is not limited to, acetic acid, acetonic acid, adipic acid, ascorbic acid, citric acid, 2,5-diketo-D-gluconic acid, formic acid, fumaric acid, glucaric acid, gluconic acid, glucuronic acid, glutaric acid, 3-hydroxypropionic acid, itaconic acid, lactic acid, malic acid, malonic acid, oxalic acid, propionic acid, succinic acid, or xylonic acid. See, for example, Chen and Lee, 1997, Appl. Biochem. Biotechnol. 63-65: 435-448.

In another embodiment, the fermentation product is polyketide.

Recovery

The fermentation product(s) may be optionally recovered from the fermentation medium using any method known in the art including, but not limited to, chromatography, electrophoretic procedures, differential solubility, distillation, or extraction. For example, alcohol is separated from the fermented cellulosic material and purified by conventional methods of distillation. Ethanol with a purity of up to about 96 vol. % may be obtained, which may be used as, for example, fuel ethanol, drinking ethanol, *i.e.*, potable neutral spirits, or industrial ethanol.

The present invention is further described by the following examples.

The following are referred to in the examples:

Cellulolytic Enzyme Preparation A ("CPrepA"): Cellulolytic enzyme composition derived from *Trichoderma reesei* further comprising an AA9 (GH61) polypeptide having cellulolytic enhancing activity of SEQ ID NO: 10, a beta-glucosidase of SEQ ID NO: 4, a cellobiohydrolase I of SEQ ID NO: 6, a cellobiohydrolase II of SEQ ID NO: 8, a xylanase of SEQ ID NO: 12, and a beta-xylosidase of SEQ ID NO: 16.

Xylanase Enzyme Preparation A ("XPrepA"): Enzyme composition from *Trichoderma reesei,* further comprising a xylanase of SEQ ID NO: 12 and a beta-xylosidase of SEQ ID NO: 16.

Xylanase Enzyme Preparation B ("XPrepB"): Enzyme composition from *Trichoderma reesei,* further comprising a xylanase of SEQ ID NO: 14 herein and a beta-xylosidase of SEQ ID NO: 18.

Endoglucanase Enzyme Preparation ("EG1"): Enzyme composition from *A. oryzae*, further comprising an endoglucanase of SEQ ID NO: 20.

## EXAMPLES

## EXAMPLE 1

### COMPARISON OF TWO STAGE HYDROLYSIS WITH FIRST STEP OF CSTR VS. BATCH

Wheat straw was introduced into a continuous reactor and subjected to a soaking treatment at a temperature of 158°C for 65 minutes. The soaked mixture was separated in a soaked liquid and a fraction containing the solid soaked raw material by means of a press. The fraction containing the solid soaked raw material was subjected to steam explosion at a temperature of 200°C for a time of 4 minutes to produce a solid stream.

Soaked liquid was subjected to a concentration step by means of a membrane filtration step, which also removes a portion of acetic acid. First, soaked liquids were subjected to a preliminary pre-separation step to remove solids, by means of centrifugation and macro filtration (bag filter with filter size of 1 micron). Centrifugation was performed by means of an Alfa Laval CLARA 80 centrifuge at 8000 RPM. The soaked liquid was then subjected to concentration by means of an Alfa Laval 2.5" equipment (membrane code NF99 2517/48), operated at a VCR (Volume Concentration Ratio) of 2.5.

The pre-treatment, including concentration step, produced a soaked liquid and a solid stream in a ratio of liquid stream: solid stream by weight of 1:1. The soaked liquid and the solid stream were used as pre-treated wheat straw material in the following enzymatic hydrolysis experiments.

The dry matter of the soaked liquid after concentration was 10%. pH of the solid stream was 4 and pH of the liquid stream was 4.

The pretreated wheat straw was subjected to one of two hydrolysis reactions: two stage hydrolysis, with the first stage in CSTR and the second stage in batch, as compared to a pure batch hydrolysis.

A first stage CSTR contained a total reaction mass of 100 kg and was operated by discharging 10 kg of hydrolysis reaction material every hour and immediately adding 10 kg of pre-treated wheat straw material and water as 3.2 kg soaked liquid, 4 kg solid stream and 2.8 kg water. At the time of each addition of new material to the CSTR, 35 g of CPrepA was added to the CSTR at a dose of 5.7% (weight/weight glucan). The CSTR retention time was 10 hour. pH was controlled at a targeted 5.2 in the CSTR with additions of 2M sodium hydroxide. The subsequent batch hydrolysis was performed in a Labfors 5 BioEtOH reactor (Infors AG, Switzerland). The reaction in the CSTR was performed at 18% dry matter, 50°C and pH 5.0 and the reaction in the Labfors 5 BioEtOH reactor was performed at 18% dry matter, 50°C and pH 5.0. No additional enzymes were added in the subsequent stage batch hydrolysis.

The pure batch hydrolysis was performed in a mixed tank reactor filled with a total of 15 kg reaction mass. CPrepA was added at the start of the reaction in a dose of 5.6% (weight/ weight glucan). The reaction was performed at 21% dry matter, 50°C and pH 5.0.

The hydrolysis performance was evaluated in terms of glucose yield and xylose yield. The total glucose and xylose concentrations from each reaction were determined by HPLC. Glucose yield is the percent ratio of the amount of glucose in the hydrolyzed mixture to the amount of glucans in the pretreated streams, expressed as glucose equivalents. Glucose equivalents were calculated including insoluble glucans, gluco-oligomers, cellobiose and glucose, present in both the solid and liquid of the lignocellulosic biomass, taking into account the different molecular weights. Equivalently, xylose yield is the percent ratio of the amount of xylose in the hydrolyzed mixture to the amount of xylans in the pretreated streams, expressed as xylose equivalents. Xylose equivalents were calculated including insoluble xylans, xylooligomers, xylobiose and xylose, present in both the solid and liquid of the lignocellulosic biomass, taking into account the different molecular weights.

Table 1 shows glucose and xylose yield at different time points of the enzymatic hydrolysis of pre-treated wheat straw using two-stage enzyme addition in a continuously stirred tank reactor followed by hydrolysis in batch (CSTR + batch) compared to hydrolysis in only batch (pure batch) with CPrepA.

A lower glucose yield was obtained with a first stage hydrolysis in a CSTR, followed by second stage hydrolysis in a batch reactor, as compared to a pure batch hydrolysis. Results are set forth in Table 1 and illustrated in Fig. 1. The hydrolysis of the CSTR + batch reaction reached a glucose yield of 42% and a xylose yield of 62% after 82h of reaction time compared to a glucose yield of 55% and a xylose yield of 65% for the pure batch hydrolysis after 72h of reaction time. These yields illustrated that resulting sugar yields are lower in first stage CSTR as compared to pure batch.

Table 1

| | Time (h) | 10h | 34h | 58h | 82h |
|---|---|---|---|---|---|
| CSTR + batch | Glucose yield | 17% | 34% | 38% | 42% |
| | Xylose yield | 30% | 50% | 56% | 62% |
| | | | | | |
| | Time (h) | 0h | 24h | 48h | 72h |
| Pure batch | Glucose yield | 0% | 41% | 50% | 53% |
| | Xylose yield | 2% | 52% | 62% | 66% |

Analytical measurements were performed according to the following standards issued by the National Renewable Energy Laboratory (NREL):

Determination of Structural Carbohydrates and Lignin in Biomass; Laboratory Analytical Procedure (LAP) Issue Date: 4/25/2008; Technical Report NREL/TP-510-42618 Revised April 2008

Determination of Extractives in Biomass; Laboratory Analytical Procedure (LAP) Issue Date: 7/17/2005; Technical Report NREL/TP-510-42619 January 2008

Preparation of Samples for Compositional Analysis; Laboratory Analytical Procedure (LAP) Issue Date: 9/28/2005; Technical Report NREL/TP-510-42620 January 2008

Determination of Total Solids in Biomass and Total Dissolved Solids in Liquid Process Samples; Laboratory Analytical Procedure (LAP) Issue Date: 3/31/2008; Technical Report NREL/TP-510-42621 Revised March 2008

Determination of Ash in Biomass; Laboratory Analytical Procedure (LAP) Issue Date: 7/17/2005; Technical Report NREL/TP-510-42622 January 2008

Determination of Sugars, By products, and Degradation Products in Liquid Fraction Process Samples; Laboratory Analytical Procedure (LAP) Issue Date: 12/08/2006; Technical Report NREL/TP-510-42623 January 2008

Determination of Insoluble Solids in Pretreated Biomass Material; Laboratory Analytical Procedure (LAP) Issue Date: 03/21/2008; NREL/TP-510-42627 March 2008

## EXAMPLE 2

## VISCOSITY REDUCTION WITH ENDOGLUCANASE

The cellulosic material used for this experiment was corn stover pretreated by steam explosion in the presence of dilute sulfuric acid. The pretreated cellulosic material was supplied by the National Renewable Energy Laboratory (NREL) in Golden, Colorado. Total solid content was 21% and the sulfuric acid concentration was approximately 0.8%. The cellulosic material was heated to 180°C for approximately 5 minutes before being discharged and steam exploded. The pH of the pretreated substrate was adjusted to 5.0 with sodium hydroxide before enzymes were added. After pH adjustment, the total solids content was 22.5%, due to the added salts.

The reaction was run in a Rapid Visco Analyzer RVA-4 (Newport Scientific Pty. Ltd., ½ Apollo Street, Warriewood, NSW 2102, Australia). This instrument provides a temperature controlled cylindrical compartment with a paddle agitator. Viscosity is deduced from the measured torque of the agitator. The agitation speed was 500 RPM, and the temperature was 50°C.

26.7g of substrate was added to the aluminum canister used for RVA analysis. 3.3 ml of de-ionized water and enzyme dilution was added to substrate. The resulting total solids content was 20%. The RVA agitator was used to provide slight initial mixing, and then the canister with paddle was added to the RVA for measurement of viscosity during hydrolysis. The experiment was run with 1 and 5 mg enzyme protein per gram cellulose, respectively, for Cellulolytic Enzyme Preparation A ("CPrepA"), and 1 mg enzyme protein per gram cellulose for the Endoglucanase Enzyme Preparation ("EG1").

Fig. 2 shows the measured viscosity of the biomass slurry versus time for the three different enzyme dosages. It is seen, that over the course of five hours, a dose of 1 mg enzyme protein per gram cellulose of CPrepA resulted in a final viscosity of approximately 250 cP. A dose of 5 mg enzyme protein per gram cellulose of CPrepA resulted in a final viscosity of approximately 200 cP. A dose of 1 mg enzyme protein per gram cellulose of the EG1 resulted in a final viscosity of approximately 200 cP, and the rate of viscosity reduction was faster than for 5 mg CPrepA. Hence, it is evident that the EG1 is more efficient at reducing the viscosity of a biomass slurry than CPrepA.

## EXAMPLE 3

## VISCOSITY REDUCTION WITH ENZYME BLEND OF ENDOGLUCANASE AND XYLANASES

Hydrolysis was performed on pre-treated wheat straw in order to measure apparent viscosity in the biomass slurry. A combination of XPrepB and EG1 was compared to CPrepA with the aim of obtaining more or equal viscosity reduction as a 5 mg EP/g glucan dose of CPrepA.

The fiber fraction and the liquid fraction of pre-treated wheat straw were combined in a ratio of Liquid/Solid = 0.75. During mixing of the solid and liquid fraction the pH of the substrate was adjusted to 5.2 with 2 M potassium hydroxide.

Hydrolysis was started by weighing the mixed and pH adjusted substrate in to 50 ml centrifuge tubes (LOT:525-0160, VWR International, Radnor, Pennsylvania, US) that correspond to a final reaction dry matter of 10%. 1 ml of 1M sodium acetate buffer at pH 5.2 was added to each tube. Double deionized water was added to all tubes so that the final weight of all tubes would be 20g after enzyme addition. To aid mixing in the tubes three stainless steel balls (LOT:412-3141, VWR International, Radnor, Pennsylvania, US) was added to all tubes. The reaction in each tube was started by adding CPrepA or the combination of XPrepB and EG1. CPrepA was dosed at 3, 4, 5, 6, 7 and 8 mg EP/g glucan. A 50:50 blend of XPrepB and EG1 was added at 1.5, 2, 3, 4, 5, 6 mg EP/g glucan. Each tube was placed in a FinePCR Thermo Rotisserie Incubators (A. Daigger & Company, Vernon Hills, Illinois) set to 50 °C. Tubes were incubated for 6h and after which the apparent viscosity was measured using a ViPr viscosimeter as described in (WO 2011/107472 A1). For this evaluation the pressure measurement obtained during the aspiration phase of the ViPr viscometer was used (MIN). Evaluation of the result showed that at an enzyme dose of 5 mg EP/g glucan a significantly ($P \leq 0.0072$) lower apparent viscosity was obtained using the 50:50 mixture of XPrepB and EG1 when compared to the 5 mg EP/g glucan of CPrepA. Results are shown in Fig. 3, Fig. 4 and Fig. 5.

| Average at 5 mgEP/g | AVG MIN | Stdev MIN | T-Test* |
|---|---|---|---|
| CPrepA | 413 | 112 | 0.0072 |
| EG1:XPrepB | 215 | 39 | |
| *T-Test performed as 2 tailed distribution with equal variance of samples | | | |

## EXAMPLE 4

## TWO-STAGE DOSING OF ENZYMES IN CONTINUOUSLY STIRRED TANK REACTORS FOLLOWED BY BATCH REACTOR

### Pretreatment

Wheat straw was introduced into a continuous reactor and subjected to a soaking treatment at a temperature of 158°C for 65 minutes. The soaked mixture was separated in a soaked liquid and a fraction containing the solid soaked raw material by means of a press. The fraction containing the solid soaked raw material was subjected to steam explosion at a temperature of 200°C for a time of 4 minutes to produce a solid stream.

Soaked liquid was subjected to a concentration step by means of a membrane filtration step, which also removes a portion of acetic acid.

First, soaked liquids were subjected to a preliminary pre-separation step to remove solids, by means of centrifugation and macro filtration (bag filter with filter size of 1 micron). Centrifugation was performed by means of an Alfa Laval CLARA 80 centrifuge at 8000 RPM.

The soaked liquid was then subjected to concentration by means of an Alfa Laval 2.5" equipment (membrane code NF99 2517/48), operated at a VCR (Volume Concentration Ratio) of 2.5.

The pre-treatment, including concentration step, produced a soaked liquid and a solid stream in a ratio liquid stream: solid stream by weight of 1:1.

The soaked liquid and the solid stream were used in the enzymatic hydrolysis experiments. pH of the solid stream was 4 and pH of the liquid stream was 4.

The dry matter of the soaked liquid after concentration was 10%.

Hydrolysis

An enzymatic hydrolysis in a CSTR+batch configuration was performed by using CPrepA, XPrepB (xylanase: 217±4.8 U/mg; beta-xylosidase: 0.26±0.02 U/mg), and EG1 (cellulase: 71±12 U/mg). The hydrolysis was conducted sequentially in a CSTR, followed by a batch reactor. On a protein dose basis the XPrepB was dosed in a 2:1 ratio over EG1. XPrepB and EG1 comprised one third of the total protein loading in the hydrolysis process and was added into the CSTR at the start of the hydrolysis. CPrepA, used in the second enzyme addition, comprised the remaining two thirds of the total protein.

The CSTR contained a total reaction mass of 200 kg and was operated by discharging 10 kg of hydrolysis reaction material every hour and immediately adding 10 kg of pre-treated wheat straw material and water. At the time of each addition of new material to the CSTR, 24.3 g of EG1 and 6.1 g of XPrepB was added to the CSTR. The CSTR retention time was 20 hour. The subsequent batch hydrolysis was performed in a Labfors 5 BioEtOH reactor (Infors AG, Switzerland). The reaction in the CSTR was performed at 21% dry matter, 50°C and pH 4.9 and the reaction in the Labfors 5 BioEtOH reactor was performed at 21% dry matter, 50°C and pH 5.0. The second dose of enzyme, CPrepA (22.2 g), was added after 20 hours of incubation in the Labfors 5 BioEtOH reactor.

As a control, a one stage batch hydrolysis was performed in a mixed tank reactor filled with a total of 15 kg reaction mass. CPrepA was added at the start of the reaction. The reaction was performed at 22% dry matter, 50 °C and pH 5.0.

Equal amounts of enzyme protein per gram of glucan was dosed in both the two stage CSTR followed by batch hydrolysis experiment and the one stage batch hydrolysis experiment.

Regulation of pH was made with additions of 2M sodium hydroxide.

Glucose and xylose concentration in the hydrolysis was determined by HPLC.

The hydrolysis performance was evaluated in terms of glucose yield and xylose yield. Glucose yield is the percent ratio of the amount of glucose in the hydrolyzed mixture to the amount of glucans in the pretreated streams, expressed as glucose equivalents. Glucose equivalents were calculated including insoluble glucans, gluco-oligomers, cellobiose and glucose, present in both the solid and liquid of the lignocellulosic biomass, taking into account the different molecular weights. Equivalently, xylose yield is the percent ratio of the amount of xylose in the hydrolyzed mixture to the amount of xylans in the pretreated streams, expressed as xylose equivalents. Glucose equivalents were calculated including insoluble xylans, xylooligomers, xylobiose and xylose, present in both the solid and liquid of the lignocellulosic biomass, taking into account the different molecular weights.

Table 2 shows glucose and xylose yield at different time points of the enzymatic hydrolysis of pre-treated wheat straw using two-stage enzyme addition in a continuously stirred tank reactor followed by hydrolysis in batch (CSTR + batch) compared to hydrolysis in only batch (Pure batch) with CPrepA.

The hydrolysis of the two stage enzyme dosing in CSTR+ batch reached a glucose yield of 54% and a xylose yield of 59% after 70h of reaction time compared to a glucose yield of 50% and a xylose yield of 63% for the one stage enzyme dosing in batch hydrolysis.

Table 2

| | Pure batch (CPrepA) | | | | | CSTR + Batch (EG1 + XPrepB + CPrepA) | | |
|---|---|---|---|---|---|---|---|---|
| Time (h) | 0 | 10 | 24 | 48 | 72 | 20 | 40 | 70 |
| glucose yield | 0% | 23% | 36% | 47% | 50% | 11% | 16% | 54% |
| xylose yield | 7% | 50% | 58% | 62% | 63% | 28% | 38% | 59% |

Analytical measurements were performed according to the following standards issued by the National Renewable Energy Laboratory (NREL):

Determination of Structural Carbohydrates and Lignin in Biomass; Laboratory Analytical Procedure (LAP) Issue Date: 4/25/2008; Technical Report NREL/TP-510-42618 Revised April 2008

Determination of Extractives in Biomass; Laboratory Analytical Procedure (LAP) Issue Date: 7/17/2005; Technical Report NREL/TP-510-42619 January 2008

Preparation of Samples for Compositional Analysis; Laboratory Analytical Procedure (LAP) Issue Date: 9/28/2005; Technical Report NREL/TP-510-42620 January 2008

Determination of Total Solids in Biomass and Total Dissolved Solids in Liquid Process Samples; Laboratory Analytical Procedure (LAP) Issue Date: 3/31/2008; Technical Report NREL/TP-510-42621 Revised March 2008

Determination of Ash in Biomass; Laboratory Analytical Procedure (LAP) Issue Date: 7/17/2005; Technical Report NREL/TP-510-42622 January 2008

Determination of Sugars, By products, and Degradation Products in Liquid Fraction Process Samples; Laboratory Analytical Procedure (LAP) Issue Date: 12/08/2006; Technical Report NREL/TP-510-42623 January 2008

Determination of Insoluble Solids in Pretreated Biomass Material; Laboratory Analytical Procedure (LAP) Issue Date: 03/21/2008; NREL/TP-510-42627 March 2008

## EXAMPLE 5

## TWO-STAGE DOSING OF ENZYMES IN INFORS REACTOR

Hydrolysis was performed on pre-treated wheat straw (pretreated as described in Example 4 above) in Labfors 5 BioEtOH reactors (Infors AG, Switzerland). The reactors were filled with a total reaction weight of 1200 g. The fiber fraction and the liquid fraction of pre-treated wheat straw was combined in a ratio of Liquid/Solid = 0.75. Substrate was added to a final concentration of 17% dry matter. Temperature, pH, mixing speed and dissolved oxygen concentration (DO) was online controlled by the Labfors reactor. The conditions during hydrolysis were; 50°C, 50 RPM and 2% dissolved oxygen. The pH during the initial 18.5h was varied at 5.2, 4.8 or unadjusted. The reactors were started up by first mixing the substrate solid and liquid fraction with calculated amount of water. After this the DO was set to 0% by helium sparging into the reactor. After this the reactor was heated to 50°C and pH was set to desired reaction set point. After this the first dose of enzymes was added. After 18h liquefaction the DO was set to 2% for all reactors and the second dose of enzymes were added to the reactors. In this experiment four reactors were used and the experimental design was as follows:

R1: single dose: 9 mg EP/g glucan of CPrepA at 2%DO and pH 5.2

R2: first dose: 1 mg EG1 + 2 mg XPrepB without pH control during the first 18.5h, second dose: 6 mg EP/g glucan CPrepA at 2% DO, pH 5.2

R3: first dose: 1 mg EG1 + 2 mg XPrepB and pH 4.8 during the first 18.5h, second dose: 6 mg EP/g glucan CPrepA at 2% DO, pH 5.2

R4: first dose: 1 mg EG1 + 2 mg XPrepB and pH 5.2 during the first 18.5h, second dose: 6 mg EP/g glucan CPrepA at 2% DO and maintained pH 5.2

In the reactor without pH adjustment (R2) the pH was observed to be approximately 4.7 during the initial 18.5h. In the reactor with only CPrepA (R1) all enzymes were added at the beginning of the reaction.

The total reaction time in this experiment was 90 hours. Samples taken throughout the reactions were diluted by weight and analyzed on HPLC for glucose and xylose concentration (g sugar /kg of slurry). Based on the composition the theoretical amount glucose and xylose release was calculated as g sugar /kg of slurry for the 17% dry matter reaction. The sugar yields were calculated by dividing HPLC measured sugar with the calculated theoretical max sugar.

Quantification of glucose and xylose was performed by high pressure liquid chromatography using a AMINEX® HPX-87H columns (Bio-Rad Laboratories, Hercules, CA, USA) with an inlet filter (Rheodyne 0,5μm filter size, 3mm ID, P/N: 7335-010) and a guard column (Micro-Guard Cation H Refill Cartridges, Bio-Rad Laboratories, Hercules, CA, USA) with a WATERS® 515 Pump, WATERS® MPSA Millipore, WATERS® 717 Plus Autosampler, WATERS® Column Heater Module and WATERS® 2414 RI detector (Waters Corporation, Milford, MA, USA). The chromatography was performed at 65°C with a flow of 0.6 ml/minute of 0.005 M sulfuric acid.

The results shows that in batch hydrolysis a two stage hydrolysis with an initial endoglucanse and xylanase hydrolysis for 18.5h followed by a dose of cellulosic CPrepA, the final glucose and xylose yield after 90h is similar to when adding the same amount of enzyme as CPrepA from the beginning of the hydrolysis reaction. The results also show that in the reactions where EG1 and XPrepB are added in the first stage, variance of the pH between 5.2 and 4.7 (R3 and R4) does not affect the final yield. (Table 3 and Fig. 6A and Fig. 6B)

Table 3

| | R1: CPrepA - (2%DO) - pH5.2 | | R2: EG1+XPrepB, 18.5h - unadjusted pH -> CPrepA (2%DO) - pH5.2 | | R3: EG1+ XPrepB, 18.5h - pH 4.8 -> CPrepA (2%DO) - pH5.2 | | R4: EG1+ XPrepB, 18.5h - pH 5.2 -> CPrepA (2%DO) - pH 5.2 | |
|---|---|---|---|---|---|---|---|---|
| Time (h) | Glucose yield | Xylose yield | Glucose yield | Xylose yield | Glucose yield | Xylose yield | Glucose yield | Xylose yield |
| 0 | 0.0 | 0.0 | 0.0 | 0.0 | 0.0 | 0.0 | 0.0 | 0.0 |
| 18.5 | 24.1 | 61.6 | 5.7 | 55.9 | 5.4 | 54.3 | 5.4 | 53.8 |
| 23 | 28.6 | 62.5 | 19.6 | 59.7 | 19.2 | 59.8 | 20.0 | 60.8 |
| 40 | 40.9 | 67.4 | 37.9 | 68.2 | 37.0 | 68.2 | 37.1 | 67.8 |
| 68 | 55.6 | 74.2 | 57.1 | 76.3 | 55.6 | 75.8 | 56.1 | 75.5 |
| 90 | 63.0 | 77.7 | 65.4 | 80.3 | 64.2 | 79.3 | 65.4 | 80.1 |

## EXAMPLE 6

## pH AND TEMPERATURE PROFILES OF ENDOGLUCANASE PREPARATION EG1

The substrate in this trial was AZCL β-Glucan (Megazyme International Ireland, Bray Business Park, Bray, Co. Wicklow, Ireland). The substrate suspension was prepared as follows. 0.4% AZCL β-glucan was suspended in buffer with addition of 0.01% Triton X-100 by gentle stirring.

For the pH profile, the following assay procedure was applied. The assay buffer was 100 mM succinic acid, 100 mM (4-(2-hydroxyethyl)-1-piperazineethanesulfonic acid) (HEPES), 100 mM N-cyclohexyl-2-aminoethanesulfonic acid (CHES), 100 mM 4-(cyclohexylamino)-1-butanesulfonic acid (CABS), 1 mM calcium dichloride, 150 mM potassium chloride, and 0.01% Triton X-100. The pH values were adjusted to 2.0, 2.5, 3.0, 3.5, 4.0, 5.0, 6.0, 7.0, 8.0, 9.0, 10.0 and 11.0 with hydrocloric acid or sodium hydroxide. 20 μl EG1 enzyme sample and 200 μl substrate suspension were mixed in a microtiter plate and placed on ice before the reaction. The assay was initiated by transferring the microtiter plate to an Eppendorf Thermomixer (Eppendorf AG, Barkhausenweg 1, 22339 Hamburg, Germany. The plate was incubated for 20 minutes at 700 RPM and 45°C. The incubation was stopped by transferring the plate back to the ice bath. Then the plate was centrifuged in an ice cold centrifuge for a few minutes and 100 μl supernatant was transferred to a microtiter plate. Optical density at 595 nm was read as a measure of enzyme activity. The reaction was done in triplicate, and a blind with buffer instead of enzyme sample was included in the assay.

For the temperature profile, the following procedure was applied. The assay buffer was 200 mM tris(hydroxymethyl)aminomethane) (Tris-HCl) buffer pH 7. 30 μl enzyme sample and 200 μl substrate suspension were mixed in a 1.5 ml Eppendorf tube and placed on ice before the reaction. The assay was initiated by transferring the Eppendorf tube to an Eppendorf Thermomixer. The tubes were incubated for 30 minutes at 1400 RPM and the temperatures 15, 20, 30, 40, 50, 60, 70, and 80°C, respectively. The incubation was stopped by transferring the tube back to the ice bath. Then the tube was centrifuged in an ice cold centrifuge for a few minutes, and 200 μl supernatant was transferred to a microtiter plate. Optical density at 595 nm was read as a measure of enzyme activity. The reaction was done in triplicate, and a blind with buffer instead of enzyme sample was included in the assay.

Fig. 7A shows the relative enzyme activity versus pH. The enzyme is active from pH 2 to pH 7, but with highest activity around pH 2-3. It has more than 50% activity at pH 7, as compared to activity at pH 2-3. Fig. 7B shows the relative activity vs. temperature. It can be seen that the enzyme is active at all the tested temperatures (20-80°C), and with highest activity around 70°C.

## EXAMPLE 7

## pH PROFILE OF BETA-XYLOSIDASE PREPARATION

An assay for β-xylosidase activity was run with the substrate p-nitrophenyl-β-d-xylopyranoside (Sigma N2132). 2 mM p-nitrophenyl-β-d-xylopyranoside (0.5424 mg/ml) was dissolved in de-ionized water with 0.01% Triton X-100.

An assay buffer was made with 100 mM phosphoric acid, 100 mM acetic acid, 100 mM boric acid, 0.01% Triton X-100, 100 mM potassium chloride, and 2 mM calcium dichloride. pH was adjusted to 3.00, 3.50, 3.75, 4.00, 4.25, 4.50, 4.75, 5.00, 5.25, 5.50, 6.00, and 7.00, respectively, with 27% sodium hydroxide.

A stop solution was made with 0.5 M glycine and 2 mM ethylene diamine tetra-acetic acid (EDTA), adjusted to pH 10.0 with sodium hydroxide.

A dilution of 0.577 mg/l *Aspergillus fumigatus* β-xylosidase (WO 2011/057140) was made in de-ionized water with 0.01% Triton X-100.

One activity unit, U, is defined by the release of 1 micro mole para-nitrophenol per minute under the conditions of the assay (37°C). Activity was expressed as U/mg total enzyme. A para-nitrophenol standard curve was run with the concentration range from 0.05 - 0.50 mM.

20 μl enzyme dilution, 60 μl assay buffer, and 60 μl substrate solution were added to a microtiter plate. The plate was sealed and incubated for 15 minutes at 37°C in an Eppendorf Thermomixer, shaking at 750 RPM. Then, the plate was refrigerated for 2 minutes, and 100 μl stop solution was added. The optical density at 405 nm was read, and the activity was calculated from the standard curve.

Fig. 8 shows that the optimum pH of this enzyme is found in the range between 4 and 4.75.

## EXAMPLE 8

## CELLULASE ASSAY

An assay of cellulase was based on the enzymatic endo-hydrolysis of the 1,4-β-D-glucosidic bonds in carboxymethylcellulose (CMC). The products of the reaction β-1,4 glucan oligosaccharides were determined colorimetrically by measuring the resulting increase in reducing groups using a 3,5-dinitrosalicylic acid reagent. Enzyme activity was calculated from the relationship between the concentration of reducing groups, as glucose equivalents, and absorbance at 540 nm.

One unit of cellulase activity is defined as the amount of enzyme which produces 1 micro mole glucose equivalents per minute (U) under the conditions of the assay (pH 5.0 and 50°C). Activity was expressed as U/mg total enzyme.

### Materials

1.0 % (w/v solution) Carboxymethylcellulose (CMC) solution in 50 mM sodium citrate buffer, pH 5.0.

3,5-Dinitrosalicylic acid (DNS) solution: 20 g/L DNS; 20 g/L NaOH; 4 g/L phenol; 1 g/L sodium metabisulphite

Glucose standard solution (1 mg/mL).

### Procedure

The enzyme was diluted with 50 mM sodium citrate buffer (pH 5) ranging from 0.2-0.05 ug of enzyme. A glucose standard curve was made using glucose concentrations of 0.06, 0.12, 0.25, 0.5, and 1 mg/mL. In a PCR plate, 50 μL of enzyme solution was mixed with 50 μL of the CMC substrate and incubated at 50°C in a thermalcycler. The reaction was stopped after 10 min by addition of 80 μL of DNS solution. This was followed by heating at 95°C for 10 minutes. Then 130 μL of solution was transferred to a flat bottom clear microplate. The optical density was measured at 540 nm for the different samples and standards.

## EXAMPLE 9

## XYLANASE ASSAY

An assay of xylanase was based on the enzymatic endo-hydrolysis of the 1,4-β-D-xylosidic bonds in birchwood xylan. The products of the reaction β-1,4 xylan oligosaccharides were determined colorimetrically by measuring the resulting increase in reducing groups using a 3,5-dinitrosalicylic acid reagent. Enzyme activity was calculated from the relationship between the concentration of reducing groups, as xylose equivalents, and absorbance at 540 nm.

One unit of xylanase activity is defined as the amount of enzyme which produces 1 micro mole xylose equivalents per minute (U) under the conditions of the assay (pH 5.0 and 50°C). Activity was expressed as U/mg total enzyme.

### Materials

1.0 % (w/v solution) birchwood xylan solution in 50 mM sodium citrate buffer, pH 5.0.

3,5-Dinitrosalicylic acid (DNS) solution: 20 g/L DNS; 20 g/L NaOH; 4 g/L phenol; 1 g/L sodium metabisulphite

Xylose standard solution (1 mg/mL).

**Procedure**

The enzyme was diluted with 50 mM sodium citrate buffer (pH 5) ranging from 0.2 -0.05 ug of enzyme. A xylose standard curve was made using xylose concentrations of 0.06, 0.12, 0.25, 0.5, and 1 mg/mL. In a PCR plate, 50 μL of enzyme solution was mixed with 50 μL of the birchwood xylan substrate and incubated at 50°C in a thermalcycler. The reaction was stopped after 10 min by addition of 80 μL of DNS solution. This was followed by heating at 95°C for 10 minutes. Then 130 μL of solution was transferred to a flat bottom clear microplate. The optical density was measured at 540 nm for the different samples and standards.

## EXAMPLE 10

## BETA-XYLOSIDASE ASSAY

An assay of β-xylosidase was based on the enzymatic hydrolysis of the paranitrophenol-β-D-xylopyranoside (pNPX) substrate. The product of the reaction (paranitrophenol) was determined colorimetrically by measuring the resulting increase in absorption at 410 nm under alkaline conditions. Enzyme activity was calculated from the relationship between product and absorbance at 410 nm.

One unit of β-xylosidase activity is defined as the amount of enzyme which produces 1 micro mole pNP product per minute (U) under the conditions of the assay (pH 5.0 and 50°C). Activity was expressed as U/mg total enzyme.

**Materials**

1 mM pNPX solution in 50 mM sodium citrate buffer, pH 5.0.

pNP standard solution (5 mM).

**Procedure**

The enzyme was diluted with 50 mM sodium citrate buffer (pH 5) ranging from 4 - 0.5 ug enzyme. A pNP standard curve was made using pNP concentrations of 0, 0.03, 0.06, 0.12, 0.25, 0.5, mM. In a microplate, 75 μL of enzyme solution was mixed with 75 μL of the pNPX substrate and incubated at 50°C. The reaction was stopped after 30 min by addition of 100 μL of 0.2 M sodium carbonate solution (pH 11). The optical density was measured at 410 nm for the different samples and standards.

## Claims

1. A process of improving a glucose yield of saccharification of a lignocellulosic material, the process comprising the steps of:

    a) a first stage comprising saccharifying a lignocellulosic material in a continuous reactor with a first enzyme composition comprising a xylanase, a beta-xylosidase and an endoglucanase; and
    b) a second stage comprising continuing saccharification of the lignocellulosic material, comprising combining the material of step a) with a second enzyme composition comprising one or more cellulases to form a hydrolyzate wherein the hydrolyzate has a glucose yield that is improved as compared to the yield from a process comprising a single saccharification step; wherein step a) is performed in a continuously stirred tank reactor (CSTR) and step b) is carried out in a separate batch reactor in series with the reactor from step a).

2. The process of claim 1, wherein the amount of xylanase in the first enzyme composition is 4.3 U to 716.1 U per gram of the lignocellulosic material.

3. The process of any of claims 1 to 2, wherein the amount of beta-xylosidase in the first enzyme composition is 0.005 U to 0.86 U per gram of the lignocellulosic material.

4. The process of any of claims 1 to 3, wherein the amount of endoglucanase in the first enzyme composition is 2.84 U to 117.2 U per gram of the lignocellulosic material.

5. The process of any of claims 1 to 4, wherein the ratio of enzyme protein of the first enzyme composition to enzyme protein of the second enzyme composition is about 1:2.

6. The process of any of claims 1 to 5, wherein the first enzyme composition comprises a xylanase selected from the group consisting of: (i) a xylanase comprising or consisting of the mature polypeptide of SEQ ID NO: 12; (ii) a xylanase comprising or consisting of an amino acid sequence having at least 70% identity to the mature polypeptide of SEQ ID NO: 12; (iii) a xylanase encoded by a polynucleotide comprising or consisting of a nucleotide sequence having at least 70% sequence identity to the mature polypeptide coding sequence of SEQ ID NO: 11; (iv) a xylanase encoded by a polynucleotide that hybridizes under at least high stringency conditions with the mature polypeptide coding sequence of SEQ ID NO: 11 or the full-length complement thereof; (v) a xylanase comprising or consisting of the mature polypeptide of SEQ ID NO: 14; (vi) a xylanase comprising or consisting of an amino acid sequence having at least 70% sequence identity to the mature polypeptide of SEQ ID NO: 14; (vii) a xylanase encoded by a polynucleotide comprising or consisting of a nucleotide sequence having at least 70% sequence identity to the mature polypeptide coding sequence of SEQ ID NO: 13; (viii) a xylanase encoded by a polynucleotide that hybridizes under at least high stringency conditions with the mature polypeptide coding sequence of SEQ ID NO: 13 or the full-length complement thereof; (ix) a xylanase comprising or consisting of the mature polypeptide of SEQ ID NO: 22; (x) a xylanase comprising or consisting of an amino acid sequence having at least 70% sequence identity to the mature polypeptide of SEQ ID NO: 22; (xi) a xylanase encoded by a polynucleotide comprising or consisting of a nucleotide sequence having at least 70% sequence identity to the mature polypeptide coding sequence of SEQ ID NO: 21; and (xii) a xylanase encoded by a polynucleotide that hybridizes under at least high stringency conditions with the mature polypeptide coding sequence of SEQ ID NO: 21 or the full-length complement thereof.

7. The process of any of claims 1 to 6, wherein the first enzyme composition comprises a beta-xylosidase selected from the group consisting of: (i) a beta-xylosidase comprising or consisting of the mature polypeptide of SEQ ID NO: 16; (ii) a beta-xylosidase comprising or consisting of an amino acid sequence having at least 70% sequence identity to the mature polypeptide of SEQ ID NO: 16; (iii) a beta-xylosidase encoded by a polynucleotide comprising or consisting of a nucleotide sequence having at least 70% sequence identity to the mature polypeptide coding sequence of SEQ ID NO: 15; (iv) a beta-xylosidase encoded by a polynucleotide that hybridizes under at least high stringency conditions with the mature polypeptide coding sequence of SEQ ID NO: 15 or the full-length complement thereof; (v) a beta-xylosidase comprising or consisting of the mature polypeptide of SEQ ID NO: 24; (vi) a beta-xylosidase comprising or consisting of an amino acid sequence having at least 70% sequence identity to the mature polypeptide of SEQ ID NO: 24; (vii) a beta-xylosidase encoded by a polynucleotide comprising or consisting of a nucleotide sequence having at least 70% sequence identity to the mature polypeptide coding sequence of SEQ ID NO: 23; and (viii) a beta-xylosidase encoded by a polynucleotide that hybridizes under at least high stringency conditions with the mature polypeptide coding sequence of SEQ ID NO: 23 or the full-length complement thereof.

8. The process of any of claims 1 to 7, wherein the first enzyme composition comprises an endoglucanase selected from the group consisting of: (i) an endoglucanase comprising or consisting of the mature polypeptide of SEQ ID NO: 20; (ii) an endoglucanase comprising or consisting of an amino acid sequence having at least 70% sequence identity to the mature polypeptide of SEQ ID NO: 20; (iii) an endoglucanase encoded by a polynucleotide comprising or consisting of a nucleotide sequence having at least 70% sequence identity to the mature polypeptide coding sequence of SEQ ID NO: 19; and (iv) an endoglucanase encoded by a polynucleotide that hybridizes under at least high stringency conditions with the mature polypeptide coding sequence of SEQ ID NO: 19 or the full-length complement thereof.

9. A process of producing a fermentation product from a lignocellulosic material, the process comprising the steps of:

a) hydrolyzing the lignocellulosic material according to the process of any of claims 1 to 8, and
b) fermenting the hydrolyzate to produce a fermentation product.

10. A process of multi-stage hydrolysis of a lignocellulosic material, the process comprising the steps of:

a) first stage comprising saccharifying a lignocellulosic material with a first enzyme composition comprising a xylanase in an amount of 4.3 U to 716.1 U per gram of the lignocellulosic material, a beta-xylosidase in an amount of 0.005 U to 0.86 U per gram of the lignocellulosic material and an endoglucanase in an amount of 2.84 U to 117.2 U, per gram of the lignocellulosic material; and

b) second stage comprising continuing saccharification of the lignocellulosic material, comprising combining the material of step a) with a second enzyme composition comprising one or more cellulases; wherein step a) is performed in a continuously stirred tank reactor (CSTR) and step b) is carried out in a separate batch reactor in series with the reactor from step a).

**11.** A process of producing a fermentation product from a lignocellulosic material, the process comprising the steps of:

a) hydrolyzing the lignocellulosic material, comprising:

1) a first stage comprising saccharifying a lignocellulosic material with a first enzyme composition comprising a xylanase in an amount of 4.3 U to 716.1 U per gram of the lignocellulosic material, a beta-xylosidase in an amount of 0.005 U to 0.86 U per gram of the lignocellulosic material and an endoglucanase in an amount of 2.84 U to 117.2 U per gram of the lignocellulosic material; and
2) a second stage comprising continuing saccharification of the lignocellulosic material, comprising combining the material of stage 1) with a second enzyme composition comprising one or more cellulases to form a hydrolyzate; and

b) fermenting the hydrolyzate to produce a fermentation product; wherein stage 1) is performed in a continuously stirred tank reactor (CSTR) and stage 2) is carried out in a separate batch reactor in series with the reactor from stage 1).

## Patentansprüche

**1.** Verfahren zum Verbessern einer Glucoseausbeute der Verzuckerung eines lignocellulosischen Materials, wobei das Verfahren die Schritte umfasst:

a) eine erste Stufe, die Verzuckern eines lignocellulosischen Materials in einem kontinuierlichen Reaktor mit einer ersten Enzymzusammensetzung umfasst, die eine Xylanase, eine beta-Xylosidase und eine Endoglucanase umfasst; und
b) eine zweite Stufe, die Fortsetzen der Verzuckerung des lignocellulosischen Materials umfasst, die Kombinieren des Materials aus Schritt a) mit einer zweiten Enzymzusammensetzung umfasst, die eine oder mehrere Cellulasen umfasst, um ein Hydrolysat zu bilden, wobei das Hydrolysat eine Glucoseausbeute aufweist, die verglichen zur Ausbeute aus einem Verfahren, das einen einzigen Verzuckerungsschritt umfasst, verbessert ist; wobei Schritt a) in einem kontinuierlich gerührten Tankreaktor (CSTR) durchgeführt wird, und Schritt b) in einem separaten Batch-Reaktor in Reihe mit dem Reaktor aus Schritt a) durchgeführt wird.

**2.** Verfahren nach Anspruch 1, wobei die Menge an Xylanase in der ersten Enzymzusammensetzung 4,3 U bis 716,1 U pro Gramm des lignocellulosischen Materials beträgt.

**3.** Verfahren nach einem beliebigen der Ansprüche 1 bis 2, wobei die Menge an beta-Xylosidase in der ersten Enzymzusammensetzung 0,005 U bis 0,86 U pro Gramm des lignocellulosischen Materials beträgt.

**4.** Verfahren nach einem beliebigen der Ansprüche 1 bis 3, wobei die Menge an Endoglucanase in der ersten Enzymzusammensetzung 2,84 U bis 117,2 U pro Gramm des lignocellulosischen Materials beträgt.

**5.** Verfahren nach einem beliebigen der Ansprüche 1 bis 4, wobei das Verhältnis von Enzymprotein der ersten Enzymzusammensetzung zu Enzymprotein der zweiten Enzymzusammensetzung etwa 1:2 beträgt.

**6.** Verfahren nach einem beliebigen der Ansprüche 1 bis 5, wobei die erste Enzymzusammensetzung eine Xylanase umfasst, die aus der Gruppe ausgewählt ist bestehend aus: (i) einer Xylanase, die das reife Polypeptid von SEQ ID NO: 12 umfasst oder daraus besteht; (ii) einer Xylanase, die eine Aminosäuresequenz mit mindestens 70% Identität zum reifen Polypeptid von SEQ ID NO: 12 umfasst oder daraus besteht; (iii) einer Xylanase, die durch ein Polynukleotid kodiert ist, das eine Nukleotidsequenz mit mindestens 70% Sequenzidentität zur das reife Polypeptid kodierenden Sequenz von SEQ ID NO: 11 umfasst oder daraus besteht; (iv) einer Xylanase, die durch ein Polynukleotid kodiert ist, das unter zumindest hohen Stringenzbedingungen mit der das reife Polypeptid kodierenden Sequenz von SEQ ID NO: 11 oder dem Komplement voller Länge davon hybridisiert; (v) einer Xylanase, die das reife Polypeptid von SEQ ID NO: 14 umfasst oder daraus besteht; (vi) einer Xylanase, die eine Aminosäuresequenz mit mindestens 70% Sequenzidentität zum reifen Polypeptid von SEQ ID NO: 14 umfasst oder daraus besteht; (vii) einer Xylanase, die durch ein Polynukleotid kodiert ist, das eine Nukleotidsequenz mit mindestens 70% Sequenzidentität zur das reife Polypeptid kodierenden Sequenz von SEQ ID NO: 13 umfasst oder daraus besteht; (viii) einer Xylanase, die durch ein Polynukleotid kodiert ist, das unter zumindest hohen Stringenzbedingungen mit der das reife Polypeptid kodierenden Sequenz von SEQ ID NO: 13 oder dem Komplement voller Länge davon hybridisiert; (ix) einer Xylanase, die das reife Polypeptid von SEQ ID NO: 22 umfasst oder daraus besteht; (x) einer Xylanase, die eine Aminosäuresequenz mit mindestens 70% Sequenzidentität zum reifen Polypeptid von SEQ ID NO: 22 umfasst oder daraus besteht; (xi) einer Xylanase, die durch ein Polynukleotid kodiert ist, das eine Nukleotidsequenz mit mindestens 70% Sequenzidentität zur das reife Polypeptid kodierenden Sequenz von SEQ ID NO: 21 umfasst oder daraus besteht; und (xii) einer Xylanase, die durch ein Polynukleotid kodiert ist, das unter zumindest hohen Stringenzbedingungen mit der das reife Polypeptid kodierenden Sequenz von SEQ ID NO: 21 oder dem Komplement voller Länge davon hybridisiert.

**7.** Verfahren nach einem beliebigen der Ansprüche 1 bis 6, wobei die erste Enzymzusammensetzung eine beta-Xylosidase umfasst, die aus der Gruppe ausgewählt ist, bestehend aus: (i) einer beta-Xylosidase, die das reife Polypeptid von SEQ ID NO: 16 umfasst oder daraus besteht; (ii) einer beta-Xylosidase, die eine Aminosäuresequenz mit mindestens 70% Sequenzidentität zum reifen Polypeptid von SEQ ID NO: 16 umfasst oder daraus besteht; (iii) einer beta-Xylosidase, die durch ein Polynukleotid kodiert ist, das eine Nukleotidsequenz mit mindestens 70% Sequenzidentität zur das reife Polypeptid kodierenden Sequenz von SEQ ID NO: 15 umfasst oder daraus besteht; (iv) einer beta-Xylosidase, die durch ein Polynukleotid kodiert ist, das unter zumindest hohen Stringenzbedingungen mit der das reife Polypeptid kodierenden Sequenz von SEQ ID NO: 15 oder dem Komplement voller Länge davon hybridisiert; (v) einer beta-Xylosidase, die das reife Polypeptid von SEQ ID NO: 24 umfasst oder daraus besteht; (vi) einer beta-Xylosidase, die eine Aminosäuresequenz mit mindestens 70% Sequenzidentität zum reifen Polypeptid von SEQ ID NO: 24 umfasst oder daraus besteht; (vii) einer beta-Xylosidase, die durch ein Polynukleotid kodiert ist, das eine Nukleotidsequenz mit mindestens 70% Sequenzidentität zur das reife Polypeptid kodierenden Sequenz von SEQ ID NO: 23 umfasst oder daraus besteht; und (viii) einer beta-Xylosidase, die durch ein Polynukleotid kodiert ist, das unter zumindest hohen Stringenzbedingungen mit der das reife Polypeptid kodierenden Sequenz von SEQ ID NO: 23 oder dem Komplement voller Länge davon hybridisiert.

**8.** Verfahren nach einem beliebigen der Ansprüche 1 bis 7, wobei die erste Enzymzusammensetzung eine Endoglucanase umfasst, die aus der Gruppe ausgewählt ist, bestehend aus: (i) einer Endoglucanase, die das reife Polypeptid von SEQ ID NO: 20 umfasst oder daraus besteht; (ii) einer Endoglucanase, die eine Aminosäuresequenz mit mindestens 70% Sequenzidentität zum reifen Polypeptid von SEQ ID NO: 20 umfasst oder daraus besteht; (iii) einer Endoglucanase, die durch ein Polynukleotid kodiert ist, das eine Nukleotidsequenz mit mindestens 70% Sequenzidentität zur das reife Polypeptid kodierenden Sequenz von SEQ ID NO: 19 umfasst oder daraus besteht; und (iv) einer Endoglucanase, die durch ein Polynukleotid kodiert ist, das unter zumindest hohen Stringenzbedingungen mit der das reife Polypeptid kodierenden Sequenz von SEQ ID NO: 19 oder dem Komplement voller Länge davon hybridisiert.

**9.** Verfahren zum Herstellen eines Fermentationsprodukts aus einem lignocellulosischen Material, wobei das Verfahren die Schritte umfasst:

a) Hydrolysieren des lignocellulosischen Materials gemäß dem Verfahren nach einem beliebigen der Ansprüche 1 bis 8, und
b) Fermentieren des Hydrolysats, um ein Fermentationsprodukt herzustellen.

**10.** Verfahren zur mehrstufigen Hydrolyse eines lignocellulosischen Materials, wobei das Verfahren die Schritte umfasst:

a) erste Stufe, die Verzuckern eines lignocellulosischen Materials mit einer ersten Enzymzusammensetzung umfasst, die eine Xylanase in einer Menge von 4,3 U bis 716,1 U pro Gramm des lignocellulosischen Materials, eine beta-Xylosidase in einer Menge von 0,005 U bis 0,86 U pro Gramm des lignocellulosischen Materials, und eine Endoglucanase in einer Menge von 2,84 U bis 117,2 U pro Gramm des lignocellulosischen Materials umfasst; und
b) zweite Stufe, die Fortsetzen der Verzuckerung des lignocellulosischen Materials umfasst, die Kombinieren des Materials aus Schritt a) mit einer zweiten Enzymzusammensetzung umfasst, die eine oder mehrere Cellulasen umfasst; wobei Schritt a) in einem kontinuierlich gerührten Tankreaktor (CSTR) durchgeführt wird, und Schritt b) in einem separaten Batch-Reaktor in Reihe mit dem Reaktor aus Schritt a) durchgeführt wird.

**11.** Verfahren zum Herstellen eines Fermentationsprodukts aus einem lignocellulosischen Material, wobei das Verfahren die Schritte umfasst:

a) Hydrolysieren des lignocellulosischen Materials, umfassend:

1) eine erste Stufe, die Verzuckern eines lignocellulosischen Materials mit einer ersten Enzymzusammensetzung umfasst, die eine Xylanase in einer Menge von 4,3 U bis 716,1 U pro Gramm des lignocellulosischen Materials, eine beta-Xylosidase in einer Menge von 0,005 U bis 0,86 U pro Gramm des lignocellulosischen Materials, und eine Endoglucanase in einer Menge von 2,84 U bis 117,2 U pro Gramm des lignocellulosischen Materials umfasst; und
2) eine zweite Stufe, die Fortsetzen der Verzuckerung des lignocellulosischen Materials umfasst, die Kombinieren des Materials der Stufe 1) mit einer zweiten Enzymzusammensetzung umfasst, die eine oder mehrere Cellulasen umfasst, um ein Hydrolysat zu bilden; und

b) Fermentieren des Hydrolysats, um ein Fermentationsprodukt herzustellen; wobei Stufe 1) in einem kontinuierlich gerührten Tankreaktor (CSTR) durchgeführt wird, und Stufe 2) in einem separaten Batch-Reaktor in Reihe mit dem Reaktor aus Stufe 1) durchgeführt wird.

## Revendications

**1.** Procédé pour améliorer le rendement en glucose de la saccharification d'un matériau lignocellulosique, le procédé comprenant les étapes suivantes :

a) une première étape comprenant la saccharification d'un matériau lignocellulosique dans un réacteur en continu avec une première composition enzymatique comprenant une xylanase, une bêta-xylosidase et une endoglucanase ; et
b) une deuxième étape comprenant la poursuite de la saccharification du matériau lignocellulosique, comprenant la combinaison du matériau de l'étape a) avec une deuxième composition enzymatique comprenant une ou plusieurs cellulases pour former un hydrolysat, dans lequel l'hydrolysat a un rendement en glucose qui est amélioré par rapport au rendement d'un procédé comprenant une seule étape de saccharification ; dans lequel l'étape a) est effectuée dans un réacteur à cuve agitée en continu (CSTR) et l'étape b) est effectuée dans un réacteur à fonctionnement discontinu séparé en série avec le réacteur de l'étape a).

**2.** Procédé selon la revendication 1, dans lequel la quantité de xylanase dans la première composition enzymatique est de 4,3 U à 716,1 U par gramme du matériau lignocellulosique.

**3.** Procédé selon l'une quelconque des revendications 1 à 2, dans lequel la quantité de bêta-xylosidase dans la première composition enzymatique est de 0,005 U à 0,86 U par gramme du matériau lignocellulosique.

**4.** Procédé selon l'une quelconque des revendications 1 à 3, dans lequel la quantité d'endoglucanase dans la première composition enzymatique est de 2,84 U à 117,2 U par gramme du matériau lignocellulosique.

**5.** Procédé selon l'une quelconque des revendications 1 à 4, dans lequel le rapport des protéines enzymatiques de la première composition enzymatique aux protéines enzymatiques de la deuxième composition enzymatique est d'environ 1 :2.

**6.** Procédé selon l'une quelconque des revendications 1 à 5, dans lequel la première composition enzymatique comprend une xylanase choisie dans le groupe constitué par : (i) une xylanase comprenant ou étant constituée du polypeptide mature de SEQ ID NO : 12 ; (ii) une xylanase comprenant ou étant constituée d'une séquence d'acides aminés ayant au moins 70 % d'identité avec le polypeptide mature de SEQ ID NO : 12 ; (iii) une xylanase codée par un polynucléotide comprenant ou étant constitué d'une séquence de nucléotides ayant au moins 70 % d'identité de séquence avec la séquence codante de polypeptide mature de SEQ ID NO : 11 ; (iv) une xylanase codée par un polynucléotide qui s'hybride dans des conditions au moins de stringence élevée avec la séquence codante de polypeptide mature de SEQ ID NO : 11 ou son complémentaire de pleine longueur ; (v) une xylanase comprenant ou étant constituée du polypeptide mature de SEQ ID NO : 14 ; (vi) une xylanase comprenant ou étant constituée d'une séquence d'acides aminés ayant au moins 70 % d'identité de séquence avec le polypeptide mature de SEQ ID NO : 14 ; (vii) une xylanase codée par un polynucléotide comprenant ou étant constitué d'une séquence de nucléotides ayant au moins 70 % d'identité de séquence avec la séquence codante de polypeptide mature de SEQ ID NO : 13 ; (viii) une xylanase codée par un polynucléotide qui s'hybride dans des conditions au moins de stringence élevée avec la séquence codante de polypeptide mature de SEQ ID NO : 13 ou son complémentaire de pleine longueur ; (ix) une xylanase comprenant ou étant constituée du polypeptide mature de SEQ ID NO : 22 ; (x) une xylanase comprenant ou étant constituée d'une séquence d'acides aminés ayant au moins 70 % d'identité de séquence avec le polypeptide mature de SEQ ID NO : 22 ; (xi) une xylanase codée par un polynucléotide comprenant ou étant constitué d'une séquence de nucléotides ayant au moins 70 % d'identité de séquence avec la séquence codante de polypeptide mature de SEQ ID NO : 21 ; et (xii) une xylanase codée par un polynucléotide qui s'hybride dans des conditions au moins de stringence élevée avec la séquence codante de polypeptide mature de SEQ ID NO : 21 ou son complémentaire de pleine longueur.

**7.** Procédé selon l'une quelconque des revendications 1 à 6, dans lequel la première composition enzymatique comprend une bêta-xylosidase choisie dans le groupe constitué par : (i) une bêta-xylosidase comprenant ou étant constituée du polypeptide mature de SEQ ID NO : 16 ; (ii) une bêta-xylosidase comprenant ou étant constituée d'une séquence d'acides aminés ayant une identité d'au moins 70 % avec le polypeptide mature de SEQ ID NO : 16 ; (iii) une bêta-xylosidase codée par un polynucléotide comprenant ou étant constitué d'une séquence de nucléotides ayant au moins 70 % d'identité de séquence avec la séquence codante de polypeptide mature de SEQ ID NO : 15 ; (iv) une bêta-xylosidase codée par un polynucléotide qui s'hybride dans des conditions au moins de stringence élevée avec la séquence codante de polypeptide mature de SEQ ID NO: 15 ou son complémentaire de pleine longueur ; (v) une bêta-xylosidase comprenant ou étant constituée du polypeptide mature de SEQ ID NO : 24 ; (vi) une bêta-xylosidase comprenant ou étant constituée d'une séquence d'acides aminés ayant au moins 70 % d'identité de séquence avec le polypeptide mature de SEQ ID NO : 24 ; (vii) une bêta-xylosidase codée par un polynucléotide comprenant ou étant constitué d'une séquence de nucléotides ayant au moins 70 % d'identité de séquence avec la séquence codante de polypeptide mature de SEQ ID NO : 23 ; et (viii) une bêta-xylosidase codée par un polynucléotide qui s'hybride dans des conditions au moins de stringence élevée avec la séquence codante de polypeptide mature de SEQ ID NO : 23 ou son complémentaire de pleine longueur.

**8.** Procédé selon l'une quelconque des revendications 1 à 7, dans lequel la première composition enzymatique comprend une endoglucanase choisie dans le groupe constitué par : (i) une endoglucanase comprenant ou étant constituée du polypeptide mature de SEQ ID NO : 20 ; (ii) une endoglucanase comprenant ou étant constituée d'une séquence d'acides aminés ayant une identité d'au moins 70 % avec le polypeptide mature de SEQ ID NO : 20 ; (iii) une endoglucanase codée par un polynucléotide comprenant ou étant constitué d'une séquence de nucléotides ayant au moins 70 % d'identité de séquence avec la séquence codante de polypeptide mature de SEQ ID NO : 19 ; et (iv) une endoglucanase codée par un polynucléotide qui s'hybride dans des conditions au moins de stringence élevée avec la séquence codante de polypeptide mature de SEQ ID NO: 19 ou son complémentaire de pleine longueur.

**9.** Procédé de production d'un produit de fermentation à partir d'un matériau lignocellulosique, le procédé comprenant les étapes suivantes :

a) hydrolyse du matériau lignocellulosique conformément au procédé de l'une quelconque des revendications 1 à 8, et
b) fermentation de l'hydrolysat pour produire un produit de fermentation.

**10.** Procédé d'hydrolyse à étapes multiples d'un matériau lignocellulosique, le procédé comprenant les étapes suivantes :

a) une première étape comprenant la saccharification d'un matériau lignocellulosique avec une première composition enzymatique comprenant une xylanase en une quantité de 4,3 U à 716,1 U par gramme du matériau lignocellulosique, une bêta-xylosidase en une quantité de 0,005 U à 0,86 U par gramme du matériau lignocellulosique et une endoglucanase en une quantité de 2,84 U à 117,2 U par gramme du matériau lignocellulosique ; et
b) une deuxième étape comprenant la poursuite de la saccharification du matériau cellulosique, comprenant la combinaison du matériau de l'étape a) avec une deuxième composition enzymatique comprenant une ou plusieurs cellulases ; dans lequel l'étape a) est effectuée dans un réacteur à cuve agitée en continu (CSTR) et l'étape b) est effectuée dans un réacteur à fonctionnement discontinu séparé en série avec le réacteur de l'étape a).

**11.** Procédé de production d'un produit de fermentation à partir d'un matériau lignocellulosique, le procédé comprenant les étapes suivantes :

a) hydrolyse du matériau lignocellulosique, comprenant :

1) une première étape comprenant la saccharification d'un matériau lignocellulosique avec une première composition enzymatique comprenant une xylanase en une quantité de 4,3 U à 716,1 U par gramme du matériau lignocellulosique, une bêta-xylosidase en une quantité de 0,005 U à 0,86 U par gramme du matériau lignocellulosique et une endoglucanase en une quantité de 2,84 U à 117,2 U par gramme du matériau lignocellulosique ; et
2) une deuxième étape comprenant la poursuite de la saccharification du matériau cellulosique, comprenant la combinaison du matériau de l'étape 1) avec une deuxième composition enzymatique comprenant une ou plusieurs cellulases pour former un hydrolysat ; et

b) fermentation de l'hydrolysat pour produire un produit de fermentation ; dans lequel l'étape 1) est effectuée dans un réacteur à cuve agitée en continu (CSTR) et l'étape 2) est effectuée dans un réacteur à fonctionnement discontinu séparé en série avec le réacteur de l'étape 1).

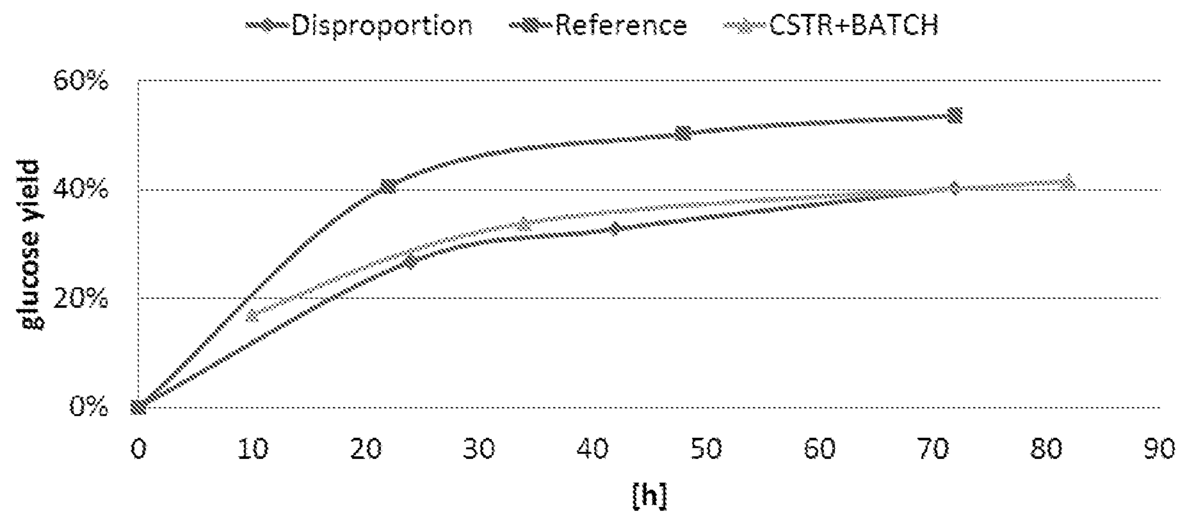
Disproportion
Reference
CSTR+BATCH
glucose yield
60%
40%
20%
0%
0
10
20
30
40
50
60
70
80
90
[h]

FIG. 1

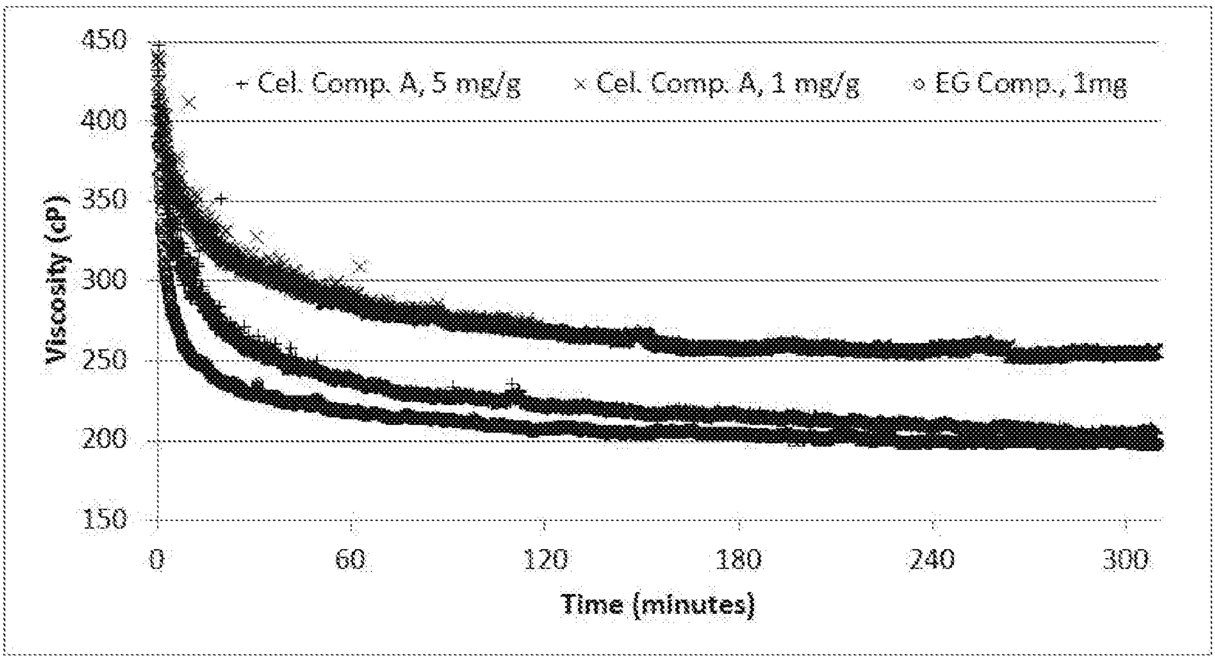
+ Cel. Comp. A, 5 mg/g
× Cel. Comp. A, 1 mg/g
◦ EG Comp., 1mg
Viscosity (cP)
450
400
350
300
250
200
150
0
60
120
180
240
300
Time (minutes)

FIG. 2

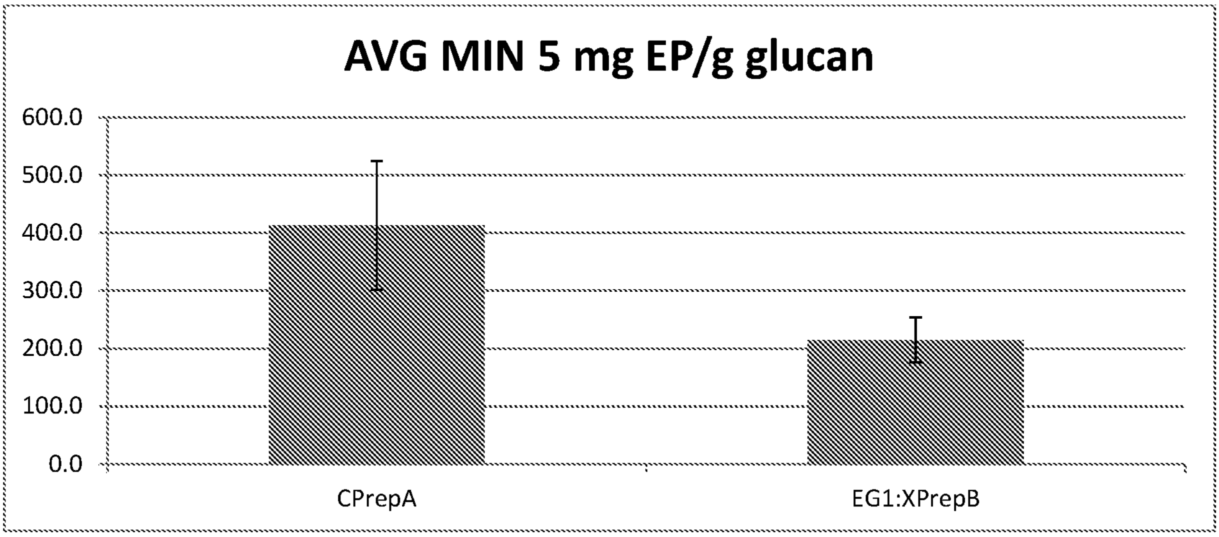
AVG MIN 5 mg EP/g glucan
600.0
500.0
400.0
300.0
200.0
100.0
0.0
CPrepA
EG1:XPrepB

FIG. 3

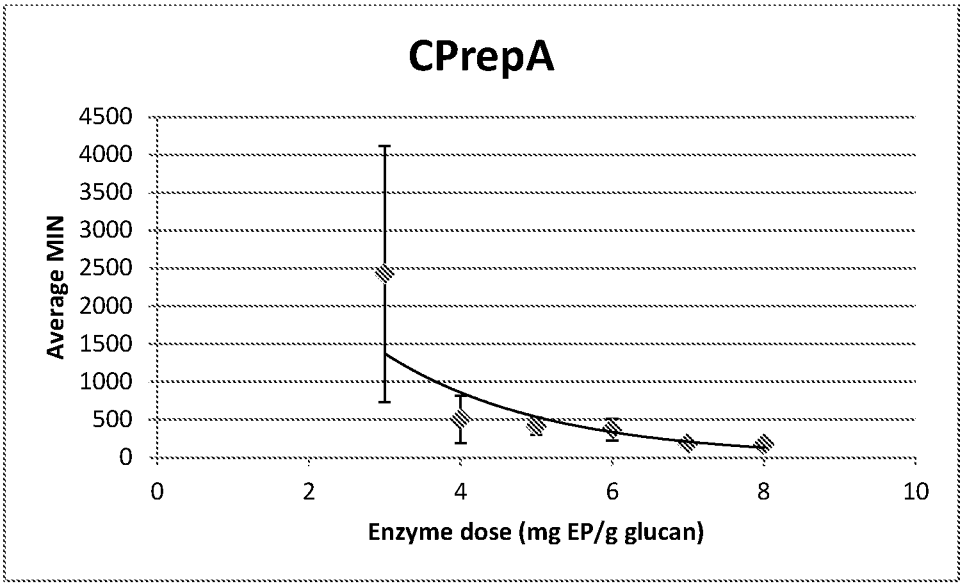
CPrepA
4500
4000
3500
3000
2500
2000
1500
1000
500
0
Average MIN
0
2
4
6
8
10
Enzyme dose (mg EP/g glucan)

FIG. 4

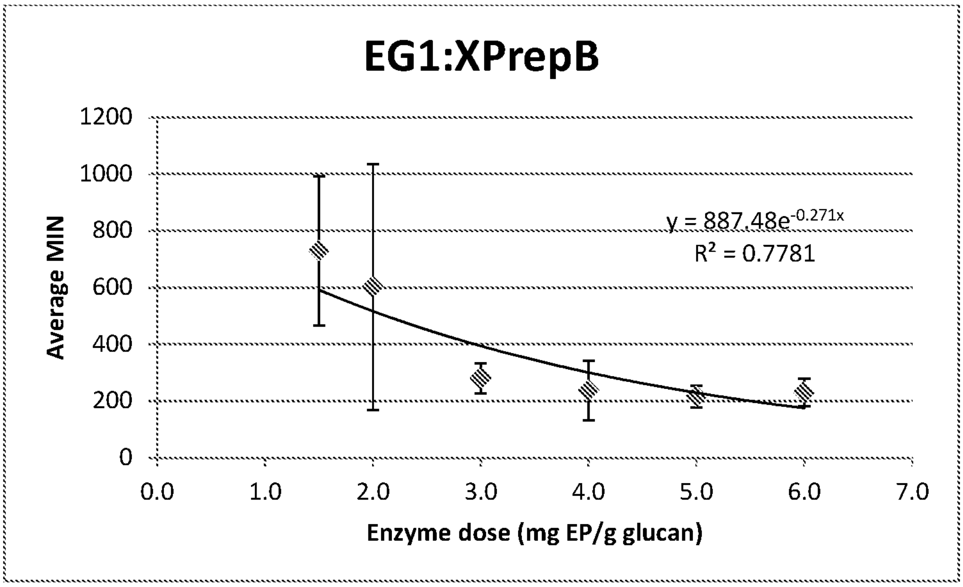
EG1:XPrepB
Average MIN
1200
1000
800
600
400
200
0
$y = 887.48e^{-0.271x}$
$R^2 = 0.7781$
0.0
1.0
2.0
3.0
4.0
5.0
6.0
7.0
Enzyme dose (mg EP/g glucan)

FIG. 5

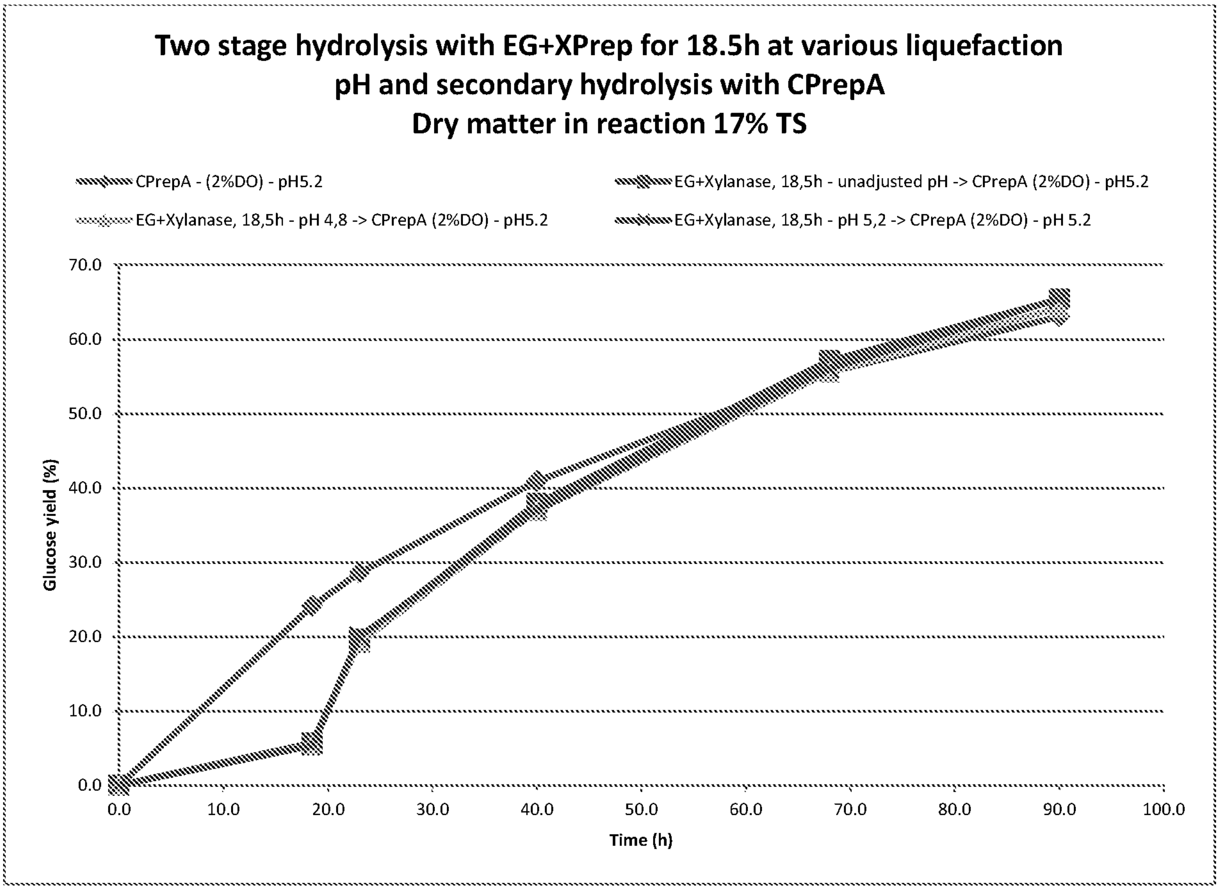
Two stage hydrolysis with EG+XPrep for 18.5h at various liquefaction pH and secondary hydrolysis with CPrepA
Dry matter in reaction 17% TS
CPrepA - (2%DO) - pH5.2
EG+Xylanase, 18,5h - unadjusted pH -> CPrepA (2%DO) - pH5.2
EG+Xylanase, 18,5h - pH 4,8 -> CPrepA (2%DO) - pH5.2
EG+Xylanase, 18,5h - pH 5,2 -> CPrepA (2%DO) - pH 5.2
Glucose yield (%)
70.0
60.0
50.0
40.0
30.0
20.0
10.0
0.0
0.0
10.0
20.0
30.0
40.0
50.0
60.0
70.0
80.0
90.0
100.0
Time (h)

FIG. 6A

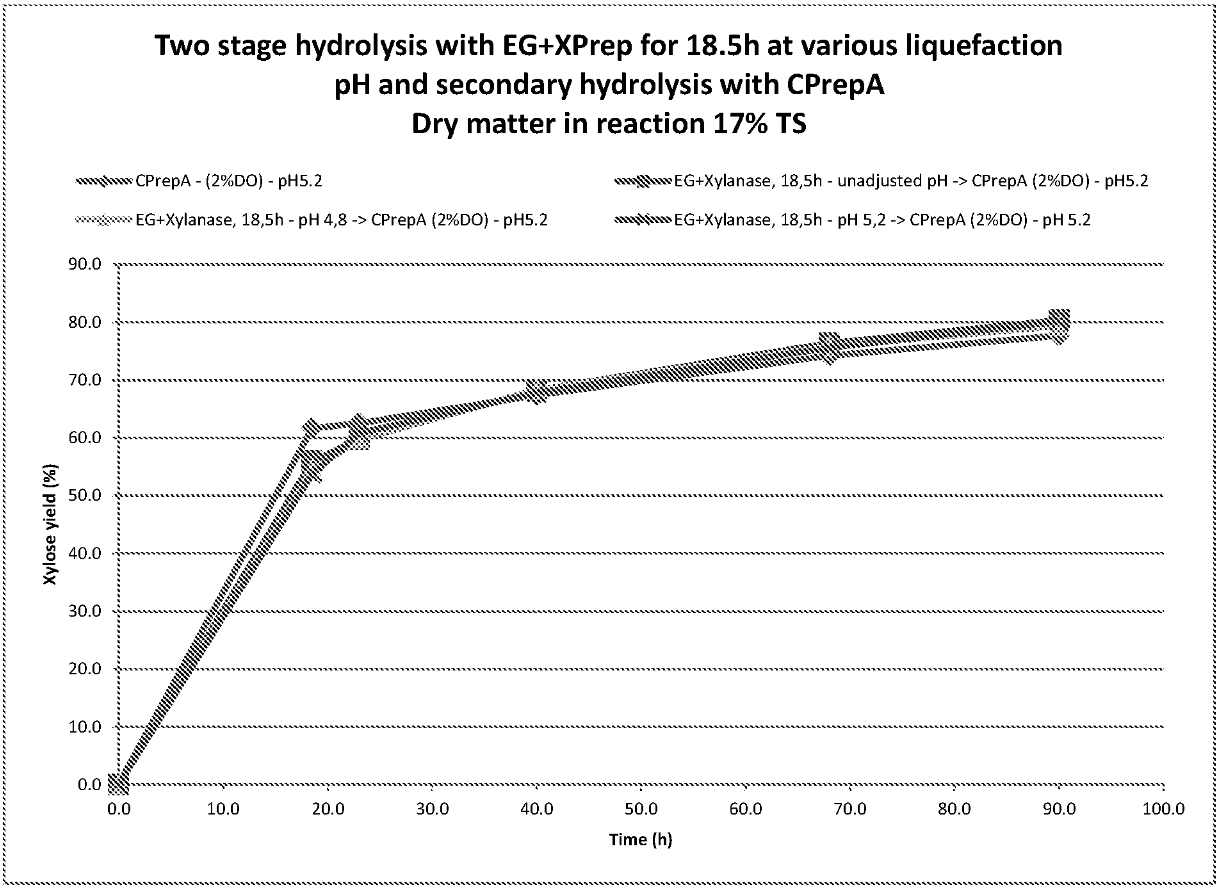
Two stage hydrolysis with EG+XPrep for 18.5h at various liquefaction pH and secondary hydrolysis with CPrepA
Dry matter in reaction 17% TS
CPrepA - (2%DO) - pH5.2
EG+Xylanase, 18,5h - unadjusted pH -> CPrepA (2%DO) - pH5.2
EG+Xylanase, 18,5h - pH 4,8 -> CPrepA (2%DO) - pH5.2
EG+Xylanase, 18,5h - pH 5,2 -> CPrepA (2%DO) - pH 5.2
Xylose yield (%)
90.0
80.0
70.0
60.0
50.0
40.0
30.0
20.0
10.0
0.0
0.0
10.0
20.0
30.0
40.0
50.0
60.0
70.0
80.0
90.0
100.0
Time (h)

FIG. 6B

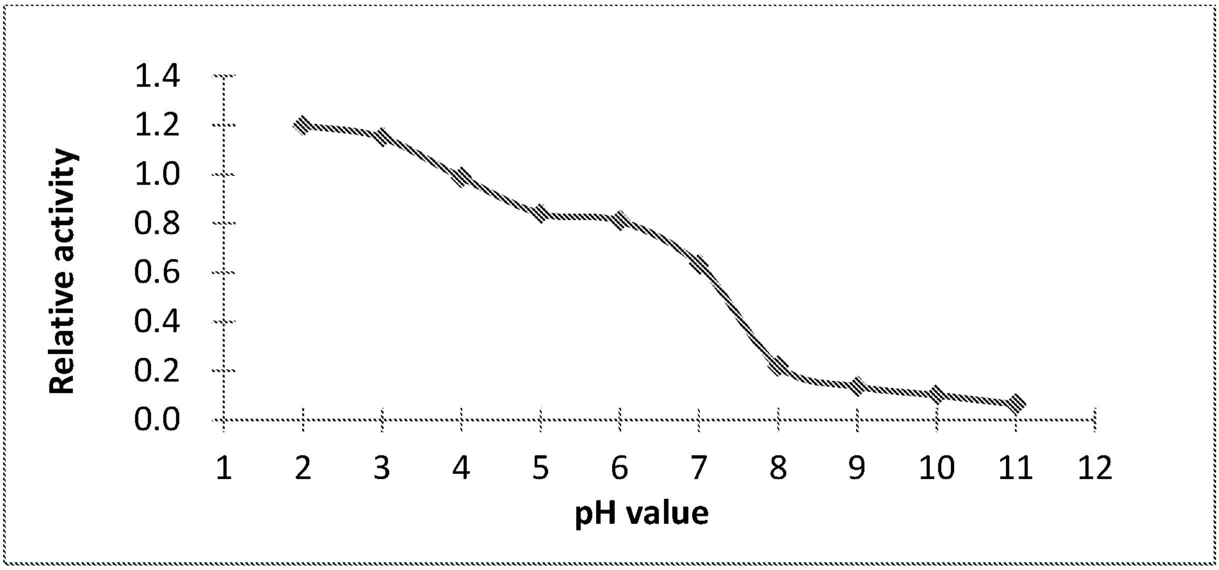
Relative activity
1.4
1.2
1.0
0.8
0.6
0.4
0.2
0.0
1
2
3
4
5
6
7
8
9
10
11
12
pH value

FIG. 7A

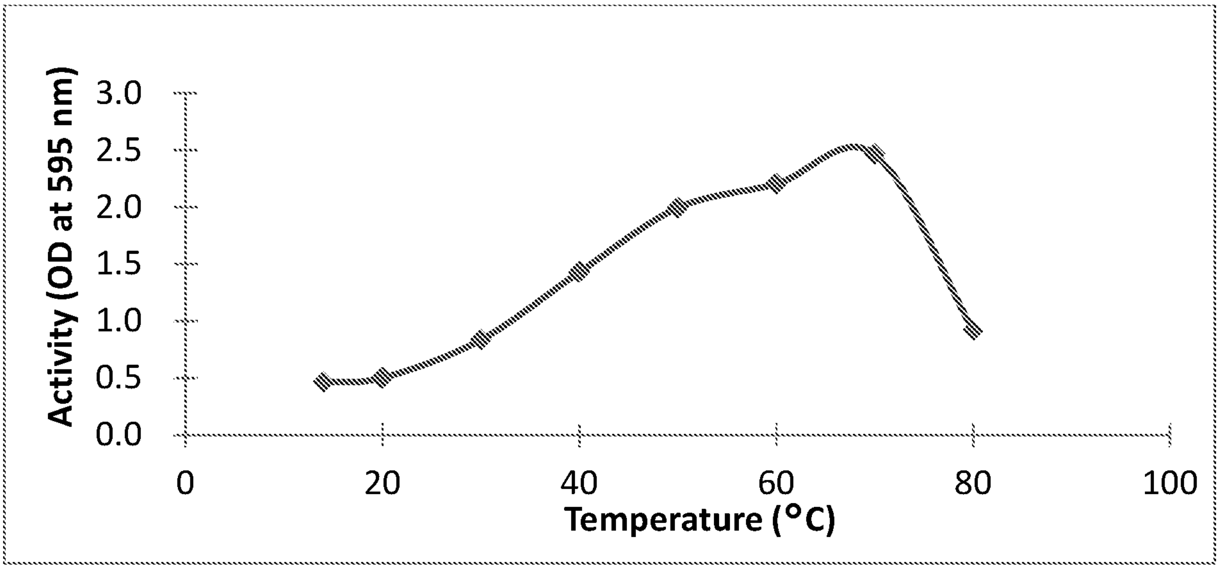
Activity (OD at 595 nm)
3.0
2.5
2.0
1.5
1.0
0.5
0.0
0
20
40
60
80
100
Temperature (°C)

FIG. 7B

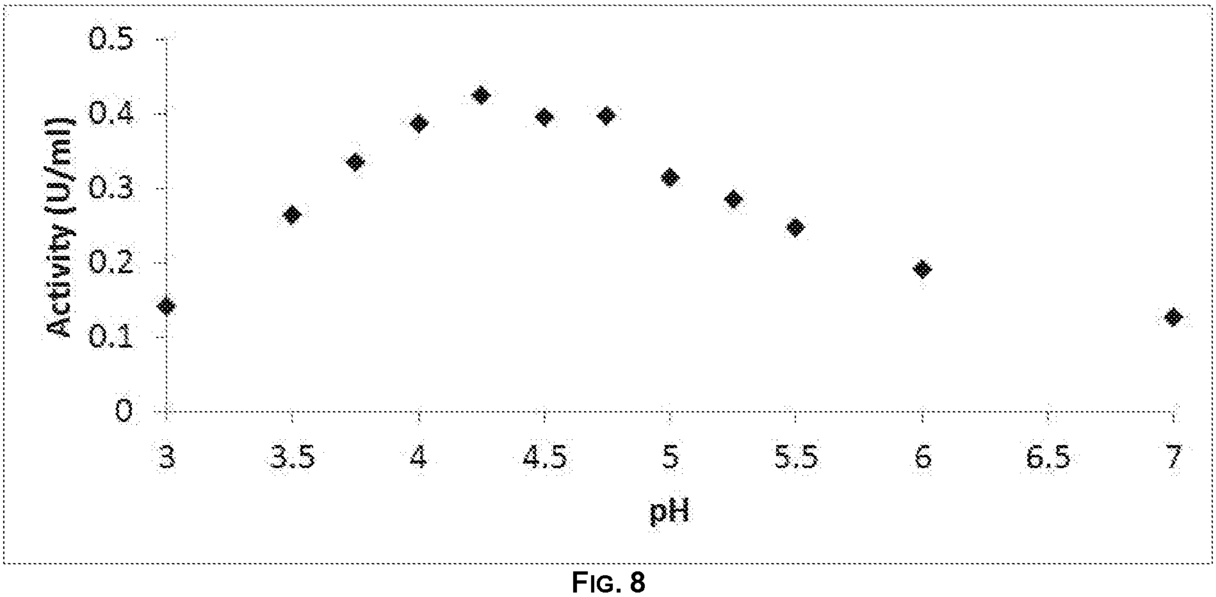
Activity (U/ml)
0.5
0.4
0.3
0.2
0.1
0
3
3.5
4
4.5
5
5.5
6
6.5
7
pH

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02095014 A
- WO 2013028928 A
- WO 2008151043 A
- WO 2012122518 A
- WO 2012021394 A
- WO 2012021395 A
- WO 2012021396 A
- WO 2012021399 A
- WO 2012021400 A
- WO 2012021401 A
- WO 2012021408 A
- WO 2012021410 A
- US 5646025 A
- US 20020164730 A
- WO 2006110891 A
- WO 2006110899 A
- WO 2006110900 A
- WO 2006110901 A
- WO 2006032282 A
- WO 2014108454 A
- WO 9117243 A
- WO 9117244 A
- WO 9421785 A
- WO 2006078256 A
- WO 2011041405 A
- WO 2010126772 A
- WO 2012130965 A
- WO 2012130950 A
- WO 2009079210 A
- WO 2011057083 A
- WO 9105039 A
- WO 9315186 A
- US 5275944 A
- WO 9602551 A
- US 5536655 A
- WO 0070031 A
- WO 05093050 A
- WO 2011057140 A
- WO 2011059740 A
- WO 2009042871 A
- WO 2010141325 A
- WO 2006074435 A
- WO 2010057086 A
- WO 2008057637 A
- WO 2007019442 A
- WO 2010088387 A
- WO 2011035029 A
- WO 2012125925 A
- WO 2010138754 A
- WO 2012122477 A
- WO 2012101206 A
- WO 2012146171 A
- WO 2009085935 A
- WO 2009085859 A
- WO 2009085864 A
- WO 2009085868 A
- WO 2009033071 A
- WO 2012027374 A
- WO 2012068236 A
- WO 2011005867 A
- WO 2011041397 A
- WO 2012000892 A
- WO 2012135659 A
- WO 2012129697 A
- WO 2005074656 A
- WO 2010065830 A
- WO 2011041504 A
- WO 2011039319 A
- WO 2012113340 A
- WO 2012129699 A
- WO 2012130964 A
- WO 2005074647 A
- WO 2008148131 A
- WO 2011035027 A
- WO 2012092676 A
- WO 2012093149 A
- WO 2007089290 A
- WO 2012149344 A
- WO 2005047499 A
- WO 2012044915 A
- WO 2010108918 A
- WO 2009073709 A
- WO 2005001036 A
- WO 2010014880 A
- WO 2009042846 A
- WO 2009076122 A
- WO 2009127729 A
- WO 2010053838 A
- WO 2010065448 A
- WO 2006114094 A
- WO 2009073383 A
- WO 2010014706 A
- WO 2009068565 A
- WO 9015861 A
- WO 2010096673 A
- WO 03062430 A
- WO 2011107472 A1

**Non-patent literature cited in the description**


- **DE VRIES.** *J. Bacteriol.,* 1998, vol. 180, 243-249
- **QUINLAN et al.** *Proc. Natl. Acad. Sci. USA,* 2011, vol. 208, 15079-15084
- **PHILLIPS et al.** *ACS Chem. Biol.,* 2011, vol. 6, 1399-1406
- **LIN et al.** *Structure,* 2012, vol. 20, 1051-1061
- **HENRISSAT.** *Biochem. J.,* 1991, vol. 280, 309-316
- **HENRISSAT ; BAIROCH.** *Biochem. J.,* 1996, vol. 316, 695-696
- **VENTURI et al.** *J. Basic Microbiol.,* 2002, vol. 42, 55-66
- **TEERI.** *Trends in Biotechnology,* 1997, vol. 15, 160-167
- **TEERI et al.** *Biochem. Soc. Trans.,* 1998, vol. 26, 173-178
- **LEVER et al.** *Anal. Biochem.,* 1972, vol. 47, 273-279
- **VAN TILBEURGH et al.** *FEBS Letters,* 1982, vol. 149, 152-156
- **VAN TILBEURGH ; CLAEYSSENS.** *FEBS Letters,* 1985, vol. 187, 283-288
- **TOMME et al.** *Eur. J. Biochem.,* 1988, vol. 170, 575-581
- **ZHANG et al.** *Biotechnology Advances,* 2006, vol. 24, 452-481
- **GHOSE.** *Pure Appl. Chem.,* 1987, vol. 59, 257-68
- **SHALLOM ; SHOHAM.** *Current Opinion In Microbiology,* 2003, vol. 6 (3), 219-228
- **GHOSE ; BISARIA.** *Pure & Appl. Chem.,* 1987, vol. 59, 1739-1752
- **NIELSEN et al.** *Protein Engineering,* 1997, vol. 10, 1-6
- **BENDTSEN et al.** *J. Mol. Biol.,* 2004, vol. 340, 783-795
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet,* 2000, vol. 16, 276-277
- **RICE et al.** *EMBOSS: The European Molecular Biology Open Software Suite,* 2000
- **EBRINGEROVA et al.** *Adv. Polym. Sci.,* 2005, vol. 186, 1-67
- **BIELY ; PUCHARD.** *Journal of the Science of Food and Agriculture,* 2006, vol. 86 (11), 1636-1647
- **SPANIKOVA ; BIELY.** *FEBS Letters,* 2006, vol. 580 (19), 4597-4601
- **HERRMANN et al.** *Biochemical Journal,* 1997, vol. 321, 375-381
- **BAILEY et al.** Interlaboratory testing of methods for assay of xylanase activity. *Journal of Biotechnology,* 1992, vol. 23 (3), 257-270
- **LEVER.** *Anal. Biochem.,* 1972, vol. 47, 273-279
- **WISELOGEL et al.** Handbook on Bioethanol. Taylor & Francis, 1995, 105-118
- **WYMAN.** *Bioresource Technology,* 1994, vol. 50, 3-16
- **LYND.** *Applied Biochemistry and Biotechnology,* 1990, vol. 24/25, 695-719
- Recent Progress in Bioconversion of Lignocellulosics. **MOSIER et al.** Advances in Biochemical Engineering/Biotechnology. Springer-Verlag, 1999, vol. 65, 23-40
- **CHANDRA et al.** *Adv. Biochem. Engin./Biotechnol.,* 2007, vol. 108, 67-93
- **GALBE ; ZACCHI.** *Adv. Biochem. Engin./Biotechnol.,* 2007, vol. 108, 41-65
- **HENDRIKS ; ZEEMAN.** *Bioresource Technology,* 2009, vol. 100, 10-18
- **MOSIER et al.** *Bioresource Technology,* 2005, vol. 96, 673-686
- **TAHERZADEH ; KARIMI.** *Int. J. Mol. Sci.,* 2008, vol. 9, 1621-1651
- **YANG ; WYMAN.** *Biofuels Bioproducts and Biorefining-Biofpr,* 2008, vol. 2, 26-40
- **DUFF ; MURRAY.** *Bioresource Technology,* 1996, vol. 855, 1-33
- **GALBE ; ZACCHI.** *Appl. Microbiol. Biotechnol.,* 2002, vol. 59, 618-628
- **BALLESTEROS et al.** *Appl. Biochem. Biotechnol.,* 2006, vol. 129-132, 496-508
- **VARGA et al.** *Appl. Biochem. Biotechnol.,* 2004, (113-116), 509-523
- **SASSNER et al.** *Enzyme Microb. Technol.,* 2006, vol. 39, 756-762
- **SCHELL et al.** *Bioresource Technology,* 2004, vol. 91, 179-188
- **LEE et al.** *Adv. Biochem. Eng. Biotechnol.,* 1999, vol. 65, 93-115
- **WYMAN et al.** *Bioresource Technology,* 2005, vol. 96, 1959-1966
- **SCHMIDT ; THOMSEN.** *Bioresource Technology,* 1998, vol. 64, 139-151
- **PALONEN et al.** *Appl. Biochem. Biotechnol.,* 2004, vol. 117, 1-17
- **VARGA et al.** *Biotechnol. Bioeng.,* 2004, vol. 88, 567-574
- **MARTIN et al.** *J. Chem. Technol. Biotechnol.,* 2006, vol. 81, 1669-1677
- **GOLLAPALLI et al.** *Appl. Biochem. Biotechnol.,* 2002, vol. 98, 23-35
- **CHUNDAWAT et al.** *Biotechnol. Bioeng.,* 2007, vol. 96, 219-231
- **ALIZADEH et al.** *Appl. Biochem. Biotechnol.,* 2005, vol. 121, 1133-1141
- **TEYMOURI et al.** *Bioresource Technology,* 2005, vol. 96, 2014-2018

- **PAN et al.** *Biotechnol. Bioeng.,* 2005, vol. 90, 473-481
- **PAN et al.** *Biotechnol. Bioeng.,* 2006, vol. 94, 851-861
- **KURABI et al.** *Appl. Biochem. Biotechnol.,* 2005, vol. 121, 219-230
- **SCHELL et al.** *Appl. Biochem. Biotechnol.,* 2003, vol. 105-108, 69-85
- Pretreatment of biomass. **HSU, T.-A.** Handbook on Bioethanol: Production and Utilization. Taylor & Francis, 1996, 179-212
- **GHOSH ; SINGH.** *Adv. Appl. Microbiol.,* 1993, vol. 39, 295-333
- Pretreating lignocellulosic biomass: a review. **MCMILLAN, J. D.** Enzymatic Conversion of Biomass for Fuels Production. ACS Symposium Series, 1994, vol. 566
- Ethanol production from renewable resources. **GONG, C. S. ; CAO, N. J. ; DU, J. ; TSAO, G. T.** Advances in Biochemical Engineering/Biotechnology. Springer-Verlag Berlin Heidelberg, 1999, vol. 65, 207-241
- **OLSSON ; HAHN-HAGERDAL.** *Enz. Microb. Tech.,* 1996, vol. 18, 312-331
- **VALLANDER ; ERIKSSON.** *Adv. Biochem. Eng./Biotechnol.,* 1990, vol. 42, 63-95
- **QING ; WYMAN.** *Biotechnology for Biofuels,* 2011, vol. 4, 18
- **DE CASTILHOS CORAZZA et al.** *Acta Scientiarum. Technology,* 2003, vol. 25, 33-38
- **GUSAKOV ; SINITSYN.** *Enz. Microb. Technol.,* 1985, vol. 7, 346-352
- **RYU ; LEE.** *Biotechnol. Bioeng.,* 1983, vol. 25, 53-65
- **SAARILAHTI et al.** *Gene,* 1990, vol. 90, 9-14
- **OOI et al.** *Nucleic Acids Research,* 1990, vol. 18, 5884
- **SAKAMOTO et al.** *Current Genetics,* 1995, vol. 27, 435-439
- **PENTTILA et al.** *Gene,* 1986, vol. 45, 253-263
- **SALOHEIMO et al.** *Gene,* 1988, vol. 63, 11-22
- **OKADA et al.** *Appl. Environ. Microbiol.,* 1988, vol. 64, 555-563
- **SALOHEIMO et al.** *Molecular Microbiology,* 1994, vol. 13, 219-228
- **KAWAGUCHI et al.** *Gene,* 1996, vol. 173, 287-288
- **DAN et al.** *J. Biol. Chem.,* 2000, vol. 275, 4973-4980
- **PHILIPPIDIS, G. P.** Cellulose bioconversion technology, in Handbook on Bioethanol: Production and Utilization. Taylor & Francis, 1996, 179-212
- **SHEEHAN ; HIMMEL.** *Biotechnol. Prog.,* 1999, vol. 15, 817-827
- **LYND et al.** *Microbiol. Mol. Biol. Reviews,* 2002, vol. 66, 506-577
- **LIN et al.** *Appl. Microbiol. Biotechnol.,* 2006, vol. 69, 627-642
- **CHEN ; HO.** *Appl. Biochem. Biotechnol.,* 1993, vol. 39-40, 135-147
- **HO et al.** *Appl. Environ. Microbiol.,* 1998, vol. 64, 1852-1859
- **KOTTER ; CIRIACY.** *Appl. Microbiol. Biotechnol.,* 1993, vol. 38, 776-783
- **WALFRIDSSON et al.** *Appl. Environ. Microbiol.,* 1995, vol. 61, 4184-4190
- **KUYPER et al.** *FEMS Yeast Research,* 2004, vol. 4, 655-664
- **BEALL et al.** *Biotech. Bioeng.,* 1991, vol. 38, 296-303
- **INGRAM et al.** *Biotechnol. Bioeng.,* 1998, vol. 58, 204-214
- **ZHANG et al.** *Science,* 1995, vol. 267, 240-243
- **DEANDA et al.** *Appl. Environ. Microbiol.,* 1996, vol. 62, 4465-4470
- The Alcohol Textbook. Nottingham University Press, 1999
- Ethanol production from renewable resources. **GONG et al.** Advances in Biochemical Engineering/Biotechnology. Springer-Verlag, 1999, vol. 65, 207-241
- **SILVEIRA ; JONAS.** *Appl. Microbiol. Biotechnol.,* 2002, vol. 59, 400-408
- **NIGAM ; SINGH.** *Process Biochemistry,* 1995, vol. 30 (2), 117-124
- **EZEJI et al.** *World Journal of Microbiology and Biotechnology,* 2003, vol. 19 (6), 595-603
- **RICHARD ; MARGARITIS.** *Biotechnology and Bioengineering,* 2004, vol. 87 (4), 501-515
- **KATAOKA et al.** *Water Science and Technology,* 1997, vol. 36 (6-7), 41-47
- **GUNASEELAN.** *Biomass and Bioenergy,* 1997, vol. 13 (1-2), 83-114
- **CHEN ; LEE.** *Appl. Biochem. Biotechnol.,* 1997, vol. 63-65, 435-448

SEQUENCE LISTING

<110> Novozymes A/S Beta Renewables Spa Frickmann, Jesper Borjesson, Johan Abbate, Eric Osborn, David Moxley, Geoffrey Riva, Daniele Ottonello, Piero Ferrero, Simone Purroti, Micol Paravisi, Stefano Prefumo, Chiara

<120> Multi-Stage Enzymatic Hydrolysis of Biomass

<130> 12823-WO-PCT

<160> 24

<170> PatentIn version 3.5

<210> 1
<211> 3060
<212> DNA
<213> Aspergillus fumigatus

<400> 1

```
atgagattcg gttggctcga ggtggccgct ctgacggccg cttctgtagc caatgcccag      60
gtttgtgatg ctttcccgtc attgtttcgg atatagttga caatagtcat ggaaataatc     120
aggaattggc tttctctcca ccattctacc cttcgccttg ggctgatggc cagggagagt     180
gggcagatgc ccatcgacgc gccgtcgaga tcgtttctca gatgacactg gcggagaagg     240
ttaaccttac aacgggtact gggtgggttg cgactttttt gttgacagtg agctttcttc     300
actgaccatc tacacagatg ggaaatggac cgatgcgtcg gtcaaaccgg cagcgttccc     360
aggtaagctt gcaattctgc aacaacgtgc aagtgtagtt gctaaaacgc ggtggtgcag     420
acttggtatc aactggggtc tttgtggcca ggattcccct ttgggtatcc gtttctgtga     480
gctatacccg cggagtcttt cagtccttgt attatgtgct gatgattgtc tctgtatagc     540
tgacctcaac tccgccttcc ctgctggtac taatgtcgcc gcgacatggg acaagacact     600
cgcctacctt cgtggcaagg ccatgggtga ggaattcaac gacaagggcg tggacatttt     660
gctggggcct gctgctggtc ctctcggcaa atacccggac ggcggcagaa tctgggaagg     720
cttctctcct gatccggttc tcactggtgt acttttcgcc gaaactatca agggtatcca     780
agacgcgggt gtgattgcta ctgccaagca ttacattctg aatgaacagg agcatttccg     840
acaggttggc gaggcccagg gatatggtta caacatcacg gagacgatca gctccaacgt     900
ggatgacaag accatgcacg agttgtacct ttggtgagta gttgacactg caaatgagga     960
ccttgattga tttgactgac ctggaatgca ggccctttgc agatgctgtg cgcggtaaga    1020
```

ttttccgtag acttgacctc gcgacgaaga aatcgctgac gaaccatcgt agctggcgtt 1080
ggcgctgtca tgtgttccta caatcaaatc aacaacagct acggttgtca aaacagtcaa 1140
actctcaaca agctcctcaa ggctgagctg ggcttccaag gcttcgtcat gagtgactgg 1200
agcgctcacc acagcggtgt cggcgctgcc ctcgctgggt tggatatgtc gatgcctgga 1260
gacatttcct tcgacgacgg actctccttc tggggcacga acctaactgt cagtgttctt 1320
aacggcaccg ttccagcctg gcgtgtcgat gacatggctg ttcgtatcat gaccgcgtac 1380
tacaaggttg gtcgtgaccg tcttcgtatt ccccctaact tcagctcctg gacccgggat 1440
gagtacggct gggagcattc tgctgtctcc gagggagcct ggaccaaggt gaacgacttc 1500
gtcaatgtgc agcgcagtca ctctcagatc atccgtgaga ttggtgccgc tagtacagtg 1560
ctcttgaaga acacgggtgc tcttcctttg accggcaagg aggttaaagt gggtgttctc 1620
ggtgaagacg ctggttccaa cccgtggggt gctaacggct gccccgaccg cggctgtgat 1680
aacggcactc ttgctatggc ctggggtagt ggtactgcca acttccctta ccttgtcacc 1740
cccgagcagg ctatccagcg agaggtcatc agcaacggcg gcaatgtctt tgctgtgact 1800
gataacgggg ctctcagcca gatggcagat gttgcatctc aatccaggtg agtgcgggct 1860
cttagaaaaa gaacgttctc tgaatgaagt tttttaacca ttgcgaacag cgtgtctttg 1920
gtgtttgtca acgccgactc tggagagggt ttcatcagtg tcgacggcaa cgagggtgac 1980
cgcaaaaatc tcactctgtg gaagaacggc gaggccgtca ttgacactgt tgtcagccac 2040
tgcaacaaca cgattgtggt tattcacagt gttgggcccg tcttgatcga ccggtggtat 2100
gataacccca acgtcactgc catcatctgg gccggcttgc ccggtcagga gagtggcaac 2160
tccctggtcg acgtgctcta tggccgcgtc aaccccagcg ccaagacccc gttcacctgg 2220
ggcaagactc gggagtctta cggggctccc ttgctcaccg agcctaacaa tggcaatggt 2280
gctccccagg atgatttcaa cgagggcgtc ttcattgact accgtcactt tgacaagcgc 2340
aatgagaccc ccatttatga gtttggccat ggcttgagct acaccacctt tggttactct 2400
caccttcggg ttcaggccct caatagttcg agttcggcat atgtcccgac tagcggagag 2460
accaagcctg cgccaaccta tggtgagatc ggtagtgccg ccgactacct gtatcccgag 2520
ggtctcaaaa gaattaccaa gtttatttac ccttggctca actcgaccga cctcgaggat 2580
tcttctgacg acccgaacta cggctgggag gactcggagt acattcccga aggcgctagg 2640
gatgggtctc ctcaacccct cctgaaggct ggcggcgctc ctggtggtaa ccctaccctt 2700
tatcaggatc ttgttagggt gtcggccacc ataaccaaca ctggtaacgt cgccggttat 2760
gaagtccctc aattggtgag tgacccgcat gttccttgcg ttgcaatttg gctaactcgc 2820
ttctagtatg tttcactggg cggaccgaac gagcctcggg tcgttctgcg caagttcgac 2880

cgaatcttcc tggctcctgg ggagcaaaag gtttggacca cgactcttaa ccgtcgtgat 2940

ctcgccaatt gggatgtgga ggctcaggac tgggtcatca caaagtaccc caagaaagtg 3000

cacgtcggca gctcctcgcg taagctgcct ctgagagcgc ctctgccccg tgtctactag 3060

<210> 2
<211> 863
<212> PRT
<213> Aspergillus fumigatus

<400> 2

```
Met Arg Phe Gly Trp Leu Glu Val Ala Ala Leu Thr Ala Ala Ser Val
1               5                   10                  15

Ala Asn Ala Gln Glu Leu Ala Phe Ser Pro Pro Phe Tyr Pro Ser Pro
            20                  25                  30

Trp Ala Asp Gly Gln Gly Glu Trp Ala Asp Ala His Arg Arg Ala Val
        35                  40                  45

Glu Ile Val Ser Gln Met Thr Leu Ala Glu Lys Val Asn Leu Thr Thr
    50                  55                  60

Gly Thr Gly Trp Glu Met Asp Arg Cys Val Gly Gln Thr Gly Ser Val
65                  70                  75                  80

Pro Arg Leu Gly Ile Asn Trp Gly Leu Cys Gly Gln Asp Ser Pro Leu
                85                  90                  95

Gly Ile Arg Phe Ser Asp Leu Asn Ser Ala Phe Pro Ala Gly Thr Asn
            100                 105                 110

Val Ala Ala Thr Trp Asp Lys Thr Leu Ala Tyr Leu Arg Gly Lys Ala
        115                 120                 125

Met Gly Glu Glu Phe Asn Asp Lys Gly Val Asp Ile Leu Leu Gly Pro
    130                 135                 140

Ala Ala Gly Pro Leu Gly Lys Tyr Pro Asp Gly Gly Arg Ile Trp Glu
145                 150                 155                 160

Gly Phe Ser Pro Asp Pro Val Leu Thr Gly Val Leu Phe Ala Glu Thr
                165                 170                 175

Ile Lys Gly Ile Gln Asp Ala Gly Val Ile Ala Thr Ala Lys His Tyr
            180                 185                 190

Ile Leu Asn Glu Gln Glu His Phe Arg Gln Val Gly Glu Ala Gln Gly
```

195 200 205

Tyr Gly Tyr Asn Ile Thr Glu Thr Ile Ser Ser Asn Val Asp Asp Lys
210 215 220

Thr Met His Glu Leu Tyr Leu Trp Pro Phe Ala Asp Ala Val Arg Ala
225 230 235 240

Gly Val Gly Ala Val Met Cys Ser Tyr Asn Gln Ile Asn Asn Ser Tyr
245 250 255

Gly Cys Gln Asn Ser Gln Thr Leu Asn Lys Leu Leu Lys Ala Glu Leu
260 265 270

Gly Phe Gln Gly Phe Val Met Ser Asp Trp Ser Ala His His Ser Gly
275 280 285

Val Gly Ala Ala Leu Ala Gly Leu Asp Met Ser Met Pro Gly Asp Ile
290 295 300

Ser Phe Asp Asp Gly Leu Ser Phe Trp Gly Thr Asn Leu Thr Val Ser
305 310 315 320

Val Leu Asn Gly Thr Val Pro Ala Trp Arg Val Asp Asp Met Ala Val
325 330 335

Arg Ile Met Thr Ala Tyr Tyr Lys Val Gly Arg Asp Arg Leu Arg Ile
340 345 350

Pro Pro Asn Phe Ser Ser Trp Thr Arg Asp Glu Tyr Gly Trp Glu His
355 360 365

Ser Ala Val Ser Glu Gly Ala Trp Thr Lys Val Asn Asp Phe Val Asn
370 375 380

Val Gln Arg Ser His Ser Gln Ile Ile Arg Glu Ile Gly Ala Ala Ser
385 390 395 400

Thr Val Leu Leu Lys Asn Thr Gly Ala Leu Pro Leu Thr Gly Lys Glu
405 410 415

Val Lys Val Gly Val Leu Gly Glu Asp Ala Gly Ser Asn Pro Trp Gly
420 425 430

Ala Asn Gly Cys Pro Asp Arg Gly Cys Asp Asn Gly Thr Leu Ala Met
435 440 445

```
Ala Trp Gly Ser Gly Thr Ala Asn Phe Pro Tyr Leu Val Thr Pro Glu
    450                 455                 460

Gln Ala Ile Gln Arg Glu Val Ile Ser Asn Gly Gly Asn Val Phe Ala
465                 470                 475                 480

Val Thr Asp Asn Gly Ala Leu Ser Gln Met Ala Asp Val Ala Ser Gln
                485                 490                 495

Ser Ser Val Ser Leu Val Phe Val Asn Ala Asp Ser Gly Glu Gly Phe
            500                 505                 510

Ile Ser Val Asp Gly Asn Glu Gly Asp Arg Lys Asn Leu Thr Leu Trp
        515                 520                 525

Lys Asn Gly Glu Ala Val Ile Asp Thr Val Val Ser His Cys Asn Asn
    530                 535                 540

Thr Ile Val Val Ile His Ser Val Gly Pro Val Leu Ile Asp Arg Trp
545                 550                 555                 560

Tyr Asp Asn Pro Asn Val Thr Ala Ile Ile Trp Ala Gly Leu Pro Gly
                565                 570                 575

Gln Glu Ser Gly Asn Ser Leu Val Asp Val Leu Tyr Gly Arg Val Asn
            580                 585                 590

Pro Ser Ala Lys Thr Pro Phe Thr Trp Gly Lys Thr Arg Glu Ser Tyr
        595                 600                 605

Gly Ala Pro Leu Leu Thr Glu Pro Asn Asn Gly Asn Gly Ala Pro Gln
    610                 615                 620

Asp Asp Phe Asn Glu Gly Val Phe Ile Asp Tyr Arg His Phe Asp Lys
625                 630                 635                 640

Arg Asn Glu Thr Pro Ile Tyr Glu Phe Gly His Gly Leu Ser Tyr Thr
                645                 650                 655

Thr Phe Gly Tyr Ser His Leu Arg Val Gln Ala Leu Asn Ser Ser Ser
            660                 665                 670

Ser Ala Tyr Val Pro Thr Ser Gly Glu Thr Lys Pro Ala Pro Thr Tyr
        675                 680                 685

Gly Glu Ile Gly Ser Ala Ala Asp Tyr Leu Tyr Pro Glu Gly Leu Lys
    690                 695                 700
```

```
Arg Ile Thr Lys Phe Ile Tyr Pro Trp Leu Asn Ser Thr Asp Leu Glu
705                 710                 715                 720

Asp Ser Ser Asp Asp Pro Asn Tyr Gly Trp Glu Asp Ser Glu Tyr Ile
                725                 730                 735

Pro Glu Gly Ala Arg Asp Gly Ser Pro Gln Pro Leu Leu Lys Ala Gly
            740                 745                 750

Gly Ala Pro Gly Gly Asn Pro Thr Leu Tyr Gln Asp Leu Val Arg Val
        755                 760                 765

Ser Ala Thr Ile Thr Asn Thr Gly Asn Val Ala Gly Tyr Glu Val Pro
    770                 775                 780

Gln Leu Tyr Val Ser Leu Gly Gly Pro Asn Glu Pro Arg Val Val Leu
785                 790                 795                 800

Arg Lys Phe Asp Arg Ile Phe Leu Ala Pro Gly Glu Gln Lys Val Trp
                805                 810                 815

Thr Thr Thr Leu Asn Arg Arg Asp Leu Ala Asn Trp Asp Val Glu Ala
            820                 825                 830

Gln Asp Trp Val Ile Thr Lys Tyr Pro Lys Lys Val His Val Gly Ser
        835                 840                 845

Ser Ser Arg Lys Leu Pro Leu Arg Ala Pro Leu Pro Arg Val Tyr
    850                 855                 860
```

<210> 3
<211> 3060
<212> DNA
<213> Aspergillus fumigatus

<400> 3

atgagattcg gttggctcga ggtggccgct ctgacggccg cttctgtagc caatgcccag 60

gtttgtgatg ctttcccgtc attgtttcgg atatagttga caatagtcat ggaaataatc 120

aggaattggc tttctctcca ccattctacc cttcgccttg ggctgatggc cagggagagt 180

gggcagatgc ccatcgacgc gccgtcgaga tcgtttctca gatgacactg gcggagaagg 240

ttaaccttac aacgggtact gggtgggttg cgactttttt gttgacagtg agctttcttc 300

actgaccatc tacacagatg ggaaatggac cgatgcgtcg gtcaaaccgg cagcgttccc 360

aggtaagctt gcaattctgc aacaacgtgc aagtgtagtt gctaaaacgc ggtggtgcag 420

acttggtatc aactggggtc tttgtggcca ggattcccct ttgggtatcc gtgactgtga 480

gctatacccg cggagtcttt cagtccttgt attatgtgct gatgattgtc tctgtatagc 540

tgacctcaac tccgccttcc ctgctggtac taatgtcgcc gcgacatggg acaagacact 600

cgcctacctt cgtggcaagg ccatgggtga ggaattcaac gacaagggcg tggacatttt 660

gctggggcct gctgctggtc ctctcggcaa atacccggac ggcggcagaa tctgggaagg 720

cttctctcct gatccggttc tcactggtgt acttttcgcc gaaactatca agggtatcca 780

agacgcgggt gtgattgcta ctgccaagca ttacattctg aatgaacagg agcatttccg 840

acaggttggc gaggcccagg gatatggtta caacatcacg gagacgatca gctccaacgt 900

ggatgacaag accatgcacg agttgtacct ttggtgagta gttgacactg caaatgagga 960

ccttgattga tttgactgac ctggaatgca ggccctttgc agatgctgtg cgcggtaaga 1020

ttttccgtag acttgacctc gcgacgaaga aatcgctgac gaaccatcgt agctggcgtt 1080

ggcgctgtca tgtgttccta caatcaaatc aacaacagct acggttgtca aaacagtcaa 1140

actctcaaca agctcctcaa ggctgagctg ggcttccaag gcttcgtcat gagtgactgg 1200

ggcgctcacc acagcggtgt cggcgctgcc ctcgctgggt tggatatgtc gatgcctgga 1260

gacatttcct tcgacgacgg actctccttc tggggcacga acctaactgt cagtgttctt 1320

aacggcaccg ttccagcctg gcgtgtcgat gacatggctg ttcgtatcat gaccgcgtac 1380

tacaaggttg gtcgtgaccg tcttcgtatt ccccctaact tcagctcctg gacccgggat 1440

gagtacggct gggagcattc tgctgtctcc gagggagcct ggaccaaggt gaacgacttc 1500

gtcaatgtgc agcgcagtca ctctcagatc atccgtgaga ttggtgccgc tagtacagtg 1560

ctcttgaaga acacgggtgc tcttcctttg accggcaagg aggttaaagt gggtgttctc 1620

ggtgaagacg ctggttccaa cccgtggggt gctaacggct gccccgaccg cggctgtgat 1680

aacggcactc ttgctatggc ctggggtagt ggtactgccg agttccctta ccttgtcacc 1740

cccgagcagg ctatccagcg agaggtcatc agcaacggcg gcaatgtctt tgctgtgact 1800

gataacgggg ctctcagcca gatggcagat gttgcatctc aatccaggtg agtgcgggct 1860

cttagaaaaa gaacgttctc tgaatgaagt tttttaacca ttgcgaacag cgtgtctttg 1920

gtgtttgtca acgccgactc tggagagggt tacatcagtg tcgacggcaa cgagggtgac 1980

cgcaaaaatc tcactctgtg gaagaacggc gaggccgtca ttgacactgt tgtcagccac 2040

tgcaacaaca cgattgtggt tattcacagt gttgggcccg tcttgatcga ccggtggtat 2100

gataacccca acgtcactgc catcatctgg gccggcttgc ccggtcagga gagtggcaac 2160

tccctggtcg acgtgctcta tggccgcgtc aaccccagcg ccaagacccc gttcacctgg 2220

ggcaagactc gggagtctta cggggctccc ttgctcaccg agcctaacaa tggcaatggt 2280

gctccccagg atgatttcaa cgagggcgtc ttcattgact accgtcactt tgacaagcgc 2340

aatgagaccc ccatttatga gtttggccat ggcttgagct acaccacctt tggttactct 2400

```
caccttcggg ttcaggccct caatagttcg agttcggcat atgtcccgac tagcggagag     2460
accaagcctg cgccaaccta tggtgagatc ggtagtgccg ccgactacct gtatcccgag     2520
ggtctcaaaa gaattaccaa gtttatttac ccttggctca actcgaccga cctcgaggat     2580
tcttctgacg acccgaacta cggctgggag gactcggagt acattcccga aggcgctagg     2640
gatgggtctc ctcaacccct cctgaaggct ggcggcgctc ctggtggtaa ccctaccctt     2700
tatcaggatc ttgttagggt gtcggccacc ataaccaaca ctggtaacgt cgccggttat     2760
gaagtccctc aattggtgag tgacccgcat gttccttgcg ttgcaatttg gctaactcgc     2820
ttctagtatg tttcactggg cggaccgaac gagcctcggg tcgttctgcg caagttcgac     2880
cgaatcttcc tggctcctgg ggagcaaaag gtttggacca cgactcttaa ccgtcgtgat     2940
ctcgccaatt gggatgtgga ggctcaggac tgggtcatca caaagtaccc caagaaagtg     3000
cacgtcggca gctcctcgcg taagctgcct ctgagagcgc ctctgccccg tgtctactag     3060
```

<210> 4
<211> 863
<212> PRT
<213> Aspergillus fumigatus

<400> 4

```
Met Arg Phe Gly Trp Leu Glu Val Ala Ala Leu Thr Ala Ala Ser Val
1               5                   10                  15

Ala Asn Ala Gln Glu Leu Ala Phe Ser Pro Pro Phe Tyr Pro Ser Pro
            20                  25                  30

Trp Ala Asp Gly Gln Gly Glu Trp Ala Asp Ala His Arg Arg Ala Val
        35                  40                  45

Glu Ile Val Ser Gln Met Thr Leu Ala Glu Lys Val Asn Leu Thr Thr
    50                  55                  60

Gly Thr Gly Trp Glu Met Asp Arg Cys Val Gly Gln Thr Gly Ser Val
65                  70                  75                  80

Pro Arg Leu Gly Ile Asn Trp Gly Leu Cys Gly Gln Asp Ser Pro Leu
                85                  90                  95

Gly Ile Arg Asp Ser Asp Leu Asn Ser Ala Phe Pro Ala Gly Thr Asn
            100                 105                 110

Val Ala Ala Thr Trp Asp Lys Thr Leu Ala Tyr Leu Arg Gly Lys Ala
        115                 120                 125
```

```
Met Gly Glu Glu Phe Asn Asp Lys Gly Val Asp Ile Leu Leu Gly Pro
    130                 135                 140

Ala Ala Gly Pro Leu Gly Lys Tyr Pro Asp Gly Gly Arg Ile Trp Glu
145                 150                 155                 160

Gly Phe Ser Pro Asp Pro Val Leu Thr Gly Val Leu Phe Ala Glu Thr
                165                 170                 175

Ile Lys Gly Ile Gln Asp Ala Gly Val Ile Ala Thr Ala Lys His Tyr
            180                 185                 190

Ile Leu Asn Glu Gln Glu His Phe Arg Gln Val Gly Glu Ala Gln Gly
        195                 200                 205

Tyr Gly Tyr Asn Ile Thr Glu Thr Ile Ser Ser Asn Val Asp Asp Lys
    210                 215                 220

Thr Met His Glu Leu Tyr Leu Trp Pro Phe Ala Asp Ala Val Arg Ala
225                 230                 235                 240

Gly Val Gly Ala Val Met Cys Ser Tyr Asn Gln Ile Asn Asn Ser Tyr
                245                 250                 255

Gly Cys Gln Asn Ser Gln Thr Leu Asn Lys Leu Leu Lys Ala Glu Leu
            260                 265                 270

Gly Phe Gln Gly Phe Val Met Ser Asp Trp Gly Ala His His Ser Gly
        275                 280                 285

Val Gly Ala Ala Leu Ala Gly Leu Asp Met Ser Met Pro Gly Asp Ile
    290                 295                 300

Ser Phe Asp Asp Gly Leu Ser Phe Trp Gly Thr Asn Leu Thr Val Ser
305                 310                 315                 320

Val Leu Asn Gly Thr Val Pro Ala Trp Arg Val Asp Asp Met Ala Val
                325                 330                 335

Arg Ile Met Thr Ala Tyr Tyr Lys Val Gly Arg Asp Arg Leu Arg Ile
            340                 345                 350

Pro Pro Asn Phe Ser Ser Trp Thr Arg Asp Glu Tyr Gly Trp Glu His
        355                 360                 365

Ser Ala Val Ser Glu Gly Ala Trp Thr Lys Val Asn Asp Phe Val Asn
    370                 375                 380
```

```
Val Gln Arg Ser His Ser Gln Ile Ile Arg Glu Ile Gly Ala Ala Ser
385                 390                 395                 400

Thr Val Leu Leu Lys Asn Thr Gly Ala Leu Pro Leu Thr Gly Lys Glu
                405                 410                 415

Val Lys Val Gly Val Leu Gly Glu Asp Ala Gly Ser Asn Pro Trp Gly
            420                 425                 430

Ala Asn Gly Cys Pro Asp Arg Gly Cys Asp Asn Gly Thr Leu Ala Met
        435                 440                 445

Ala Trp Gly Ser Gly Thr Ala Glu Phe Pro Tyr Leu Val Thr Pro Glu
    450                 455                 460

Gln Ala Ile Gln Arg Glu Val Ile Ser Asn Gly Gly Asn Val Phe Ala
465                 470                 475                 480

Val Thr Asp Asn Gly Ala Leu Ser Gln Met Ala Asp Val Ala Ser Gln
                485                 490                 495

Ser Ser Val Ser Leu Val Phe Val Asn Ala Asp Ser Gly Glu Gly Tyr
            500                 505                 510

Ile Ser Val Asp Gly Asn Glu Gly Asp Arg Lys Asn Leu Thr Leu Trp
        515                 520                 525

Lys Asn Gly Glu Ala Val Ile Asp Thr Val Val Ser His Cys Asn Asn
    530                 535                 540

Thr Ile Val Val Ile His Ser Val Gly Pro Val Leu Ile Asp Arg Trp
545                 550                 555                 560

Tyr Asp Asn Pro Asn Val Thr Ala Ile Ile Trp Ala Gly Leu Pro Gly
                565                 570                 575

Gln Glu Ser Gly Asn Ser Leu Val Asp Val Leu Tyr Gly Arg Val Asn
            580                 585                 590

Pro Ser Ala Lys Thr Pro Phe Thr Trp Gly Lys Thr Arg Glu Ser Tyr
        595                 600                 605

Gly Ala Pro Leu Leu Thr Glu Pro Asn Asn Gly Asn Gly Ala Pro Gln
    610                 615                 620

Asp Asp Phe Asn Glu Gly Val Phe Ile Asp Tyr Arg His Phe Asp Lys
625                 630                 635                 640
```

```
Arg Asn Glu Thr Pro Ile Tyr Glu Phe Gly His Gly Leu Ser Tyr Thr
                645                 650                 655

Thr Phe Gly Tyr Ser His Leu Arg Val Gln Ala Leu Asn Ser Ser Ser
            660                 665                 670

Ser Ala Tyr Val Pro Thr Ser Gly Glu Thr Lys Pro Ala Pro Thr Tyr
        675                 680                 685

Gly Glu Ile Gly Ser Ala Ala Asp Tyr Leu Tyr Pro Glu Gly Leu Lys
    690                 695                 700

Arg Ile Thr Lys Phe Ile Tyr Pro Trp Leu Asn Ser Thr Asp Leu Glu
705                 710                 715                 720

Asp Ser Ser Asp Asp Pro Asn Tyr Gly Trp Glu Asp Ser Glu Tyr Ile
                725                 730                 735

Pro Glu Gly Ala Arg Asp Gly Ser Pro Gln Pro Leu Leu Lys Ala Gly
            740                 745                 750

Gly Ala Pro Gly Gly Asn Pro Thr Leu Tyr Gln Asp Leu Val Arg Val
        755                 760                 765

Ser Ala Thr Ile Thr Asn Thr Gly Asn Val Ala Gly Tyr Glu Val Pro
    770                 775                 780

Gln Leu Tyr Val Ser Leu Gly Gly Pro Asn Glu Pro Arg Val Val Leu
785                 790                 795                 800

Arg Lys Phe Asp Arg Ile Phe Leu Ala Pro Gly Glu Gln Lys Val Trp
                805                 810                 815

Thr Thr Thr Leu Asn Arg Arg Asp Leu Ala Asn Trp Asp Val Glu Ala
            820                 825                 830

Gln Asp Trp Val Ile Thr Lys Tyr Pro Lys Lys Val His Val Gly Ser
        835                 840                 845

Ser Ser Arg Lys Leu Pro Leu Arg Ala Pro Leu Pro Arg Val Tyr
    850                 855                 860
```

<210> 5
<211> 1599
<212> DNA
<213> Aspergillus fumigatus

<400> 5

```
atgctggcct ccaccttctc ctaccgcatg tacaagaccg cgctcatcct ggccgccctt      60
ctgggctctg gccaggctca gcaggtcggt acttcccagg cggaagtgca tccgtccatg     120
acctggcaga gctgcacggc tggcggcagc tgcaccacca acaacggcaa ggtggtcatc     180
gacgcgaact ggcgttgggt gcacaaagtc ggcgactaca ccaactgcta caccggcaac     240
acctgggaca cgactatctg ccctgacgat gcgacctgcg catccaactg cgcccttgag     300
ggtgccaact acgaatccac ctatggtgtg accgccagcg gcaattccct ccgcctcaac     360
ttcgtcacca ccagccagca gaagaacatt ggctcgcgtc tgtacatgat gaaggacgac     420
tcgacctacg agatgtttaa gctgctgaac caggagttca ccttcgatgt cgatgtctcc     480
aacctcccct gcggtctcaa cggtgctctg tactttgtcg ccatggacgc cgacggtggc     540
atgtccaagt acccaaccaa caaggccggt gccaagtacg gtactggata ctgtgactcg     600
cagtgccctc gcgacctcaa gttcatcaac ggtcaggcca acgtcgaagg gtggcagccc     660
tcctccaacg atgccaatgc gggtaccggc aaccacgggt cctgctgcgc ggagatggat     720
atctgggagg ccaacagcat ctccacggcc ttcaccccc atccgtgcga cacgcccggc      780
caggtgatgt gcaccggtga tgcctgcggt ggcacctaca gctccgaccg ctacggcggc     840
acctgcgacc ccgacggatg tgatttcaac tccttccgcc agggcaacaa gaccttctac     900
ggccctggca tgaccgtcga caccaagagc aagtttaccg tcgtcaccca gttcatcacc     960
gacgacggca cctccagcgg caccctcaag gagatcaagc gcttctacgt gcagaacggc    1020
aaggtgatcc ccaactcgga gtcgacctgg accggcgtca gcggcaactc catcaccacc    1080
gagtactgca ccgcccagaa gagcctgttc caggaccaga acgtcttcga aaagcacggc    1140
ggcctcgagg gcatgggtgc tgccctcgcc cagggtatgg ttctcgtcat gtccctgtgg    1200
gatgatcact cggccaacat gctctggctc gacagcaact acccgaccac tgcctcttcc    1260
accactcccg gcgtcgcccg tggtacctgc gacatctcct ccggcgtccc tgcggatgtc    1320
gaggcgaacc accccgacgc ctacgtcgtc tactccaaca tcaaggtcgg ccccatcggc    1380
tcgaccttca acagcggtgg ctcgaacccc ggtggcggaa ccaccacgac aactaccacc    1440
cagcctacta ccaccacgac cacggctgga aaccctggcg gcaccggagt cgcacagcac    1500
tatggccagt gtggtggaat cggatggacc ggacccacaa cctgtgccag cccttatacc    1560
tgccagaagc tgaatgatta ttactctcag tgcctgtag                           1599
```

<210> 6
<211> 532
<212> PRT
<213> Aspergillus fumigatus

<400> 6

```
Met Leu Ala Ser Thr Phe Ser Tyr Arg Met Tyr Lys Thr Ala Leu Ile
1               5                   10                  15

Leu Ala Ala Leu Leu Gly Ser Gly Gln Ala Gln Gln Val Gly Thr Ser
            20                  25                  30

Gln Ala Glu Val His Pro Ser Met Thr Trp Gln Ser Cys Thr Ala Gly
        35                  40                  45

Gly Ser Cys Thr Thr Asn Asn Gly Lys Val Val Ile Asp Ala Asn Trp
    50                  55                  60

Arg Trp Val His Lys Val Gly Asp Tyr Thr Asn Cys Tyr Thr Gly Asn
65                  70                  75                  80

Thr Trp Asp Thr Thr Ile Cys Pro Asp Asp Ala Thr Cys Ala Ser Asn
                85                  90                  95

Cys Ala Leu Glu Gly Ala Asn Tyr Glu Ser Thr Tyr Gly Val Thr Ala
            100                 105                 110

Ser Gly Asn Ser Leu Arg Leu Asn Phe Val Thr Thr Ser Gln Gln Lys
        115                 120                 125

Asn Ile Gly Ser Arg Leu Tyr Met Met Lys Asp Asp Ser Thr Tyr Glu
    130                 135                 140

Met Phe Lys Leu Leu Asn Gln Glu Phe Thr Phe Asp Val Asp Val Ser
145                 150                 155                 160

Asn Leu Pro Cys Gly Leu Asn Gly Ala Leu Tyr Phe Val Ala Met Asp
                165                 170                 175

Ala Asp Gly Gly Met Ser Lys Tyr Pro Thr Asn Lys Ala Gly Ala Lys
            180                 185                 190

Tyr Gly Thr Gly Tyr Cys Asp Ser Gln Cys Pro Arg Asp Leu Lys Phe
        195                 200                 205

Ile Asn Gly Gln Ala Asn Val Glu Gly Trp Gln Pro Ser Ser Asn Asp
    210                 215                 220

Ala Asn Ala Gly Thr Gly Asn His Gly Ser Cys Cys Ala Glu Met Asp
225                 230                 235                 240

Ile Trp Glu Ala Asn Ser Ile Ser Thr Ala Phe Thr Pro His Pro Cys
                245                 250                 255
```

Asp Thr Pro Gly Gln Val Met Cys Thr Gly Asp Ala Cys Gly Gly Thr
260 265 270

Tyr Ser Ser Asp Arg Tyr Gly Gly Thr Cys Asp Pro Asp Gly Cys Asp
275 280 285

Phe Asn Ser Phe Arg Gln Gly Asn Lys Thr Phe Tyr Gly Pro Gly Met
290 295 300

Thr Val Asp Thr Lys Ser Lys Phe Thr Val Val Thr Gln Phe Ile Thr
305 310 315 320

Asp Asp Gly Thr Ser Ser Gly Thr Leu Lys Glu Ile Lys Arg Phe Tyr
325 330 335

Val Gln Asn Gly Lys Val Ile Pro Asn Ser Glu Ser Thr Trp Thr Gly
340 345 350

Val Ser Gly Asn Ser Ile Thr Thr Glu Tyr Cys Thr Ala Gln Lys Ser
355 360 365

Leu Phe Gln Asp Gln Asn Val Phe Glu Lys His Gly Gly Leu Glu Gly
370 375 380

Met Gly Ala Ala Leu Ala Gln Gly Met Val Leu Val Met Ser Leu Trp
385 390 395 400

Asp Asp His Ser Ala Asn Met Leu Trp Leu Asp Ser Asn Tyr Pro Thr
405 410 415

Thr Ala Ser Ser Thr Thr Pro Gly Val Ala Arg Gly Thr Cys Asp Ile
420 425 430

Ser Ser Gly Val Pro Ala Asp Val Glu Ala Asn His Pro Asp Ala Tyr
435 440 445

Val Val Tyr Ser Asn Ile Lys Val Gly Pro Ile Gly Ser Thr Phe Asn
450 455 460

Ser Gly Gly Ser Asn Pro Gly Gly Gly Thr Thr Thr Thr Thr Thr Thr
465 470 475 480

Gln Pro Thr Thr Thr Thr Thr Thr Ala Gly Asn Pro Gly Gly Thr Gly
485 490 495

Val Ala Gln His Tyr Gly Gln Cys Gly Gly Ile Gly Trp Thr Gly Pro
500 505 510

```
Thr Thr Cys Ala Ser Pro Tyr Thr Cys Gln Lys Leu Asn Asp Tyr Tyr
        515                 520                 525

Ser Gln Cys Leu
    530
```

<210> 7
<211> 1713
<212> DNA
<213> Aspergillus fumigatus

<400> 7

atgaagcacc ttgcatcttc catcgcattg actctactgt tgcctgccgt gcaggcccag 60
cagaccgtat ggggccaatg tatgttctgg ctgtcactgg aataagactg tatcaactgc 120
tgatatgctt ctaggtggcg gccaaggctg gtctggcccg acgagctgtg ttgccggcgc 180
agcctgtagc acactgaatc cctgtatgtt agatatcgtc ctgagtggag acttatactg 240
acttccttag actacgctca gtgtatcccg ggagccaccg cgacgtccac caccctcacg 300
acgacgacgg cggcgacgac gacatcccag accaccacca aacctaccac gactggtcca 360
actacatccg cacccaccgt gaccgcatcc ggtaaccctt tcagcggcta ccagctgtat 420
gccaacccct actactcctc cgaggtccat actctggcca tgccttctct gcccagctcg 480
ctgcagccca aggctagtgc tgttgctgaa gtgccctcat ttgtttggct gtaagtggcc 540
ttatcccaat actgagacca actctctgac agtcgtagcg acgttgccgc caaggtgccc 600
actatgggaa cctacctggc cgacattcag gccaagaaca aggccggcgc caaccctcct 660
atcgctggta tcttcgtggt ctacgacttg ccggaccgtg actgcgccgc tctggccagt 720
aatggcgagt actcaattgc caacaacggt gtggccaact acaaggcgta cattgacgcc 780
atccgtgctc agctggtgaa gtactctgac gttcacacca tcctcgtcat cggtaggccg 840
tacacctccg ttgcgcgccg cctttctctg acatcttgca gaacccgaca gcttggccaa 900
cctggtgacc aacctcaacg tcgccaaatg cgccaatgcg cagagcgcct acctggagtg 960
tgtcgactat gctctgaagc agctcaacct gcccaacgtc gccatgtacc tcgacgcagg 1020
tatgcctcac ttcccgcatt ctgtatccct tccagacact aactcatcag gccatgcggg 1080
ctggctcgga tggcccgcca acttgggccc cgccgcaaca ctcttcgcca aagtctacac 1140
cgacgcgggt tcccccgcgg ctgttcgtgg cctggccacc aacgtcgcca actacaacgc 1200
ctggtcgctc agtacctgcc cctcctacac ccagggagac cccaactgcg acgagaagaa 1260
gtacatcaac gccatggcgc ctcttctcaa ggaagccggc ttcgatgccc acttcatcat 1320
ggatacctgt aagtgcttat tccaatcgcc gatgtgtgcc gactaatcaa tgtttcagcc 1380
cggaatggcg tccagcccac gaagcaaaac gcctggggtg actggtgcaa cgtcatcggc 1440
accggcttcg gtgttcgccc ctcgactaac accggcgatc cgctccagga tgcctttgtg 1500
tggatcaagc ccggtggaga gagtgatggc acgtccaact cgacttcccc ccggtatgac 1560
gcgcactgcg gatatagtga tgctctgcag cctgctcctg aggctggtac ttggttccag 1620
gtatgtcatc cattagccag atgagggata agtgactgac ggacctaggc ctactttgag 1680
cagcttctga ccaacgctaa cccgtccttt taa 1713

<210> 8
<211> 454

<212> PRT
<213> Aspergillus fumigatus

<400> 8

```
Met Lys His Leu Ala Ser Ser Ile Ala Leu Thr Leu Leu Leu Pro Ala
1               5                   10                  15

Val Gln Ala Gln Gln Thr Val Trp Gly Gln Cys Gly Gly Gln Gly Trp
            20                  25                  30

Ser Gly Pro Thr Ser Cys Val Ala Gly Ala Ala Cys Ser Thr Leu Asn
        35                  40                  45

Pro Tyr Tyr Ala Gln Cys Ile Pro Gly Ala Thr Ala Thr Ser Thr Thr
    50                  55                  60

Leu Thr Thr Thr Thr Ala Ala Thr Thr Thr Ser Gln Thr Thr Thr Lys
65                  70                  75                  80

Pro Thr Thr Thr Gly Pro Thr Thr Ser Ala Pro Thr Val Thr Ala Ser
                85                  90                  95

Gly Asn Pro Phe Ser Gly Tyr Gln Leu Tyr Ala Asn Pro Tyr Tyr Ser
            100                 105                 110

Ser Glu Val His Thr Leu Ala Met Pro Ser Leu Pro Ser Ser Leu Gln
        115                 120                 125

Pro Lys Ala Ser Ala Val Ala Glu Val Pro Ser Phe Val Trp Leu Asp
    130                 135                 140

Val Ala Ala Lys Val Pro Thr Met Gly Thr Tyr Leu Ala Asp Ile Gln
145                 150                 155                 160

Ala Lys Asn Lys Ala Gly Ala Asn Pro Pro Ile Ala Gly Ile Phe Val
                165                 170                 175
```

```
Val Tyr Asp Leu Pro Asp Arg Asp Cys Ala Ala Leu Ala Ser Asn Gly
            180                 185                 190

Glu Tyr Ser Ile Ala Asn Asn Gly Val Ala Asn Tyr Lys Ala Tyr Ile
        195                 200                 205

Asp Ala Ile Arg Ala Gln Leu Val Lys Tyr Ser Asp Val His Thr Ile
    210                 215                 220

Leu Val Ile Glu Pro Asp Ser Leu Ala Asn Leu Val Thr Asn Leu Asn
225                 230                 235                 240

Val Ala Lys Cys Ala Asn Ala Gln Ser Ala Tyr Leu Glu Cys Val Asp
                245                 250                 255

Tyr Ala Leu Lys Gln Leu Asn Leu Pro Asn Val Ala Met Tyr Leu Asp
            260                 265                 270

Ala Gly His Ala Gly Trp Leu Gly Trp Pro Ala Asn Leu Gly Pro Ala
        275                 280                 285

Ala Thr Leu Phe Ala Lys Val Tyr Thr Asp Ala Gly Ser Pro Ala Ala
    290                 295                 300

Val Arg Gly Leu Ala Thr Asn Val Ala Asn Tyr Asn Ala Trp Ser Leu
305                 310                 315                 320

Ser Thr Cys Pro Ser Tyr Thr Gln Gly Asp Pro Asn Cys Asp Glu Lys
                325                 330                 335

Lys Tyr Ile Asn Ala Met Ala Pro Leu Leu Lys Glu Ala Gly Phe Asp
            340                 345                 350

Ala His Phe Ile Met Asp Thr Ser Arg Asn Gly Val Gln Pro Thr Lys
        355                 360                 365

Gln Asn Ala Trp Gly Asp Trp Cys Asn Val Ile Gly Thr Gly Phe Gly
    370                 375                 380

Val Arg Pro Ser Thr Asn Thr Gly Asp Pro Leu Gln Asp Ala Phe Val
385                 390                 395                 400

Trp Ile Lys Pro Gly Gly Glu Ser Asp Gly Thr Ser Asn Ser Thr Ser
                405                 410                 415

Pro Arg Tyr Asp Ala His Cys Gly Tyr Ser Asp Ala Leu Gln Pro Ala
            420                 425                 430
```

Pro Glu Ala Gly Thr Trp Phe Gln Ala Tyr Phe Glu Gln Leu Leu Thr
435 440 445

Asn Ala Asn Pro Ser Phe
450

<210> 9
<211> 835
<212> DNA
<213> Penicillium emersonii

<400> 9

atgctgtctt cgacgactcg caccctcgcc tttacaggcc ttgcgggcct tctgtccgct 60

cccctggtca aggcccatgg ctttgtccag ggcattgtca tcggtgacca attgtaagtc 120

cctctcttgc agttctgtcg attaactgct ggactgcttg cttgactccc tgctgactcc 180

caacagctac agcgggtaca tcgtcaactc gttcccctac gaatccaacc cacccccgt 240

catcggctgg gccacgaccg ccaccgacct gggcttcgtc gacggcacag gataccaagg 300

cccggacatc atctgccacc ggaatgcgac gcccgcgccg ctgacagccc ccgtggccgc 360

cggcggcacc gtcgagctgc agtggacgcc gtggccggac agccaccacg gacccgtcat 420

cacctacctg gcgccgtgca acggcaactg ctcgaccgtc gacaagacga cgctggagtt 480

cttcaagatc gaccagcagg gcctgatcga cgacacgagc ccgccgggca cctgggcgtc 540

ggacaacctc atcgccaaca acaatagctg gaccgtcacc attcccaaca gcgtcgcccc 600

cggcaactac gtcctgcgcc acgagatcat cgccctgcac tcggccaaca acaaggacgg 660

cgcccagaac tacccccagt gcatcaacat cgaggtcacg ggcggcggct ccgacgcgcc 720

tgagggtact ctgggcgagg atctctacca tgacaccgac ccgggcattc tggtcgacat 780

ttacgagccc attgcgacgt ataccattcc ggggccgcct gagccgacgt tctag 835

<210> 10
<211> 253
<212> PRT
<213> Penicillium emersonii

<400> 10

Met Leu Ser Ser Thr Thr Arg Thr Leu Ala Phe Thr Gly Leu Ala Gly
1 5 10 15

Leu Leu Ser Ala Pro Leu Val Lys Ala His Gly Phe Val Gln Gly Ile
20 25 30

Val Ile Gly Asp Gln Phe Tyr Ser Gly Tyr Ile Val Asn Ser Phe Pro
35 40 45

```
Tyr Glu Ser Asn Pro Pro Pro Val Ile Gly Trp Ala Thr Thr Ala Thr
    50                  55                  60

Asp Leu Gly Phe Val Asp Gly Thr Gly Tyr Gln Gly Pro Asp Ile Ile
65                  70                  75                  80

Cys His Arg Asn Ala Thr Pro Ala Pro Leu Thr Ala Pro Val Ala Ala
                85                  90                  95

Gly Gly Thr Val Glu Leu Gln Trp Thr Pro Trp Pro Asp Ser His His
            100                 105                 110

Gly Pro Val Ile Thr Tyr Leu Ala Pro Cys Asn Gly Asn Cys Ser Thr
        115                 120                 125

Val Asp Lys Thr Thr Leu Glu Phe Phe Lys Ile Asp Gln Gln Gly Leu
    130                 135                 140

Ile Asp Asp Thr Ser Pro Pro Gly Thr Trp Ala Ser Asp Asn Leu Ile
145                 150                 155                 160

Ala Asn Asn Asn Ser Trp Thr Val Thr Ile Pro Asn Ser Val Ala Pro
                165                 170                 175

Gly Asn Tyr Val Leu Arg His Glu Ile Ile Ala Leu His Ser Ala Asn
            180                 185                 190

Asn Lys Asp Gly Ala Gln Asn Tyr Pro Gln Cys Ile Asn Ile Glu Val
        195                 200                 205

Thr Gly Gly Gly Ser Asp Ala Pro Glu Gly Thr Leu Gly Glu Asp Leu
    210                 215                 220

Tyr His Asp Thr Asp Pro Gly Ile Leu Val Asp Ile Tyr Glu Pro Ile
225                 230                 235                 240

Ala Thr Tyr Thr Ile Pro Gly Pro Pro Glu Pro Thr Phe
                245                 250
```

<210> 11
<211> 1415
<212> DNA
<213> Aspergillus fumigatus

<400> 11

```
atggtccatc tatcttcatt ggcagcagcc ctggctgctc tgcctctgta tgtttaccca      60
ctcacgagag gaggaacagc tttgacattg ctatagtgta tatggagctg gcctgaacac     120
agcagccaaa gccaaaggac taaagtactt tggttccgcc acggacaatc cagagctcac     180
ggactctgcg tatgtcgcgc aactgagcaa caccgatgat tttggtcaaa tcacacccgg     240
aaactccatg aaggtttgct tacgtctgcc tccctggagc attgcctcaa aagctaattg     300
gttgttttgt ttggatagtg ggatgccacc gagccttctc agaattcttt ttcgttcgca     360
aatggagacg ccgtggtcaa tctggcgaac aagaatggcc agctgatgcg atgccatact     420
ctggtctggc acagtcagct accgaactgg ggtatgtaaa cgtcttgtct attctcaaat     480
actctctaac agttgacagt ctctagcggg tcatggacca atgcgaccct tttggcggcc     540
atgaagaatc atatcaccaa tgtggttact cactacaagg ggaagtgcta cgcctgggat     600
gttgtcaatg aaggtttgtt gctccatcta tcctcaatag ttcttttgaa actgacaagc     660
ctgtcaatct agccctgaac gaggacggta ctttccgtaa ctctgtcttc taccagatca     720
tcggcccagc atacattcct attgcgttcg ccacggctgc tgccgcagat cccgacgtga     780
aactctacta caacgactac aacattgaat actcaggcgc caaagcgact gctgcgcaga     840
atatcgtcaa gatgatcaag gcctacggcg cgaagatcga cggcgtcggc ctccaggcac     900
actttatcgt cggcagcact ccgagtcaat cggatctgac gaccgtcttg aagggctaca     960
ctgctctcgg cgttgaggtg gcctataccg aacttgacat ccgcatgcag ctgccctcga    1020
ccgccgcaaa gctggcccag cagtccactg acttccaagg cgtggccgca gcatgcgtta    1080
gcaccactgg ctgcgtgggt gtcactatct gggactggac cgacaagtac tcctgggtcc    1140
ccagcgtgtt ccaaggctac ggcgccccat tgccttggga tgagaactat gtgaagaagc    1200
cagcgtacga tggcctgatg gcgggtcttg gagcaagcgg ctccggcacc acaacgacca    1260
ctactactac ttctactacg acaggaggta cggaccctac tggagtcgct cagaaatggg    1320
gacagtgtgg cggtattggc tggaccgggc caacaacttg tgtcagtggt accacttgcc    1380
aaaagctgaa tgactggtac tcacagtgcc tgtaa                               1415
```

<210> 12
<211> 397
<212> PRT
<213> Aspergillus fumigatus

<400> 12

```
Met Val His Leu Ser Ser Leu Ala Ala Ala Leu Ala Ala Leu Pro Leu
1               5                   10                  15

Val Tyr Gly Ala Gly Leu Asn Thr Ala Ala Lys Ala Lys Gly Leu Lys
            20                  25                  30

Tyr Phe Gly Ser Ala Thr Asp Asn Pro Glu Leu Thr Asp Ser Ala Tyr
        35                  40                  45
```

```
Val Ala Gln Leu Ser Asn Thr Asp Asp Phe Gly Gln Ile Thr Pro Gly
    50                  55                  60

Asn Ser Met Lys Trp Asp Ala Thr Glu Pro Ser Gln Asn Ser Phe Ser
65                  70                  75                  80

Phe Ala Asn Gly Asp Ala Val Val Asn Leu Ala Asn Lys Asn Gly Gln
                85                  90                  95

Leu Met Arg Cys His Thr Leu Val Trp His Ser Gln Leu Pro Asn Trp
            100                 105                 110

Val Ser Ser Gly Ser Trp Thr Asn Ala Thr Leu Leu Ala Ala Met Lys
        115                 120                 125

Asn His Ile Thr Asn Val Val Thr His Tyr Lys Gly Lys Cys Tyr Ala
    130                 135                 140

Trp Asp Val Val Asn Glu Ala Leu Asn Glu Asp Gly Thr Phe Arg Asn
145                 150                 155                 160

Ser Val Phe Tyr Gln Ile Ile Gly Pro Ala Tyr Ile Pro Ile Ala Phe
                165                 170                 175

Ala Thr Ala Ala Ala Ala Asp Pro Asp Val Lys Leu Tyr Tyr Asn Asp
            180                 185                 190

Tyr Asn Ile Glu Tyr Ser Gly Ala Lys Ala Thr Ala Ala Gln Asn Ile
        195                 200                 205

Val Lys Met Ile Lys Ala Tyr Gly Ala Lys Ile Asp Gly Val Gly Leu
    210                 215                 220

Gln Ala His Phe Ile Val Gly Ser Thr Pro Ser Gln Ser Asp Leu Thr
225                 230                 235                 240

Thr Val Leu Lys Gly Tyr Thr Ala Leu Gly Val Glu Val Ala Tyr Thr
                245                 250                 255

Glu Leu Asp Ile Arg Met Gln Leu Pro Ser Thr Ala Ala Lys Leu Ala
            260                 265                 270

Gln Gln Ser Thr Asp Phe Gln Gly Val Ala Ala Ala Cys Val Ser Thr
        275                 280                 285

Thr Gly Cys Val Gly Val Thr Ile Trp Asp Trp Thr Asp Lys Tyr Ser
    290                 295                 300
```

Trp Val Pro Ser Val Phe Gln Gly Tyr Gly Ala Pro Leu Pro Trp Asp
305 310 315 320

Glu Asn Tyr Val Lys Lys Pro Ala Tyr Asp Gly Leu Met Ala Gly Leu
325 330 335

Gly Ala Ser Gly Ser Gly Thr Thr Thr Thr Thr Thr Thr Thr Ser Thr
340 345 350

Thr Thr Gly Gly Thr Asp Pro Thr Gly Val Ala Gln Lys Trp Gly Gln
355 360 365

Cys Gly Gly Ile Gly Trp Thr Gly Pro Thr Thr Cys Val Ser Gly Thr
370 375 380

Thr Cys Gln Lys Leu Asn Asp Trp Tyr Ser Gln Cys Leu
385 390 395

<210> 13
<211> 1197
<212> DNA
<213> Trichophaea saccata

<400> 13

```
atgcgtacct tctcgtctct tctcggtgtt gcccttctct tgggtgcagc taatgcccag      60
gtcgcggttt ggggacagtg tggtggcatt ggttactctg gctcgacaac ctgcgctgcg     120
ggaacgactt gtgttaagct gaacgactac tactcccaat gccaacccgg cggtaccact     180
ttgacaacca ccaccaaacc cgccaccact accactacca ccacggcaac ttctccctca     240
tcttctcccg gattaaatgc cctggcacaa aagagcggcc ggtacttcgg tagtgcaact     300
gacaacccag agctctccga tgcggcatac attgccatcc tgagcaacaa aaacgagttt     360
gggatcatca cgcctggaaa ctcgatgaaa tgggatgcta ctgaaccgtc ccgcgggagt     420
ttctcgttca ctggtggaca gcaaattgtt gattttgcgc agggcaatgg gcaggctatc     480
agaggccata ctcttgtctg gtactcccag ttgccgtcct gggttactag cggaaacttc     540
gataaagcta cattgacatc gatcatgcaa aatcacatta caactcttgt cagccactgg     600
aagggccagc tcgcctactg ggatgttgtc aacgaagcat tcaacgatga tggcactttc     660
cgtcaaaacg tgttctacac aaccattgga gaggactaca tccagctcgc cttcgaagcc     720
gcccgtgccg ccgacccgac cgcaaagctc tgcatcaacg actacaacat cgagggcact     780
ggagccaagt caacagccat gtacaatctc gtctcgaagc tgaaatccgc cggcgttccc     840
atcgactgta ttggtgttca gggacacctc atcgtcggtg aagttcccac caccatccaa     900
gcaaaccttg cccagtttgc gtctttgggt gtggatgtcg cgatcacgga gctagatatc     960
agaatgacgc tgccatctac gactgcattg ctccagcagc aggctaagga ttacgtctcg    1020
gttgttacag cctgcatgaa tgttcccagg tgtatcggta tcaccatctg ggactacact    1080
gataaatact cttgggtgcc acaaaccttc agcggccagg gcgatgcttg cccatgggat    1140
gccaacctgc agaagaagcc agcctactcc gctattgcgt ctgctcttgc ggcttga       1197
```

<210> 14
<211> 398
<212> PRT
<213> Trichophaea saccata

<400> 14

```
Met Arg Thr Phe Ser Ser Leu Leu Gly Val Ala Leu Leu Leu Gly Ala
1               5                   10                  15

Ala Asn Ala Gln Val Ala Val Trp Gly Gln Cys Gly Gly Ile Gly Tyr
            20                  25                  30

Ser Gly Ser Thr Thr Cys Ala Ala Gly Thr Thr Cys Val Lys Leu Asn
        35                  40                  45

Asp Tyr Tyr Ser Gln Cys Gln Pro Gly Gly Thr Thr Leu Thr Thr Thr
    50                  55                  60

Thr Lys Pro Ala Thr Thr Thr Thr Thr Thr Thr Ala Thr Ser Pro Ser
65                  70                  75                  80

Ser Ser Pro Gly Leu Asn Ala Leu Ala Gln Lys Ser Gly Arg Tyr Phe
                85                  90                  95

Gly Ser Ala Thr Asp Asn Pro Glu Leu Ser Asp Ala Ala Tyr Ile Ala
            100                 105                 110

Ile Leu Ser Asn Lys Asn Glu Phe Gly Ile Ile Thr Pro Gly Asn Ser
        115                 120                 125

Met Lys Trp Asp Ala Thr Glu Pro Ser Arg Gly Ser Phe Ser Phe Thr
    130                 135                 140

Gly Gly Gln Gln Ile Val Asp Phe Ala Gln Gly Asn Gly Gln Ala Ile
145                 150                 155                 160

Arg Gly His Thr Leu Val Trp Tyr Ser Gln Leu Pro Ser Trp Val Thr
                165                 170                 175

Ser Gly Asn Phe Asp Lys Ala Thr Leu Thr Ser Ile Met Gln Asn His
            180                 185                 190
```

```
Ile Thr Thr Leu Val Ser His Trp Lys Gly Gln Leu Ala Tyr Trp Asp
        195                 200                 205

Val Val Asn Glu Ala Phe Asn Asp Asp Gly Thr Phe Arg Gln Asn Val
    210                 215                 220

Phe Tyr Thr Thr Ile Gly Glu Asp Tyr Ile Gln Leu Ala Phe Glu Ala
225                 230                 235                 240

Ala Arg Ala Ala Asp Pro Thr Ala Lys Leu Cys Ile Asn Asp Tyr Asn
                245                 250                 255

Ile Glu Gly Thr Gly Ala Lys Ser Thr Ala Met Tyr Asn Leu Val Ser
            260                 265                 270

Lys Leu Lys Ser Ala Gly Val Pro Ile Asp Cys Ile Gly Val Gln Gly
        275                 280                 285

His Leu Ile Val Gly Glu Val Pro Thr Thr Ile Gln Ala Asn Leu Ala
    290                 295                 300

Gln Phe Ala Ser Leu Gly Val Asp Val Ala Ile Thr Glu Leu Asp Ile
305                 310                 315                 320

Arg Met Thr Leu Pro Ser Thr Thr Ala Leu Leu Gln Gln Gln Ala Lys
                325                 330                 335

Asp Tyr Val Ser Val Val Thr Ala Cys Met Asn Val Pro Arg Cys Ile
            340                 345                 350

Gly Ile Thr Ile Trp Asp Tyr Thr Asp Lys Tyr Ser Trp Val Pro Gln
        355                 360                 365

Thr Phe Ser Gly Gln Gly Asp Ala Cys Pro Trp Asp Ala Asn Leu Gln
    370                 375                 380

Lys Lys Pro Ala Tyr Ser Ala Ile Ala Ser Ala Leu Ala Ala
385                 390                 395
```

<210> 15
<211> 2376
<212> DNA
<213> Aspergillus fumigatus

<400> 15

atggcggttg ccaaatctat tgctgccgtg ctggtagcac tgttgcctgg tgcgcttgct 60

caggcgaata caagctatgt tgattacaat gtggaggcga atccggatct cacccctcag 120

```
tcggtcgcta cgattgacct gtcctttccc gactgcgaga atggaccgct cagcaagact      180
ctcgtttgcg acacgtcggc tcggccgcat gaccgagctg ctgccctggt ttccatgttc      240
accttcgagg agctggtgaa caacacaggc aacactagcc ctggtgttcc aagacttggt      300
ctccctccgt accaagtatg gagcgaggct ctccatggac ttgaccgcgc caacttcaca      360
aacgagggag agtacagctg ggccacctcg ttccccatgc ctatcctgac aatgtcggcc      420
ttgaaccgaa ccctgatcaa ccagatcgcg accatcatcg caactcaagg acgagctttc      480
aataacgttg ggcggtatgg gctggacgtg tacgccccga atataaatgc attcagatcg      540
gctatgtggg gaagaggtca agagaccccc ggagaagacg cttactgcct ggcatcggcg      600
tatgcgtacg agtatatcac tggcatccag ggtggtgttg atccggaaca cctcaagttg      660
gtggccactg ccaaacacta tgcgggctac gatcttgaga actgggacgg tcactcccgt      720
ttgggcaacg atatgaacat tacacagcag gaactttccg aatactacac ccctcagttc      780
cttgttgcag ccagagacgc caaagtgcac agtgtcatgt gctcctacaa cgcggtaaat      840
ggggtgccca gctgcgcaaa ctcgttcttc ctccagaccc tcctccgtga cacattcggc      900
ttcgtcgagg atggttatgt atccagcgac tgcgactcgg cgtacaatgt ctggaacccg      960
cacgagtttg cggccaacat cacgggggcc gctgcagact ctatccgggc ggggacggac     1020
attgattgcg gcactactta tcaatactat ttcggcgaag cctttgacga gcaagaggtc     1080
acccgtgcag aaatcgaaag aggtgtgatc cgcctgtaca gcaacttggt gcgtctcggc     1140
tatttcgatg gcaatggaag cgtgtatcgg gacctgacgt ggaatgatgt cgtgaccacg     1200
gatgcctgga atatctcata cgaagccgct gtagaaggca ttgtcctact gaagaacgat     1260
ggaaccttgc ctctcgccaa gtcggtccgc agtgttgcat tgattgggcc ctggatgaat     1320
gtgacgactc agcttcaggg caactacttt ggaccggcgc cttatctgat tagtccgttg     1380
aatgccttcc agaattctga cttcgacgtg aactacgctt tcggcacgaa catttcatcc     1440
cactccacag atgggttttc cgaggcgttg tctgctgcga agaaatccga cgtcatcata     1500
ttcgcgggcg ggattgacaa cactttggaa gcagaagcca tggatcgcat gaatatcaca     1560
tggcccggca atcagctaca gctcatcgac cagttgagcc aactcggcaa accgctgatc     1620
gtcctccaga tgggcggcgg ccaagtcgac tcctcctcgc tcaagtccaa caagaatgtc     1680
aactccctga tctggggtgg ataccccgga caatccggcg ggcaggctct cctagacatc     1740
atcaccggca agcgcgcccc cgccggccga ctcgtggtca cgcagtaccc ggccgaatac     1800
gcaacccagt tccccgccac cgacatgagc ctgcggcctc acggcaataa tcccggccag     1860
acctacatgt ggtacaccgg caccccgtc tacgagtttg gccacgggct cttctacacg     1920
accttccacg cctccctccc tggcaccggc aaggacaaga cctccttcaa catccaagac     1980
ctcctcacgc agccgcatcc gggcttcgca aacgtcgagc aaatgccttt gctcaacttc     2040
```

accgtgacga tcaccaatac cggcaaggtc gcttccgact acactgctat gctcttcgcg 2100

aacaccaccg cgggacctgc tccatacccg aacaagtggc tcgtcggctt cgaccggctg 2160

gcgagcctgg aaccgcacag gtcgcagact atgaccatcc ccgtgactat cgacagcgtg 2220

gctcgtacgg atgaggccgg caatcgggtt ctctacccgg gaaagtacga gttggccctg 2280

aacaatgagc ggtcggttgt ccttcagttt gtgctgacag gccgagaggc tgtgattttc 2340

aagtggcctg tagagcagca gcagatttcg tctgcg 2376

<210> 16
<211> 792
<212> PRT
<213> Aspergillus fumigatus

<400> 16

```
Met Ala Val Ala Lys Ser Ile Ala Ala Val Leu Val Ala Leu Leu Pro
1               5                   10                  15

Gly Ala Leu Ala Gln Ala Asn Thr Ser Tyr Val Asp Tyr Asn Val Glu
            20                  25                  30

Ala Asn Pro Asp Leu Thr Pro Gln Ser Val Ala Thr Ile Asp Leu Ser
        35                  40                  45

Phe Pro Asp Cys Glu Asn Gly Pro Leu Ser Lys Thr Leu Val Cys Asp
    50                  55                  60

Thr Ser Ala Arg Pro His Asp Arg Ala Ala Ala Leu Val Ser Met Phe
65                  70                  75                  80

Thr Phe Glu Glu Leu Val Asn Asn Thr Gly Asn Thr Ser Pro Gly Val
                85                  90                  95

Pro Arg Leu Gly Leu Pro Pro Tyr Gln Val Trp Ser Glu Ala Leu His
            100                 105                 110

Gly Leu Asp Arg Ala Asn Phe Thr Asn Glu Gly Glu Tyr Ser Trp Ala
        115                 120                 125

Thr Ser Phe Pro Met Pro Ile Leu Thr Met Ser Ala Leu Asn Arg Thr
    130                 135                 140

Leu Ile Asn Gln Ile Ala Thr Ile Ile Ala Thr Gln Gly Arg Ala Phe
145                 150                 155                 160

Asn Asn Val Gly Arg Tyr Gly Leu Asp Val Tyr Ala Pro Asn Ile Asn
                165                 170                 175
```

```
Ala Phe Arg Ser Ala Met Trp Gly Arg Gly Gln Glu Thr Pro Gly Glu
            180                 185                 190

Asp Ala Tyr Cys Leu Ala Ser Ala Tyr Ala Tyr Glu Tyr Ile Thr Gly
        195                 200                 205

Ile Gln Gly Gly Val Asp Pro Glu His Leu Lys Leu Val Ala Thr Ala
    210                 215                 220

Lys His Tyr Ala Gly Tyr Asp Leu Glu Asn Trp Asp Gly His Ser Arg
225                 230                 235                 240

Leu Gly Asn Asp Met Asn Ile Thr Gln Gln Glu Leu Ser Glu Tyr Tyr
                245                 250                 255

Thr Pro Gln Phe Leu Val Ala Ala Arg Asp Ala Lys Val His Ser Val
            260                 265                 270

Met Cys Ser Tyr Asn Ala Val Asn Gly Val Pro Ser Cys Ala Asn Ser
        275                 280                 285

Phe Phe Leu Gln Thr Leu Leu Arg Asp Thr Phe Gly Phe Val Glu Asp
    290                 295                 300

Gly Tyr Val Ser Ser Asp Cys Asp Ser Ala Tyr Asn Val Trp Asn Pro
305                 310                 315                 320

His Glu Phe Ala Ala Asn Ile Thr Gly Ala Ala Ala Asp Ser Ile Arg
                325                 330                 335

Ala Gly Thr Asp Ile Asp Cys Gly Thr Thr Tyr Gln Tyr Tyr Phe Gly
            340                 345                 350

Glu Ala Phe Asp Glu Gln Glu Val Thr Arg Ala Glu Ile Glu Arg Gly
        355                 360                 365

Val Ile Arg Leu Tyr Ser Asn Leu Val Arg Leu Gly Tyr Phe Asp Gly
    370                 375                 380

Asn Gly Ser Val Tyr Arg Asp Leu Thr Trp Asn Asp Val Val Thr Thr
385                 390                 395                 400

Asp Ala Trp Asn Ile Ser Tyr Glu Ala Ala Val Glu Gly Ile Val Leu
                405                 410                 415

Leu Lys Asn Asp Gly Thr Leu Pro Leu Ala Lys Ser Val Arg Ser Val
```

420 425 430

Ala Leu Ile Gly Pro Trp Met Asn Val Thr Thr Gln Leu Gln Gly Asn
435 440 445

Tyr Phe Gly Pro Ala Pro Tyr Leu Ile Ser Pro Leu Asn Ala Phe Gln
450 455 460

Asn Ser Asp Phe Asp Val Asn Tyr Ala Phe Gly Thr Asn Ile Ser Ser
465 470 475 480

His Ser Thr Asp Gly Phe Ser Glu Ala Leu Ser Ala Ala Lys Lys Ser
485 490 495

Asp Val Ile Ile Phe Ala Gly Gly Ile Asp Asn Thr Leu Glu Ala Glu
500 505 510

Ala Met Asp Arg Met Asn Ile Thr Trp Pro Gly Asn Gln Leu Gln Leu
515 520 525

Ile Asp Gln Leu Ser Gln Leu Gly Lys Pro Leu Ile Val Leu Gln Met
530 535 540

Gly Gly Gly Gln Val Asp Ser Ser Ser Leu Lys Ser Asn Lys Asn Val
545 550 555 560

Asn Ser Leu Ile Trp Gly Gly Tyr Pro Gly Gln Ser Gly Gly Gln Ala
565 570 575

Leu Leu Asp Ile Ile Thr Gly Lys Arg Ala Pro Ala Gly Arg Leu Val
580 585 590

Val Thr Gln Tyr Pro Ala Glu Tyr Ala Thr Gln Phe Pro Ala Thr Asp
595 600 605

Met Ser Leu Arg Pro His Gly Asn Asn Pro Gly Gln Thr Tyr Met Trp
610 615 620

Tyr Thr Gly Thr Pro Val Tyr Glu Phe Gly His Gly Leu Phe Tyr Thr
625 630 635 640

Thr Phe His Ala Ser Leu Pro Gly Thr Gly Lys Asp Lys Thr Ser Phe
645 650 655

Asn Ile Gln Asp Leu Leu Thr Gln Pro His Pro Gly Phe Ala Asn Val
660 665 670

Glu Gln Met Pro Leu Leu Asn Phe Thr Val Thr Ile Thr Asn Thr Gly
675 680 685

Lys Val Ala Ser Asp Tyr Thr Ala Met Leu Phe Ala Asn Thr Thr Ala
690 695 700

Gly Pro Ala Pro Tyr Pro Asn Lys Trp Leu Val Gly Phe Asp Arg Leu
705 710 715 720

Ala Ser Leu Glu Pro His Arg Ser Gln Thr Met Thr Ile Pro Val Thr
725 730 735

Ile Asp Ser Val Ala Arg Thr Asp Glu Ala Gly Asn Arg Val Leu Tyr
740 745 750

Pro Gly Lys Tyr Glu Leu Ala Leu Asn Asn Glu Arg Ser Val Val Leu
755 760 765

Gln Phe Val Leu Thr Gly Arg Glu Ala Val Ile Phe Lys Trp Pro Val
770 775 780

Glu Gln Gln Gln Ile Ser Ser Ala
785 790

<210> 17
<211> 2391
<212> DNA
<213> Talaromyces emersonii

<400> 17

```
atgatgactc ccacggcgat tctcaccgca gtggcggcgc tcctgcccac cgcgacatgg      60
gcacaggata accaaaccta tgccaattac tcgtcgcagt ctcagccgga cctgtttccc     120
cggaccgtcg cgaccatcga cctgtccttc cccgactgtg agaatggccc gctcagcacg     180
aacctggtgt gcaacaaatc ggccgatccc tgggcccgag ctgaggccct catctcgctc     240
tttaccctcg aagagctgat taacaacacc cagaacaccg ctcctggcgt gccccgtttg     300
ggtctgcccc agtatcaggt gtggaatgaa gctctgcacg gactggaccg cgccaatttc     360
tcccattcgg gcgaatacag ctgggccacg tccttcccca tgcccatcct gtcgatggcg     420
tccttcaacc ggaccctcat caaccagatt gcctccatca ttgcaacgca agcccgtgcc     480
ttcaacaacg ccggccgtta cggccttgac agctatgcgc ccaacatcaa tggcttccgc     540
agtcccctct ggggccgtgg acaggagacg cctggtgagg atgcgttctt cttgagttcc     600
acctatgcgt acgagtacat cacaggcctg cagggcggtg tcgacccaga gcatgtcaag     660
atcgtcgcga cggcgaagca cttcgccggc tatgatctgg agaactgggg caacgtctct     720
cggctggggt tcaatgctat catcacgcag caggatctct ccgagtacta cacccctcag     780
```

ttcctggcgt ctgctcgata cgccaagacg cgcagcatca tgtgctccta caatgcagtg 840
aatggagtcc caagctgtgc caactccttc ttcctccaga cgcttctccg agaaaacttt 900
gacttcgttg acgacgggta cgtctcgtcg gattgcgacg ccgtctacaa cgtcttcaac 960
ccacacggtt acgcccttaa ccagtcggga gccgctgcgg actcgctcct agcaggtacc 1020
gatatcgact gtggtcagac cttgccgtgg cacctgaatg agtccttcgt agaaggatac 1080
gtctcccgcg gtgatatcga gaaatccctc acccgtctct actcaaacct ggtgcgtctc 1140
ggctactttg acggcaacaa cagcgagtac cgcaacctca actggaacga cgtcgtgact 1200
acggacgcct ggaacatctc gtacgaggcc gcggtggaag gtatcaccct gctcaagaac 1260
gacggaacgc tgccgctgtc caagaaggtc cgcagcattg cgctcatcgg tccttgggcc 1320
aatgccacgg tgcagatgca gggtaactac tatggaacgc caccgtatct gatcagtccg 1380
ctggaagccg ccaaggccag tgggttcacg gtcaactatg cattcggtac caacatctcg 1440
accgattcta cccagtggtt cgcggaagcc atcgcggcgg cgaagaagtc ggacgtgatc 1500
atctacgccg gtggtattga caacacgatc gaggcagagg gacaggaccg cacggatctc 1560
aagtggccgg ggaaccagct ggatctgatc gagcagctca gccaggtggg caagcccttg 1620
gtcgtcctgc agatgggcgg tggccaggtg gattcgtcgt cactcaaggc caacaagaat 1680
gtcaacgctc tggtgtgggg tggctatccc ggacagtcgg gtggtgcggc cctgtttgac 1740
atccttacgg gcaagcgtgc gccggccggt cgtctggtga gcacgcagta cccggccgag 1800
tatgcgacgc agttcccggc caacgacatg aacctgcgtc cgaacggcag caacccggga 1860
cagacataca tctggtacac gggcacgccc gtgtatgagt tcggccacgg tctgttctac 1920
acggagttcc aggagtcggc tgcggcgggc acgaacaaga cgtcgacttt cgacattctg 1980
gaccttttct ccacccctca tccgggatac gagtacatcg agcaggttcc gttcatcaac 2040
gtgactgtgg acgtgaagaa cgtcggccac acgccatcgc cgtacacggg tctgttgttc 2100
gcgaacacga cagccgggcc caagccgtac ccgaacaaat ggctcgtcgg gttcgactgg 2160
ctgccgacga tccagccggg cgagactgcc aagttgacga tcccggtgcc gttgggcgcg 2220
attgcgtggg cggacgagaa cggcaacaag gtggtcttcc cgggcaacta cgaattggca 2280
ctgaacaatg agcgatcggt agtggtgtcg ttcacgctga cgggcgatgc ggcgactcta 2340
gagaaatggc ctttgtggga gcaggcggtt ccgggggtgc tgcagcaata a 2391

<210> 18
<211> 796
<212> PRT
<213> Talaromyces emersonii

<400> 18

Met Met Thr Pro Thr Ala Ile Leu Thr Ala Val Ala Ala Leu Leu Pro
1               5                   10                  15

Thr Ala Thr Trp Ala Gln Asp Asn Gln Thr Tyr Ala Asn Tyr Ser Ser
            20                  25                  30

Gln Ser Gln Pro Asp Leu Phe Pro Arg Thr Val Ala Thr Ile Asp Leu
        35                  40                  45

Ser Phe Pro Asp Cys Glu Asn Gly Pro Leu Ser Thr Asn Leu Val Cys
    50                  55                  60

Asn Lys Ser Ala Asp Pro Trp Ala Arg Ala Glu Ala Leu Ile Ser Leu
65                  70                  75                  80

Phe Thr Leu Glu Glu Leu Ile Asn Asn Thr Gln Asn Thr Ala Pro Gly
                85                  90                  95

Val Pro Arg Leu Gly Leu Pro Gln Tyr Gln Val Trp Asn Glu Ala Leu
            100                 105                 110

His Gly Leu Asp Arg Ala Asn Phe Ser His Ser Gly Glu Tyr Ser Trp
        115                 120                 125

Ala Thr Ser Phe Pro Met Pro Ile Leu Ser Met Ala Ser Phe Asn Arg
    130                 135                 140

Thr Leu Ile Asn Gln Ile Ala Ser Ile Ile Ala Thr Gln Ala Arg Ala
145                 150                 155                 160

Phe Asn Asn Ala Gly Arg Tyr Gly Leu Asp Ser Tyr Ala Pro Asn Ile
                165                 170                 175

Asn Gly Phe Arg Ser Pro Leu Trp Gly Arg Gly Gln Glu Thr Pro Gly
            180                 185                 190

Glu Asp Ala Phe Phe Leu Ser Ser Thr Tyr Ala Tyr Glu Tyr Ile Thr
        195                 200                 205

Gly Leu Gln Gly Gly Val Asp Pro Glu His Val Lys Ile Val Ala Thr
    210                 215                 220

Ala Lys His Phe Ala Gly Tyr Asp Leu Glu Asn Trp Gly Asn Val Ser
225                 230                 235                 240

Arg Leu Gly Phe Asn Ala Ile Ile Thr Gln Gln Asp Leu Ser Glu Tyr
                245                 250                 255

```
Tyr Thr Pro Gln Phe Leu Ala Ser Ala Arg Tyr Ala Lys Thr Arg Ser
            260                 265                 270

Ile Met Cys Ser Tyr Asn Ala Val Asn Gly Val Pro Ser Cys Ala Asn
        275                 280                 285

Ser Phe Phe Leu Gln Thr Leu Leu Arg Glu Asn Phe Asp Phe Val Asp
    290                 295                 300

Asp Gly Tyr Val Ser Ser Asp Cys Asp Ala Val Tyr Asn Val Phe Asn
305                 310                 315                 320

Pro His Gly Tyr Ala Leu Asn Gln Ser Gly Ala Ala Ala Asp Ser Leu
                325                 330                 335

Leu Ala Gly Thr Asp Ile Asp Cys Gly Gln Thr Leu Pro Trp His Leu
            340                 345                 350

Asn Glu Ser Phe Val Glu Gly Tyr Val Ser Arg Gly Asp Ile Glu Lys
        355                 360                 365

Ser Leu Thr Arg Leu Tyr Ser Asn Leu Val Arg Leu Gly Tyr Phe Asp
    370                 375                 380

Gly Asn Asn Ser Glu Tyr Arg Asn Leu Asn Trp Asn Asp Val Val Thr
385                 390                 395                 400

Thr Asp Ala Trp Asn Ile Ser Tyr Glu Ala Ala Val Glu Gly Ile Thr
                405                 410                 415

Leu Leu Lys Asn Asp Gly Thr Leu Pro Leu Ser Lys Lys Val Arg Ser
            420                 425                 430

Ile Ala Leu Ile Gly Pro Trp Ala Asn Ala Thr Val Gln Met Gln Gly
        435                 440                 445

Asn Tyr Tyr Gly Thr Pro Pro Tyr Leu Ile Ser Pro Leu Glu Ala Ala
    450                 455                 460

Lys Ala Ser Gly Phe Thr Val Asn Tyr Ala Phe Gly Thr Asn Ile Ser
465                 470                 475                 480

Thr Asp Ser Thr Gln Trp Phe Ala Glu Ala Ile Ala Ala Ala Lys Lys
                485                 490                 495

Ser Asp Val Ile Ile Tyr Ala Gly Gly Ile Asp Asn Thr Ile Glu Ala
            500                 505                 510
```

```
Glu Gly Gln Asp Arg Thr Asp Leu Lys Trp Pro Gly Asn Gln Leu Asp
        515                 520                 525

Leu Ile Glu Gln Leu Ser Gln Val Gly Lys Pro Leu Val Val Leu Gln
    530                 535                 540

Met Gly Gly Gly Gln Val Asp Ser Ser Ser Leu Lys Ala Asn Lys Asn
545                 550                 555                 560

Val Asn Ala Leu Val Trp Gly Gly Tyr Pro Gly Gln Ser Gly Gly Ala
                565                 570                 575

Ala Leu Phe Asp Ile Leu Thr Gly Lys Arg Ala Pro Ala Gly Arg Leu
            580                 585                 590

Val Ser Thr Gln Tyr Pro Ala Glu Tyr Ala Thr Gln Phe Pro Ala Asn
        595                 600                 605

Asp Met Asn Leu Arg Pro Asn Gly Ser Asn Pro Gly Gln Thr Tyr Ile
    610                 615                 620

Trp Tyr Thr Gly Thr Pro Val Tyr Glu Phe Gly His Gly Leu Phe Tyr
625                 630                 635                 640

Thr Glu Phe Gln Glu Ser Ala Ala Ala Gly Thr Asn Lys Thr Ser Thr
                645                 650                 655

Phe Asp Ile Leu Asp Leu Phe Ser Thr Pro His Pro Gly Tyr Glu Tyr
            660                 665                 670

Ile Glu Gln Val Pro Phe Ile Asn Val Thr Val Asp Val Lys Asn Val
        675                 680                 685

Gly His Thr Pro Ser Pro Tyr Thr Gly Leu Leu Phe Ala Asn Thr Thr
    690                 695                 700

Ala Gly Pro Lys Pro Tyr Pro Asn Lys Trp Leu Val Gly Phe Asp Trp
705                 710                 715                 720

Leu Pro Thr Ile Gln Pro Gly Glu Thr Ala Lys Leu Thr Ile Pro Val
                725                 730                 735

Pro Leu Gly Ala Ile Ala Trp Ala Asp Glu Asn Gly Asn Lys Val Val
            740                 745                 750

Phe Pro Gly Asn Tyr Glu Leu Ala Leu Asn Asn Glu Arg Ser Val Val
```

755 760 765

Val Ser Phe Thr Leu Thr Gly Asp Ala Ala Thr Leu Glu Lys Trp Pro
770 775 780

Leu Trp Glu Gln Ala Val Pro Gly Val Leu Gln Gln
785 790 795

<210> 19
<211> 1008
<212> DNA
<213> Thermoascus aurantiacus

<400> 19

```
atgaagctcg gctctctcgt gctcgctctc agcgcagcta ggcttacact gtcggcccct      60
ctcgcagaca gaaagcagga gaccaagcgt gcgaaagtat tccaatggtt cggttcgaac     120
gagtccggtg ctgaattcgg aagccagaac cttccaggag tcgagggaaa ggattatata     180
tggcctgatc ccaacaccat tgacacattg atcagcaagg ggatgaacat ctttcgtgtc     240
ccctttatga tggagagatt ggttcccaac tcaatgaccg gctctccgga tccgaactac     300
ctggcagatc tcatagcgac tgtaaatgca atcacccaga aaggtgccta cgccgtcgtc     360
gatcctcata actacggcag atactacaat tctataatct cgagcccttc cgatttccag     420
accttctgga aaacggtcgc ctcacagttt gcttcgaatc cactggtcat cttcgacact     480
aataacgaat accacgatat ggaccagacc ttagtcctca atctcaacca ggccgctatc     540
gacggcatcc gttccgccgg agccacttcc cagtacatct ttgtcgaggg caattcgtgg     600
accggggcat ggacctggac gaacgtgaac gataacatga aaagcctgac cgacccatct     660
gacaagatca tatacgagat gcaccagtac ctggactctg acggatccgg gacatcagcg     720
acctgcgtat cttcgaccat cggtcaagag cgaatcacca gcgcaacgca gtggctcagg     780
gccaacggga agaagggcat catcggcgag tttgcgggcg gagccaacga cgtctgcgag     840
acggccatca cgggcatgct ggactacatg gcccagaaca cagacgtctg gactggcgcc     900
atctggtggg cggccgggcc gtggtgggga gactacatat tctccatgga gccggacaat     960
ggcatcgcgt atcagcagat acttcctatt ttgactccgt atctttga                 1008
```

<210> 20
<211> 335
<212> PRT
<213> Thermoascus aurantiacus

<400> 20

Met Lys Leu Gly Ser Leu Val Leu Ala Leu Ser Ala Ala Arg Leu Thr
1               5                   10                  15

```
Leu Ser Ala Pro Leu Ala Asp Arg Lys Gln Glu Thr Lys Arg Ala Lys
            20                  25                  30

Val Phe Gln Trp Phe Gly Ser Asn Glu Ser Gly Ala Glu Phe Gly Ser
        35                  40                  45

Gln Asn Leu Pro Gly Val Glu Gly Lys Asp Tyr Ile Trp Pro Asp Pro
    50                  55                  60

Asn Thr Ile Asp Thr Leu Ile Ser Lys Gly Met Asn Ile Phe Arg Val
65                  70                  75                  80

Pro Phe Met Met Glu Arg Leu Val Pro Asn Ser Met Thr Gly Ser Pro
                85                  90                  95

Asp Pro Asn Tyr Leu Ala Asp Leu Ile Ala Thr Val Asn Ala Ile Thr
            100                 105                 110

Gln Lys Gly Ala Tyr Ala Val Val Asp Pro His Asn Tyr Gly Arg Tyr
        115                 120                 125

Tyr Asn Ser Ile Ile Ser Ser Pro Ser Asp Phe Gln Thr Phe Trp Lys
    130                 135                 140

Thr Val Ala Ser Gln Phe Ala Ser Asn Pro Leu Val Ile Phe Asp Thr
145                 150                 155                 160

Asn Asn Glu Tyr His Asp Met Asp Gln Thr Leu Val Leu Asn Leu Asn
                165                 170                 175

Gln Ala Ala Ile Asp Gly Ile Arg Ser Ala Gly Ala Thr Ser Gln Tyr
            180                 185                 190

Ile Phe Val Glu Gly Asn Ser Trp Thr Gly Ala Trp Thr Trp Thr Asn
        195                 200                 205

Val Asn Asp Asn Met Lys Ser Leu Thr Asp Pro Ser Asp Lys Ile Ile
    210                 215                 220

Tyr Glu Met His Gln Tyr Leu Asp Ser Asp Gly Ser Gly Thr Ser Ala
225                 230                 235                 240

Thr Cys Val Ser Ser Thr Ile Gly Gln Glu Arg Ile Thr Ser Ala Thr
                245                 250                 255

Gln Trp Leu Arg Ala Asn Gly Lys Lys Gly Ile Ile Gly Glu Phe Ala
            260                 265                 270
```

```
Gly Gly Ala Asn Asp Val Cys Glu Thr Ala Ile Thr Gly Met Leu Asp
        275                 280                 285

Tyr Met Ala Gln Asn Thr Asp Val Trp Thr Gly Ala Ile Trp Trp Ala
    290                 295                 300

Ala Gly Pro Trp Trp Gly Asp Tyr Ile Phe Ser Met Glu Pro Asp Asn
305                 310                 315                 320

Gly Ile Ala Tyr Gln Gln Ile Leu Pro Ile Leu Thr Pro Tyr Leu
                325                 330                 335
```

<210> 21
<211> 1520
<212> DNA
<213> Talaromyces leycettanus

<400> 21

atggtccatc tttcttccct ggccctggct ttggccgccg gctcgcagct gtatgtgatc 60
catgccatga ctcgagaagt gctcccaaaa ctgactccaa gtctcaatct tagtgcccaa 120
gctgcaggtc ttaacactgc tgccaaagcg attggaaagc tctatttcgg taccgcaacc 180
gacaacccgg agctgtccga cagcacatac atgcaggaga cggataacac cgatgatttc 240
ggccaactca ccccagctaa ctccatgaag gttcgctgac atcttagttc cccccccctt 300
ttgggaatct gcgcggagat atgctgagcc ttcaaaacta gtgggatgcc accgagccct 360
ctcagaacac cttcaccttc accaacggtg atcagatcgc aaaccttgct aagagcaacg 420
gtcagatgct gagatgccac aacctggtgt ggtacaacca gttgcccagc tggggtaagc 480
aaccggttct gttaatatca tcagcgtgac cgcatcgatc gtattgcgcg gagattggaa 540
agatttgcaa gctaatgtca ctacagtcac cagcggatct tggaccaatg ccacgcttct 600
tgcggccatg aagaaccaca tcaccaacgt tgtgacccac tacaagggac agtgctacgc 660
ttgggatgtt gtcaacgaag gtacgtttcg attcggcttc cctcggaccg tatctgcagg 720
caaaaaggtc aatcaattga caatcgtgat ccccagctct caacgatgat ggcacctacc 780
gatccaatgt cttctatcag tacatcggcg aggcatacat tcccattgcc tttgcgaccg 840
ctgccgccgc cgatccaaac gcgaagctct actacaacga ctacaacatt gagtaccccg 900
gcgccaaggc caccgccgcc cagaacatcg tcaagatggt caaggcttac ggcgcgaaaa 960
tcgacggtgt cggtctgcaa tctcacttca tcgttggcag cacccctagc cagagctccc 1020
agcagagcaa catggctgct ttcaccgcgc tcggcgtcga ggtcgccatc accgaactgg 1080
atatccgcat gacgttgcct tccaccagtg ctctcttggc ccagcaatcc accgattacc 1140
agagcactgt gtcggcttgc gtgaacactc cgaagtgcat tggtatcacc ctctgggact 1200
ggaccgacaa gtactcctgg gttcccaaca ccttctccgg ccaaggtgac gcctgcccct 1260
gggattctaa ctaccagaag aagcctgcct actacggtat cttgactgcg ctcggaggca 1320
gcgcttccac ctccaccacc accactctgg tgacctccac caggacttcg actacgacca 1380
gcacttcggc cacctccacg tctactggcg ttgctcagca ctggggccag tgcggtggta 1440
tcggctggac agggccgact acctgcgcta gcccctacac ctgccaggaa ctgaatccct 1500
actactacca gtgcctgtaa 1520

<210> 22
<211> 405
<212> PRT
<213> Talaromyces leycettanus

<400> 22

Met Val His Leu Ser Ser Leu Ala Leu Ala Leu Ala Ala Gly Ser Gln
1               5                   10                  15

Leu Ala Gln Ala Ala Gly Leu Asn Thr Ala Ala Lys Ala Ile Gly Lys
            20                  25                  30

Leu Tyr Phe Gly Thr Ala Thr Asp Asn Pro Glu Leu Ser Asp Ser Thr
        35                  40                  45

Tyr Met Gln Glu Thr Asp Asn Thr Asp Asp Phe Gly Gln Leu Thr Pro
    50                  55                  60

Ala Asn Ser Met Lys Trp Asp Ala Thr Glu Pro Ser Gln Asn Thr Phe
65                  70                  75                  80

Thr Phe Thr Asn Gly Asp Gln Ile Ala Asn Leu Ala Lys Ser Asn Gly
                85                  90                  95

Gln Met Leu Arg Cys His Asn Leu Val Trp Tyr Asn Gln Leu Pro Ser
            100                 105                 110

Trp Val Thr Ser Gly Ser Trp Thr Asn Ala Thr Leu Leu Ala Ala Met
        115                 120                 125

Lys Asn His Ile Thr Asn Val Val Thr His Tyr Lys Gly Gln Cys Tyr
    130                 135                 140

Ala Trp Asp Val Val Asn Glu Ala Leu Asn Asp Asp Gly Thr Tyr Arg
145                 150                 155                 160

Ser Asn Val Phe Tyr Gln Tyr Ile Gly Glu Ala Tyr Ile Pro Ile Ala
                165                 170                 175

```
Phe Ala Thr Ala Ala Ala Ala Asp Pro Asn Ala Lys Leu Tyr Tyr Asn
            180                 185                 190

Asp Tyr Asn Ile Glu Tyr Pro Gly Ala Lys Ala Thr Ala Ala Gln Asn
        195                 200                 205

Ile Val Lys Met Val Lys Ala Tyr Gly Ala Lys Ile Asp Gly Val Gly
    210                 215                 220

Leu Gln Ser His Phe Ile Val Gly Ser Thr Pro Ser Gln Ser Ser Gln
225                 230                 235                 240

Gln Ser Asn Met Ala Ala Phe Thr Ala Leu Gly Val Glu Val Ala Ile
                245                 250                 255

Thr Glu Leu Asp Ile Arg Met Thr Leu Pro Ser Thr Ser Ala Leu Leu
            260                 265                 270

Ala Gln Gln Ser Thr Asp Tyr Gln Ser Thr Val Ser Ala Cys Val Asn
        275                 280                 285

Thr Pro Lys Cys Ile Gly Ile Thr Leu Trp Asp Trp Thr Asp Lys Tyr
    290                 295                 300

Ser Trp Val Pro Asn Thr Phe Ser Gly Gln Gly Asp Ala Cys Pro Trp
305                 310                 315                 320

Asp Ser Asn Tyr Gln Lys Lys Pro Ala Tyr Tyr Gly Ile Leu Thr Ala
                325                 330                 335

Leu Gly Gly Ser Ala Ser Thr Ser Thr Thr Thr Thr Leu Val Thr Ser
            340                 345                 350

Thr Arg Thr Ser Thr Thr Thr Ser Thr Ser Ala Thr Ser Thr Ser Thr
        355                 360                 365

Gly Val Ala Gln His Trp Gly Gln Cys Gly Gly Ile Gly Trp Thr Gly
    370                 375                 380

Pro Thr Thr Cys Ala Ser Pro Tyr Thr Cys Gln Glu Leu Asn Pro Tyr
385                 390                 395                 400

Tyr Tyr Gln Cys Leu
                405
```

<210> 23
<211> 2391

<212> DNA
<213> Talaromyces emersonii

<400> 23

```
atgatgactc ccacggcgat tctcaccgca gtggcggcgc tcctgcccac cgcgacatgg      60
gcacaggata accaaaccta tgccaattac tcgtcgcagt ctcagccgga cctgtttccc     120
cggaccgtcg cgaccatcga cctgtccttc cccgactgtg agaatggccc gctcagcacg     180
aacctggtgt gcaacaaatc ggccgatccc tgggcccgag ctgaggccct catctcgctc     240
tttaccctcg aagagctgat taacaacacc cagaacaccg ctcctggcgt gccccgtttg     300
ggtctgcccc agtatcaggt gtggaatgaa gctctgcacg gactggaccg cgccaatttc     360
tcccattcgg gcgaatacag ctgggccacg tccttcccca tgcccatcct gtcgatggcg     420
tccttcaacc ggaccctcat caaccagatt gcctccatca ttgcaacgca agcccgtgcc     480
ttcaacaacg ccggccgtta cggccttgac agctatgcgc ccaacatcaa tggcttccgc     540
agtcccctct ggggccgtgg acaggagacg cctggtgagg atgcgttctt cttgagttcc     600
acctatgcgt acgagtacat cacaggcctg cagggcggtg tcgacccaga gcatgtcaag     660
atcgtcgcga cggcgaagca cttcgccggc tatgatctgg agaactgggg caacgtctct     720
cggctggggt tcaatgctat catcacgcag caggatctct ccgagtacta cacccctcag     780
ttcctggcgt ctgctcgata cgccaagacg cgcagcatca tgtgctccta caatgcagtg     840
aatggagtcc caagctgtgc caactccttc ttcctccaga cgcttctccg agaaaacttt     900
gacttcgttg acgacgggta cgtctcgtcg gattgcgacg ccgtctacaa cgtcttcaac     960
ccacacggtt acgcccttaa ccagtcggga gccgctgcgg actcgctcct agcaggtacc    1020
gatatcgact gtggtcagac cttgccgtgg cacctgaatg agtccttcgt agaaggatac    1080
gtctcccgcg gtgatatcga gaaatccctc acccgtctct actcaaacct ggtgcgtctc    1140
ggctactttg acggcaacaa cagcgagtac cgcaacctca actggaacga cgtcgtgact    1200
acggacgcct ggaacatctc gtacgaggcc gcggtggaag gtatcaccct gctcaagaac    1260
gacggaacgc tgccgctgtc caagaaggtc cgcagcattg cgctcatcgg tccttgggcc    1320
aatgccacgg tgcagatgca gggtaactac tatggaacgc caccgtatct gatcagtccg    1380
ctggaagccg ccaaggccag tgggttcacg gtcaactatg cattcggtac caacatctcg    1440
accgattcta cccagtggtt cgcggaagcc atcgcggcgg cgaagaagtc ggacgtgatc    1500
atctacgccg gtggtattga caacacgatc gaggcagagg gacaggaccg cacggatctc    1560
aagtggccgg ggaaccagct ggatctgatc gagcagctca gccaggtggg caagcccttg    1620
gtcgtcctgc agatgggcgg tggccaggtg gattcgtcgt cactcaaggc caacaagaat    1680
gtcaacgctc tggtgtgggg tggctatccc ggacagtcgg gtggtgcggc cctgtttgac    1740
atccttacgg gcaagcgtgc gccggccggt cgtctggtga gcacgcagta cccggccgag    1800
```

```
tatgcgacgc agttcccggc caacgacatg aacctgcgtc cgaacggcag caacccggga     1860
cagacataca tctggtacac gggcacgccc gtgtatgagt tcggccacgg tctgttctac     1920
acggagttcc aggagtcggc tgcggcgggc acgaacaaga cgtcgacttt cgacattctg     1980
gaccttttct ccacccctca tccgggatac gagtacatcg agcaggttcc gttcatcaac     2040
gtgactgtgg acgtgaagaa cgtcggccac acgccatcgc cgtacacggg tctgttgttc     2100
gcgaacacga cagccgggcc caagccgtac ccgaacaaat ggctcgtcgg gttcgactgg     2160
ctgccgacga tccagccggg cgagactgcc aagttgacga tcccggtgcc gttgggcgcg     2220
attgcgtggg cggacgagaa cggcaacaag gtggtcttcc cgggcaacta cgaattggca     2280
ctgaacaatg agcgatcggt agtggtgtcg ttcacgctga cgggcgatgc ggcgactcta     2340
gagaaatggc ctttgtggga gcaggcggtt ccgggggtgc tgcagcaata a              2391
```

<210> 24
<211> 796
<212> PRT
<213> Talaromyces emersonii

<400> 24

```
Met Met Thr Pro Thr Ala Ile Leu Thr Ala Val Ala Ala Leu Leu Pro
1               5                   10                  15

Thr Ala Thr Trp Ala Gln Asp Asn Gln Thr Tyr Ala Asn Tyr Ser Ser
            20                  25                  30

Gln Ser Gln Pro Asp Leu Phe Pro Arg Thr Val Ala Thr Ile Asp Leu
        35                  40                  45

Ser Phe Pro Asp Cys Glu Asn Gly Pro Leu Ser Thr Asn Leu Val Cys
    50                  55                  60

Asn Lys Ser Ala Asp Pro Trp Ala Arg Ala Glu Ala Leu Ile Ser Leu
65                  70                  75                  80

Phe Thr Leu Glu Glu Leu Ile Asn Asn Thr Gln Asn Thr Ala Pro Gly
                85                  90                  95

Val Pro Arg Leu Gly Leu Pro Gln Tyr Gln Val Trp Asn Glu Ala Leu
            100                 105                 110

His Gly Leu Asp Arg Ala Asn Phe Ser His Ser Gly Glu Tyr Ser Trp
        115                 120                 125

Ala Thr Ser Phe Pro Met Pro Ile Leu Ser Met Ala Ser Phe Asn Arg
    130                 135                 140
```

```
Thr Leu Ile Asn Gln Ile Ala Ser Ile Ile Ala Thr Gln Ala Arg Ala
145                 150                 155                 160

Phe Asn Asn Ala Gly Arg Tyr Gly Leu Asp Ser Tyr Ala Pro Asn Ile
                165                 170                 175

Asn Gly Phe Arg Ser Pro Leu Trp Gly Arg Gly Gln Glu Thr Pro Gly
            180                 185                 190

Glu Asp Ala Phe Phe Leu Ser Ser Thr Tyr Ala Tyr Glu Tyr Ile Thr
        195                 200                 205

Gly Leu Gln Gly Gly Val Asp Pro Glu His Val Lys Ile Val Ala Thr
    210                 215                 220

Ala Lys His Phe Ala Gly Tyr Asp Leu Glu Asn Trp Gly Asn Val Ser
225                 230                 235                 240

Arg Leu Gly Phe Asn Ala Ile Ile Thr Gln Gln Asp Leu Ser Glu Tyr
                245                 250                 255

Tyr Thr Pro Gln Phe Leu Ala Ser Ala Arg Tyr Ala Lys Thr Arg Ser
            260                 265                 270

Ile Met Cys Ser Tyr Asn Ala Val Asn Gly Val Pro Ser Cys Ala Asn
        275                 280                 285

Ser Phe Phe Leu Gln Thr Leu Leu Arg Glu Asn Phe Asp Phe Val Asp
    290                 295                 300

Asp Gly Tyr Val Ser Ser Asp Cys Asp Ala Val Tyr Asn Val Phe Asn
305                 310                 315                 320

Pro His Gly Tyr Ala Leu Asn Gln Ser Gly Ala Ala Ala Asp Ser Leu
                325                 330                 335

Leu Ala Gly Thr Asp Ile Asp Cys Gly Gln Thr Leu Pro Trp His Leu
            340                 345                 350

Asn Glu Ser Phe Val Glu Gly Tyr Val Ser Arg Gly Asp Ile Glu Lys
        355                 360                 365

Ser Leu Thr Arg Leu Tyr Ser Asn Leu Val Arg Leu Gly Tyr Phe Asp
    370                 375                 380

Gly Asn Asn Ser Glu Tyr Arg Asn Leu Asn Trp Asn Asp Val Val Thr
```

385 390 395 400

Thr Asp Ala Trp Asn Ile Ser Tyr Glu Ala Ala Val Glu Gly Ile Thr
405 410 415

Leu Leu Lys Asn Asp Gly Thr Leu Pro Leu Ser Lys Lys Val Arg Ser
420 425 430

Ile Ala Leu Ile Gly Pro Trp Ala Asn Ala Thr Val Gln Met Gln Gly
435 440 445

Asn Tyr Tyr Gly Thr Pro Pro Tyr Leu Ile Ser Pro Leu Glu Ala Ala
450 455 460

Lys Ala Ser Gly Phe Thr Val Asn Tyr Ala Phe Gly Thr Asn Ile Ser
465 470 475 480

Thr Asp Ser Thr Gln Trp Phe Ala Glu Ala Ile Ala Ala Ala Lys Lys
485 490 495

Ser Asp Val Ile Ile Tyr Ala Gly Gly Ile Asp Asn Thr Ile Glu Ala
500 505 510

Glu Gly Gln Asp Arg Thr Asp Leu Lys Trp Pro Gly Asn Gln Leu Asp
515 520 525

Leu Ile Glu Gln Leu Ser Gln Val Gly Lys Pro Leu Val Val Leu Gln
530 535 540

Met Gly Gly Gly Gln Val Asp Ser Ser Ser Leu Lys Ala Asn Lys Asn
545 550 555 560

Val Asn Ala Leu Val Trp Gly Gly Tyr Pro Gly Gln Ser Gly Gly Ala
565 570 575

Ala Leu Phe Asp Ile Leu Thr Gly Lys Arg Ala Pro Ala Gly Arg Leu
580 585 590

Val Ser Thr Gln Tyr Pro Ala Glu Tyr Ala Thr Gln Phe Pro Ala Asn
595 600 605

Asp Met Asn Leu Arg Pro Asn Gly Ser Asn Pro Gly Gln Thr Tyr Ile
610 615 620

Trp Tyr Thr Gly Thr Pro Val Tyr Glu Phe Gly His Gly Leu Phe Tyr
625 630 635 640

```
Thr Glu Phe Gln Glu Ser Ala Ala Ala Gly Thr Asn Lys Thr Ser Thr
                645                 650                 655

Phe Asp Ile Leu Asp Leu Phe Ser Thr Pro His Pro Gly Tyr Glu Tyr
            660                 665                 670

Ile Glu Gln Val Pro Phe Ile Asn Val Thr Val Asp Val Lys Asn Val
        675                 680                 685

Gly His Thr Pro Ser Pro Tyr Thr Gly Leu Leu Phe Ala Asn Thr Thr
    690                 695                 700

Ala Gly Pro Lys Pro Tyr Pro Asn Lys Trp Leu Val Gly Phe Asp Trp
705                 710                 715                 720

Leu Pro Thr Ile Gln Pro Gly Glu Thr Ala Lys Leu Thr Ile Pro Val
                725                 730                 735

Pro Leu Gly Ala Ile Ala Trp Ala Asp Glu Asn Gly Asn Lys Val Val
            740                 745                 750

Phe Pro Gly Asn Tyr Glu Leu Ala Leu Asn Asn Glu Arg Ser Val Val
        755                 760                 765

Val Ser Phe Thr Leu Thr Gly Asp Ala Ala Thr Leu Glu Lys Trp Pro
    770                 775                 780

Leu Trp Glu Gln Ala Val Pro Gly Val Leu Gln Gln
785                 790                 795
```